# EUROPÄISCHE PATENTSCHRIFT

(11) **EP 1 963 300 B1**
(45) Veröffentlichungstag und Bekanntmachung des Hinweises auf die Patenterteilung: **22.08.2012**
(21) Anmeldenummer: 06829304.2
(22) Anmeldetag: 05.12.2006
(51) Int. Cl.: C07D 311/80, A61K 31/35, A61P 25/00

(54) **BENZOCHROMENDERIVATE ZUR VERWENDUNG IN FLÜSSIGKRISTALLMEDIEN UND ALS THERAPEUTISCHE WIRKSTOFFE**
BENZOCHROMENE DERIVATIVES FOR USE IN LIQUID CRYSTAL MEDIA AND AS THERAPEUTIC ACTIVE SUBSTANCES
DERIVES BENZOCHROMENE DESTINES A ETRE EMPLOYES DANS DES FLUIDES A CRISTAUX LIQUIDES ET EN TANT QU'AGENTS ACTIFS THERAPEUTIQUES

(30) Priorität: 22.12.2005 DE 102005062101
(43) Veröffentlichungstag der Anmeldung: 03.09.2008
(73) Patentinhaber: Merck Patent GmbH, 64293 Darmstadt (DE)
(72) Erfinder: TAUGERBECK, Andreas, 64285 Darmstadt (DE); MONTENEGRO, Elvira, 69469 Weinheim (DE); MANABE, Atsutaka, 64625 Bensheim (DE); PLACH, Herbert, 64291 Darmstadt (DE)
(86) Internationale Anmeldenummer: PCT/EP2006/011660
(87) Internationale Veröffentlichungsnummer: WO 2007/079842

(56) Entgegenhaltungen:
- DE-A1-102004 004 228
- NOVAROLI ET AL: "Human recombinant monoamine oxidase B as reliable and efficient enzyme source for inhibitor screening" BIOORGANIC & MEDICINAL CHEMISTRY, ELSEVIER SCIENCE LTD, GB, Bd. 13, Nr. 22, 15. November 2005 (2005-11-15), Seiten 6212-6217, XP005098160 ISSN: 0968-0896
- KIM ET AL.: "Estrogen receptor Ligands. 12. Synthesis of the Major" ORGANIC LETTERS, Bd. 7, Nr. 3, Mai 2005 (2005-05), Seiten 411-414, XP002419869

## Beschreibung

Die vorliegende Erfindung betrifft Benzochromenderivate, bevorzugt mesogene Benzochromenderivate, insbesondere flüssigkristalline Benzochromenderivate, sowie diese Benzochromenderivate enthaltende flüssigkristalline Medien. Ferner betrifft die vorliegende Erfindung Flüssigkristallanzeigen, insbesondere mittels einer aktiven Matrix angesteuerte Flüssigkristallanzeigen (AMDs oder AM LCDs nach Englisch "active matrix addressed liquid crystal displays"), also Flüssigkristallanzeigen die mittels einer Matrix von aktiven elektrischen Elementen wie z.B. TFTs ("thin film transistors"), Varistoren, Dioden oder MIMs ("metal insulator metal"), angesteuert werden und dadurch ausgezeichnet Bildqualität haben. Für diese werden insbesondere der TN ("twisted nematc") und der IPS ("in plane switching") Effekt eingesetzt, bei denen nematische Flüssigkristalle mit einer positiven dielektrischen Anisotropie (Δε) verwendet werden.

In derartigen Flüssigkristallanzeigen werden die Flüssigkristalle als Dielektrika verwendet, deren optische Eigenschaften sich bei Anlegen einer elektrischen Spannung reversibel ändern. Elektrooptische Anzeigen die Flüssigkristalle als Medien verwenden sind dem Fachmann bekannt. Diese Flüssigkristallanzeigen verwenden verschiedene elektrooptische Effekte. Die gebräuchlichsten hiervon sind der TN-Effekt (Englisch "twisted nematic"), mit einer homogenen, nahezu planaren Ausgangsorientierung des Flüssigkristalldirektors und einer um ca. 90° verdrillten nematischen Struktur), der STN-Effekt (Englisch "super-twisted nematic") und der SBE-Effekt (Englisch "supertwisted birefringence effect" mit einer 180° oder mehr verdrillten nematischen Struktur). Bei diesen und ähnlichen elektrooptischen Effekten werden flüssigkristalline Medien mit positiver dielektrischer Anisotropie (Δε) verwendet.

Ein elektrooptischer Effekt mit hervorragender, kleiner Blickwinkelabhängigkeit des Kontrasts verwendet axial symmetrische Micropixel (ASM von Englisch "axially symmetric micro pixel"). Bei diesem Effekt ist der Flüssigkristall jedes Pixels zylinderförmig von einem Polymermaterial umgeben. Dieser Mode eignet sich besonders zur Kombination mit der Adressierung durch Plasmakanäle. So lassen sich insbesondere großflächige PA (Englisch "plasma addressed") LCDs mit guter Blickwinkelabhängigkeit des Kontrasts realisieren.

Der in letzter Zeit verstärkt eingesetzte IPS-Effekt (Englisch "in plane switching") kann sowohl dielektrisch positive wie auch dielektrisch negative Flüssigkristallmedien verwenden, ähnlich wie auch "guest/host"-Anzeigen also Gast/Wirt-Anzeigen, die Farbstoffe je nach verwandtem Anzeigemodus entweder in dielektrisch positiven oder in dielektrisch negativen Medien einsetzen können.

Weiterhin sind auch LCOS-Anzeigen und Anzeigen, die auf einem Doppelbrechungseffekt beruhen, wie OCB-Anzeigen, interessant. Da bei Flüssigkristallanzeigen im allgemeinen, also auch bei Anzeigen nach diesen Effekten, die Betriebsspannung möglichst gering sein soll, werden Flüssigkristallmedien mit einem großen Absolutwert der dielektrischen Anisotropie eingesetzt, die in der Regel überwiegend und meist sogar weitestgehend aus Flüssigkristallverbindungen mit einer dielektrischen Anisotropie mit dem entsprechenden Vorzeichen bestehen. Also, bei dielektrisch positiven Medien aus Verbindungen mit positiver dielektrischer Anisotropie und bei dielektrisch negativen Medien aus Verbindungen mit negativer dielektrischer Anisotropie. Bei den jeweiligen Arten von Medien (dielektrisch positiv bzw. dielektrisch negativ) werden typischer Weise allenfalls nennenswerte Mengen an dielektrisch neutralen Flüssigkristallverbindungen eingesetzt. Flüssigkristallverbindungen mit dem der dielektrischen Anisotropie des Medium entgegengesetzten Vorzeichen der dielektrischen Anisotropie werden in der Regel äußerst sparsam oder gar nicht eingesetzt.

Eine Ausnahme bilden hier flüssigkristalline Medien für MIM-Anzeigen (Englisch "metal insultator metal", Simmons, J.G., Phys. Rev. 155 Nr. 3, S. 657-660 und Niwa, J.G. et al., SID 84 Digest, S. 304-307, Juni 1984) bei denen die Flüssigkristallmedien mit einer aktiven Matrix aus Dünnfilmtransistoren angesteuert werden. Bei dieser Art von Ansteuerung, welche die nicht lineare Kennlinie der Diodenschaltung ausnutzt, kann im Gegensatz zu TFT-Anzeigen kein Speicherkondensator gemeinsam mit den Elektroden der Flüssigkristallanzeigeelemente (Pixeln) aufgeladen werden. Somit ist zur Verminderung des Effekts des Spannungsabfalls während des Ansteuerzyklus ein möglichst großer Grundwert der Dielektrizitätskonstante erforderlich. Bei dielektrisch positiven Medien, wie sie z.B. bei MIM-TN-Anzeigen eingesetzt werden, muß also die Dielektrizitätskonstante senkrecht zur Molekülachse (ε_{⊥}) möglichst groß sein, da sie die Grundkapazität des Pixels bestimmt. Hierzu werden, wie z.B. in WO 93/01253, EP 0 663 502 und DE 195 21 483 beschrieben, in den dielektrisch positiven Flüssigkristallmedien, neben dielektrisch positiven Verbindungen, gleichzeitig auch Verbindungen mit negativer dielektrischer Anisotropie eingesetzt.

Eine weitere Ausnahme bilden STN-Anzeigen in denen z.B. nach DE 41 00 287 dielektrisch positive Flüssigkristallmedien mit dielektrisch negativen Flüssigkristallverbindungen eingesetzt werden um die Steilheit der elektrooptischen Kennlinie zu erhöhen.

Die Bildpunkte der Flüssigkristallanzeigen können direkt angesteuert werden, zeitsequentiell, also im Zeitmultiplexverfahren oder mittels einer Matrix von aktiven Elementen mit nichtlinearen elektrischen Kennlinien angesteuert werden.

Die bislang gebräuchlichsten AMDs verwenden diskrete aktive elektronische Schaltelemente, wie z.B. dreipolige Schaltelemente wie MOS (Englisch "metal oxide silicon") Transistoren oder Dünnfilmtransistoren (TFTs von Englisch "thin film transistors") oder Varistoren oder 2-polige Schaltelemente wie z.B. MIM (Englisch "metal insulator metal") Dioden, Ringdioden oder "back to back"-Dioden. Bei den TFTs werden verschiedene Halbleitermaterialien, überwiegend Silizium, aber auch Cadmiumselenid, verwendet. Insbesondere wird amorphes Silizium oder polykristallines Silizium verwendet.

Gemäß der vorliegenden Anmeldung sind Flüssigkristallmedien mit positiver dielektrischer Anisotropie (Δε > 0) bevorzugt.

NOVAROLI ET AL.: "Human recombinat monoamine oxidase ...", BIOORGANIC & MEDICAL CHEMISTRY, ELSEVIER SCIENCE LTD, GB, Bd. 13, Nr. 22m 15. November 2005 (2005-11-15), Seiten 6212-6217 und KIM ET AL.:"ESTROGEN receptor Ligands. 12. Synthesis of the Major", ORGANIC LETTERS, Bd. 7, Nr. 3, Mai 2005 (2005-05), Seiten 411-414 offenbaren Einzelverbindungen mit kondensierten Ringen.

DE 10 2004 004228 A1 offenbart Verbindungen mit kondensierten Ringen, die für den Einsatz in dielktrisch negativen Flüssigkristallmischungen vorgesehen sind.

1,2,3,4,4a,9,10,10a-Octahydro-phenanthrene zur Verwendung in Flüssigkristallmischungen sind aus EP 1 162 185 B1 bekannt. Der Erfindung lag die Aufgabe zugrunde, neue Komponenten für Flüssigkristallmischungen bereitzustellen, um den unterschiedlichen Anforderungen der Displayhersteller gerecht zu werden. Insbesondere werden Flüssigkristallmischungen benötigt, die aufgrund einer hohen dielektrischen Anisotropie die Herstellung von Flüssigkristallanzeigen mit besonders niedriger Schaltspannung ermöglichen. Somit ist ersichtlich, dass sowohl ein Bedarf an weiteren mesogenen Verbindungen, als auch insbesondere ein Bedarf an Flüssigkristallmedien mit positiver dielektrischer Anisotropie, mit einem großen Wert der dielektrischen Anisotropie, einem der jeweiligen Anwendung entsprechenden Wert der optischen Anisotropie (Δn), einer breiten nematischen Phase, guter Stabilität gegen UV, Wärme und elektrische Spannung und einer niedrigen Rotationsviskosität besteht.

Dies wird erreicht, durch Einsatz der erfindungsgemäßen mesogenen Verbindungen der Formel I. 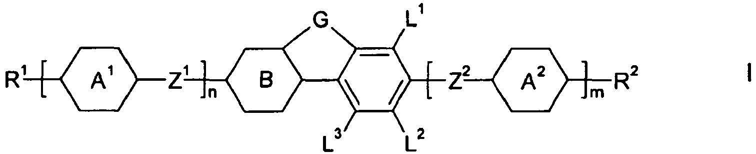 worin
das Strukturelement 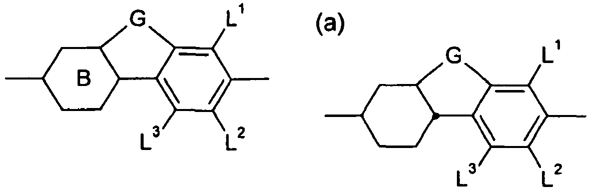 oder 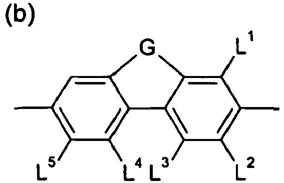 sowie

| | |
|---|---|
| G | -CO-O-, -CH₂-O-, -CF₂-O-, -O-CO-, -CH₂-O- oder -O-CF₂-, bevorzugt CH₂O |
| 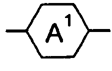 | |
| und | |
| 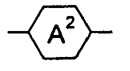 | , jeweils unabhängig voneinander, und wenn mehrfach vorhanden auch diese unabhängig voneinander, |

(a) einen trans-1,4-Cyclohexylenrest, worin auch eine oder zwei nicht benachbarte CH₂-Gruppen durch -O- und/oder -S-ersetzt sein können,
(b) einen 1,4-Cyclohexenylenrest,
(c) einen 1,4-Phenylenrest, worin auch eine oder zwei nicht benachbarte CH-Gruppen durch N ersetzt sein können oder
(d) Naphtalin-2,6-diyl, Decahydronaphthalin-2,6-diyl und 1,2,3,4-Tetrahydronaphthalin-2,6-diyl,
(e) einen Rest ausgewählt aus der Gruppe 1,4-Bicyclo-[2,2,2]-octylen, 1,3-Bicyclo-[1,1,1]-pentylen oder Spiro-[3,3]-heptan-2,6-diyl, 1,3-Cyclobutylen,
   wobei in
   (a) und (b) eine oder mehrere -CH₂- Gruppen, unabhängig voneinander, jeweils durch eine -CHF- oder eine -CF₂-Gruppe ersetzt sein können und in
   (c) und (d) eine oder mehrere -CH= Gruppen, unabhängig voneinander, jeweils durch eine -CF=, eine -C(CN)=, eine -C(CH₃)=, eine -C(CH₂F)=, eine -C(CHF₂)=, eine -C(O-CH₃)=, eine -C(O-CHF₂)= oder eine -C(O-CF₃)=Gruppe, bevorzugt eine -CF= Gruppe, ersetzt sein können und bevorzugt 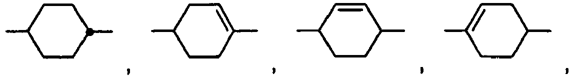 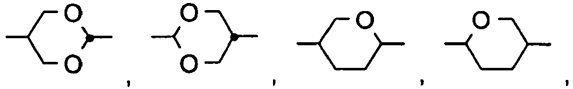 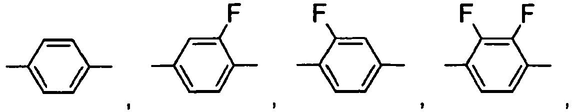 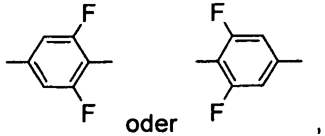
      - L¹ und L²: F,
      - L³: H,
      - L⁴ und L⁵: , jeweils unabhängig voneinander, H oder F,
      - R¹ und R²: , jeweils unabhängig voneinander, Alkyl und Alkoxy mit 1 bis 15 C-Atomen, Alkoxyalkyl, Alkenyl oder Alkenyloxy mit 2 bis 15 C-Atomen, Alkinyl oder Alkinyloxy mit 2 bis 15 C-Atomen, H, Halogen, -CN, -SCN, -NCS, -OCN, -SF₅, -CF₃, -CHF₂, -CH₂F, -OCF₃, -OCHF₂, eine einfach durch -CN oder -CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 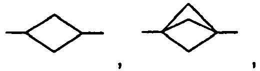 -CO-, -CO-O-, -O-CO- oder -O-CO-O- so ersetzt sein können, dass weder O-noch S-Atome direkt miteinander verknüpft sind, bevorzugt einer von
      - R¹ und R²: Alkyl und Alkoxy mit 1 bis 12 C-Atomen, Alkoxyalkyl, Alkenyl oder Alkenyloxy mit 2 bis 12 C-Atomen und der andere, unabhängig vom ersten, Halogen, -CN, -SCN, -NCS, -OCN, -SF₅, -CF₃, -CHF₂, -CH₂F, -OCF₃, -OCHF₂,
      - Z¹ und Z²: , jeweils unabhängig voneinander, und wenn mehrfach vorhanden auch diese unabhängig voneinander, -CH₂-CH₂-, -(CH₂)₄-, -CF₂-CF₂-, -CF₂-CH₂-, -CH₂-CF₂-, -CH=CH-, -CF=CF-, -CF=CH-, -CH=CF-, -C≡C-, -CO-O-, -O-CO-, -CH₂-O-, -O-CH₂-, -CF₂-O-, -O-CF₂-, oder eine Kombination von zweien dieser Gruppen, wobei keine zwei O-Atome miteinander verbunden sind, oder eine Einfachbindung, bevorzugt -(CH₂)₄-, -CH₂-CH₂-, -CF₂-CF₂-, -CH=CH-, -CF=CF-, -C≡C-, -CH₂-O-, -CF₂-O- oder eine Einfachbindung, besonders bevorzugt -CH₂-O-, -CH₂-CH₂-, -CF₂-CF₂-, -CF=CF-, -CF₂-O-oder eine Einfachbindung und
      - n und m: jeweils 0, 1 oder 2, wobei
      - n + m: 0, 1, 2 oder 3, bevorzugt 0, 1 oder 2, besonders bevorzugt 0 oder 1,
bedeuten.

Besonders bevorzugt sind Flüssigkristallverbindungen der Formel I mit positiver dielektrischer Anisotropie.

Bevorzugt sind weiterhin Verbindungen der Formel I bei denen bei den Verbindungen mit dem Strukturelement (a) einer von
- L¹ und L²: F bedeutet oder beide F bedeuten
bei den Verbindungen mit dem Strukturelement (b) einer oder mehrere, bevorzugt zwei oder drei, von
- L¹, L² und: L⁴ F bedeuten.

Bevorzugt sind weiterhin die Verbindungen der Formel I, die das Strukturelement (a) enthalten.

Ganz besonders bevorzugt sind Flüssigkristallverbindungen der Formel I der Unterformeln I-A und I-B, besonders I-A, 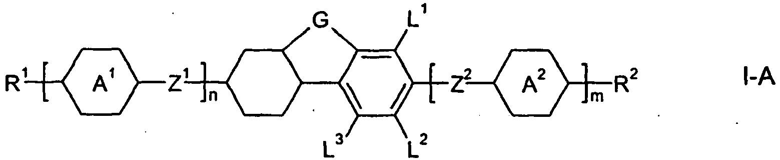 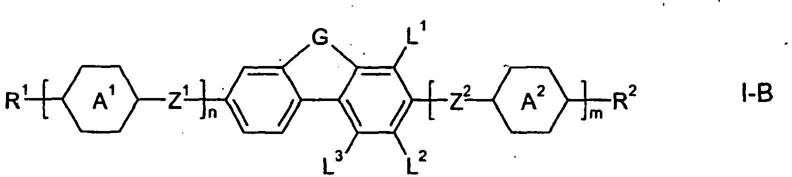 worin die Parameter die oben unter Formel I gegebene Bedeutung haben und der zweite aromatische Ring in Formel I-B optional einfach oder mehrfach durch F substituiert sein kann, und bevorzugt einer oder beide von
- L¹ und L²: F bedeutet.

Ganz besonders bevorzugt sind Flüssigkristallverbindungen der Formel I der Unterformeln I-A1 bis I-A3 und I-B1 bis I-B3, besonders der Formeln I-A1 bis I-A3, 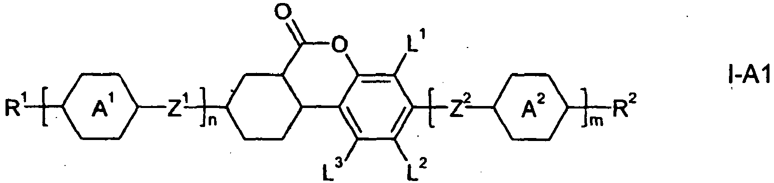 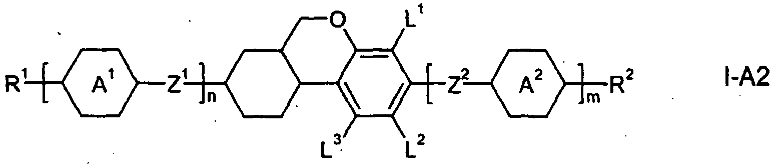 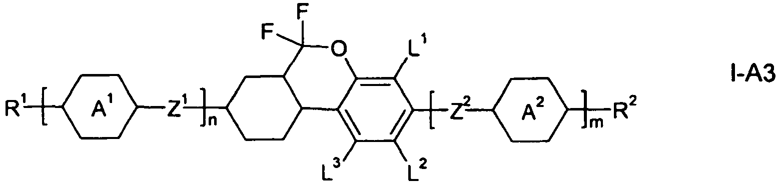 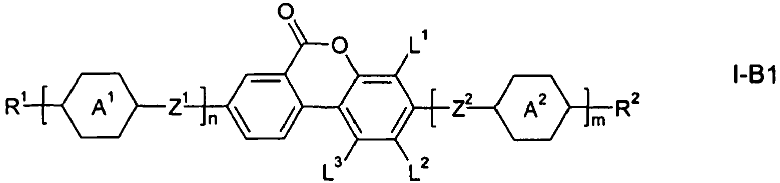 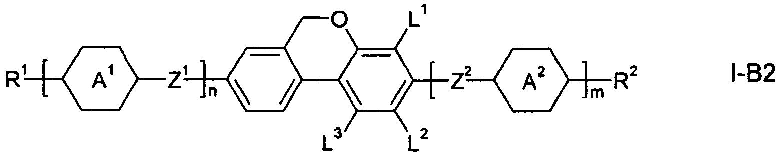 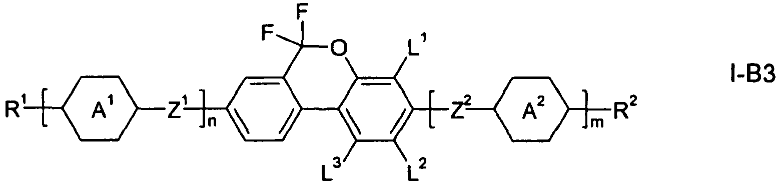 worin die Parameter die oben unter Formel I gegebene Bedeutung haben und der zweite aromatische Ring in den Formeln I-B1 bis I-B3 optional einfach oder mehrfach durch F substituiert sein kann.

Bevorzugt sind Verbindungen der Formel I, bevorzugt ausgewählt aus der Gruppe der Verbindungen der Formeln I-A1 bis I-A3 und I-B1 bis I-B3 bei denen
die Summe n + m 0 oder 1, bevorzugt 0, beträgt.

Eine bevorzugte Ausführungsform stellen die Verbindungen der Formel I, bei denen die Summe n + m 1 beträgt und bevorzugt
- m oder n: 1 ,

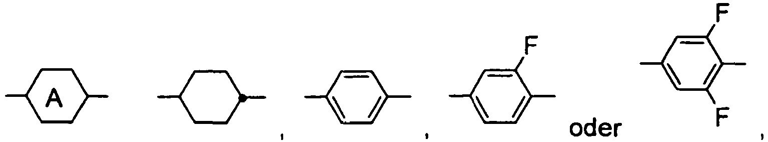

- Z: bevorzugt -(CH₂)₄-, -CH₂-CH₂-, -CF₂-CF₂-, -CH=CH-, -CF=CF-, -C≡C-, -CH₂-O-, -CF₂-O- oder eine Einfachbindung, besonders bevorzugt -CH₂-O-, -CH₂-CH₂-, -CF₂-CF₂-, -CF=CF-, -CF₂-O oder eine Einfachbindung
bedeuten und L, R¹ und R² die oben bei Formel I gegebene Bedeutung haben und L bevorzugt F bedeutet, dar.

Besonders bevorzugt sind Verbindungen der Formel I, bevorzugt ausgewählt aus der Gruppe der Verbindungen der Formeln I-A1 bis I-A3 und I-B1 bis I-B3 bei denen
- n und m: beide 0 bedeuten und
- L¹ bis L³, R¹ und R²: die oben bei der entsprechenden Formel gegebene Bedeutung haben.

Verbindungen der Formel I mit verzweigten Flügelgruppen R¹ und/oder R² können gelegentlich wegen einer besseren Löslichkeit in den üblichen flüssigkristallinen Basismaterialien von Bedeutung sein, insbesondere aber als chirale Dotierstoffe, wenn sie optisch aktiv sind. Smektische Verbindungen dieser Art eignen sich als Komponenten für ferroelektrische Materialien. Verbindungen der Formel I mit S_{A}-Phasen eignen sich beispielsweise für thermisch adressierte Displays.

Falls R¹ und/oder R² einen Alkylrest und/oder einen Alkoxyrest bedeutet, so kann dieser geradkettig oder verzweigt sein. Vorzugsweise ist er geradkettig, hat 2, 3, 4, 5, 6 oder 7 C-Atome und bedeutet demnach bevorzugt Ethyl, Propyl, Butyl, Pentyl, Hexyl, Heptyl, Ethoxy, Propoxy, Butoxy, Pentoxy, Hexoxy oder Heptoxy, ferner Methyl, Octyl, Nonyl, Decyl, Undecyl, Dodecyl, Tridecyl, Tetradecyl, Pentadecyl, Methoxy, Octoxy, Nonoxy, Decoxy, Undecoxy, Dodecoxy, Tridecoxy oder Tetradecoxy.

Oxaalkyl, bzw. Alkoxyalkyl bedeutet vorzugsweise geradkettiges 2-Oxapropyl (= Methoxymethyl), 2- (= Ethoxymethyl) oder 3-Oxabutyl (= 2-Methoxyethyl), 2-, 3- oder 4-Oxapentyl, 2-, 3-, 4- oder 5-Oxahexyl, 2-, 3-, 4-, 5- oder 6-Oxaheptyl, 2-, 3-, 4-, 5-, 6- oder 7-Oxaoctyl, 2-, 3-, 4-, 5-, 6-, 7- oder 8-Oxanonyl, 2-, 3-, 4-, 5-, 6-, 7-, 8- oder 9-Oxadecyl.

Falls R¹ und/oder R² einen Alkylrest bedeutet, in dem eine CH₂-Gruppe durch -CH=CH- ersetzt ist, so kann dieser geradkettig oder verzweigt sein. Vorzugsweise ist er geradkettig und hat 2 bis 10 C-Atome. Er bedeutet demnach besonders Vinyl, Prop-1-, oder Prop-2-enyl, But-1-, 2- oder But-3-enyl, Pent-1-, 2-, 3- oder Pent-4-enyl, Hex-1-, 2-, 3-, 4- oder Hex-5-enyl, Hept-1-, 2-, 3-, 4-, 5- oder Hept-6-enyl, Oct-1-, 2-, 3-, 4-, 5-, 6- oder Oct-7-enyl, Non-1-, 2-, 3-, 4-, 5-, 6-, 7- oder Non-8-enyl, Dec-1-, 2-, 3-, 4-, 5-, 6-, 7-, 8- oder Dec-9-enyl.

Falls R¹ und/oder R² einen Alkylrest bedeutet, in dem eine CH₂-Gruppe durch -O- und eine durch -CO- ersetzt ist, so sind diese bevorzugt benachbart. Somit beinhalten diese eine Acyloxygruppe -CO-O- oder eine Oxycarbonylgruppe -O-CO-. Vorzugsweise sind diese geradkettig und haben 2 bis 6 C-Atome. Sie bedeuten demnach besonders Acetyloxy, Propionyloxy, Butyryloxy, Pentanoyloxy, Hexanoyloxy, Acetyloxymethyl, Propionyloxymethyl, Butyryloxymethyl, Pentanoyloxymethyl, 2-Acetyloxyethyl, 2-Propionyloxyethyl, 2-Butyryloxyethyl, 3-Acetyloxypropyl, 3-Propionyloxypropyl, 4-Acetyloxybutyl, Methoxycarbonyl, Ethoxycarbonyl, Propoxycarbonyl, Butoxycarbonyl, Pentoxycarbonyl, Methoxycarbonylmethyl, Ethoxycarbonylmethyl, Propoxycarbonylmethyl, Butoxycarbonylmethyl, 2-(Methoxycarbonyl)ethyl, 2-(Ethoxycarbonyl)ethyl, 2-(Propoxycarbonyl)ethyl, 3-(Methoxycarbonyl)propyl, 3-(Ethoxycarbonyl)propyl, 4-(Methoxycarbonyl)-butyl.

Falls R¹ und/oder R² einen Alkylrest bedeutet, in dem eine CH₂-Gruppe durch unsubstituiertes oder substituiertes -CH=CH- und eine benachbarte CH₂-Gruppe durch CO oder CO-O oder O-CO ersetzt ist, so kann dieser geradkettig oder verzweigt sein. Vorzugsweise ist er geradkettig und hat 4 bis 13 C-Atome. Er bedeutet demnach besonders Acryloyloxymethyl, 2-Acryloyloxyethyl, 3-Acryloyloxypropyl, 4-Acryloyloxybutyl, 5-Acryloyloxypentyl, 6-Acryloyloxyhexyl, 7-Acryloyloxyheptyl, 8-Acryloyloxyoctyl, 9-Acryloyloxynonyl, 10-Acryloyloxydecyl, Methacryloyloxymethyl, 2-Methacryloyloxyethyl, 3-Methacryloyloxypropyl, 4-Methacryloyloxybutyl, 5-Methacryloyloxypentyl, 6-Methacryloyloxyhexyl, 7-Methacryloyloxyheptyl, 8-Methacryloyloxyoctyl, 9-Methacryloyloxynonyl.

Falls R¹ und/oder R² einen einfach durch CN oder CF₃ substituierten Alkyloder Alkenylrest bedeutet, so ist dieser Rest vorzugsweise geradkettig. Die Substitution durch CN oder CF₃ ist in beliebiger Position.

Falls R¹ und/oder R² einen mindestens einfach durch Halogen substituierten Alkyl- oder Alkenylrest bedeutet, so ist dieser Rest vorzugsweise geradkettig und Halogen ist vorzugsweise F oder Cl. Bei Mehrfachsubstitution ist Halogen vorzugsweise F. Die resultierenden Reste schließen auch perfluorierte Reste ein. Bei Einfachsubstitution kann der Fluor- oder Chlorsubstituent in beliebiger Position sein, vorzugsweise jedoch in ω-Position.

Verzweigte Gruppen enthalten in der Regel nicht mehr als eine Kettenverzweigung. Bevorzugte verzweigte Reste R sind Isopropyl, 2-Butyl (= 1-Methylpropyl), Isobutyl (= 2-Methylpropyl), 2-Methylbutyl, Isopentyl (= 3-Methylbutyl), 2-Methylpentyl, 3-Methylpentyl, 2-Ethylhexyl, 2-Propylpentyl, Isopropoxy, 2-Methylpropoxy, 2-Methylbutoxy, 3-Methylbutoxy, 2-Methylpentoxy, 3-Methylpentoxy, 2-Ethylhexoxy, 1-Methylhexoxy, 1-Methylheptoxy.

Falls R¹ und/oder R² einen Alkylrest darstellt, in dem zwei oder mehr CH₂-Guppen durch -O- und/oder -CO-O- ersetzt sind, so kann dieser geradkettig oder verzweigt sein. Vorzugsweise ist er verzweigt und hat 3 bis 12 C-Atome. Er bedeutet demnach besonders Bis-carboxy-methyl, 2,2-Bis-carboxy-ethyl, 3,3-Bis-carboxy-propyl, 4,4-Bis-carboxy-butyl, 5,5-Bis-carboxy-pentyl, 6,6-Bis-carboxy-hexyl, 7,7-Bis-carboxy-heptyl, 8,8-Biscarboxy-octyl, 9,9-Bis-carboxy-nonyl, 10,10-Bis-carboxy-decyl, Bis-(methoxycarbonyl)-methyl, 2,2-Bis-(methoxycarbonyl)-ethyl, 3,3-Bis-(methoxycarbonyl)-propyl, 4,4-Bis-(methoxycarbonyl)-butyl, 5,5-Bis-(methoxycarbonyl)-pentyl, 6,6-Bis-(methoxycarbonyl)-hexyl, 7,7-Bis-(methoxycarbonyl)-heptyl, 8,8-Bis-(methoxycarbonyl)-octyl, Bis(ethoxycarbonyl)-methyl, 2,2-Bis-(ethoxycarbonyl)-ethyl, 3,3-Bis-(ethoxycarbonyl)-propyl, 4,4-Bis-(ethoxycarbonyl)-butyl, 5,5-Bis(ethoxycarbonyl)-hexyl.

Insbesondere bevorzugt sind Verbindungen der Formel I bei denen n = 0 oder 1 und m = 0 sowie R¹ Methyl, Ethyl, Propyl, Butyl, Pentyl, Vinyl, 1 E-Propenyl, 1 E-Butenyl oder 1 E-Pentenyl bedeutet so wie diese Verbindungen enthaltende Medien. Insbesondere bevorzugt von diesen Verbindungen sind die durch Alkyl substituierten Verbindungen eingesetzt.
Die Verbindungen der Formel I können aufgrund asymmetrisch substituierter Kohlenstoffatome im Ring B als Stereoisomere vorliegen. Gegenstand der Erfindung sind sämtliche Isomere, sowohl in reiner Form, als Racemat und auch als Mischung von Diastereomeren oder Enantiomeren. Optisch aktive Verbindungen der Formel I können auch als Dotierstoffe in Flüssigkristallmischungen verwendet werden.

Die Synthese der Verbindungen der Formel I (s. Schemata Ia bis Ic und II bis IX) erfolgt nach den in der Literatur beschriebenen Verfahren (Houben Weyl, Methoden der Organischen Chemie, Georg Thieme Verlag, Stuttgart, New York, 4. Aufl. 1993. Zu Schema II s.a. DE 10 2004 004 228 (A) sowie Taugerbeck, M. Klasen-Memmer, Anmeldung Aktenzeichen 10 2005 031 554.2)

In den folgenden Schemata werden die Verbindungen der Formel I kurz als Verbindungen **1** bezeichnet. Die Verbindungen **1b** und **1c** sind hierbei aus den Lactonen **1a** zugänglich. So erhält man **1b** entweder direkt durch Reduktion von **1a** mit Natriumborhydrid in Gegenwart von Bortrifluorid oder in zwei Stufen z. B. durch Reduktion von **1a** zum Lactol **2** und anschließendes Behandeln mit Triethylsilan in Gegenwart von Bortrifluorid, oder durch Reduktion von **1a** zum Diol **3** und anschließende Veretherung, z. B. durch Behandeln mit Säuren oder durch Mitsunobu-Reaktion mit Triphenylphosphin und Azodicabonsäurediethylester (siehe Schemata Ia bis Ic). 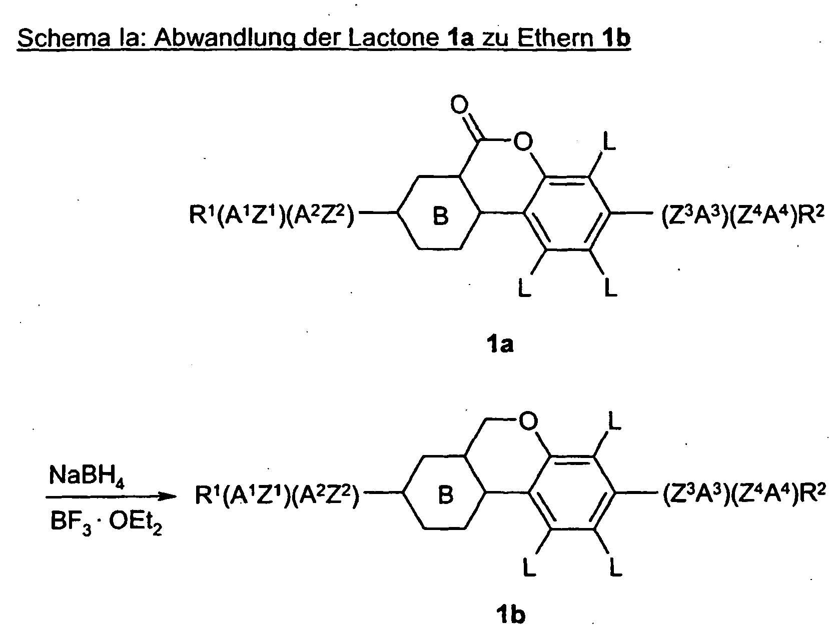 , worin, wie in den folgenden Schemata, wenn nicht explizit anders angegeben,
- R¹ und R²,: jeweils unabhängig voneinander, die oben bei Formel I für R¹ bzw. R² und die anderen Parameter jeweils die entsprechenden oben bei Formel I angegebenen Bedeutungen haben.

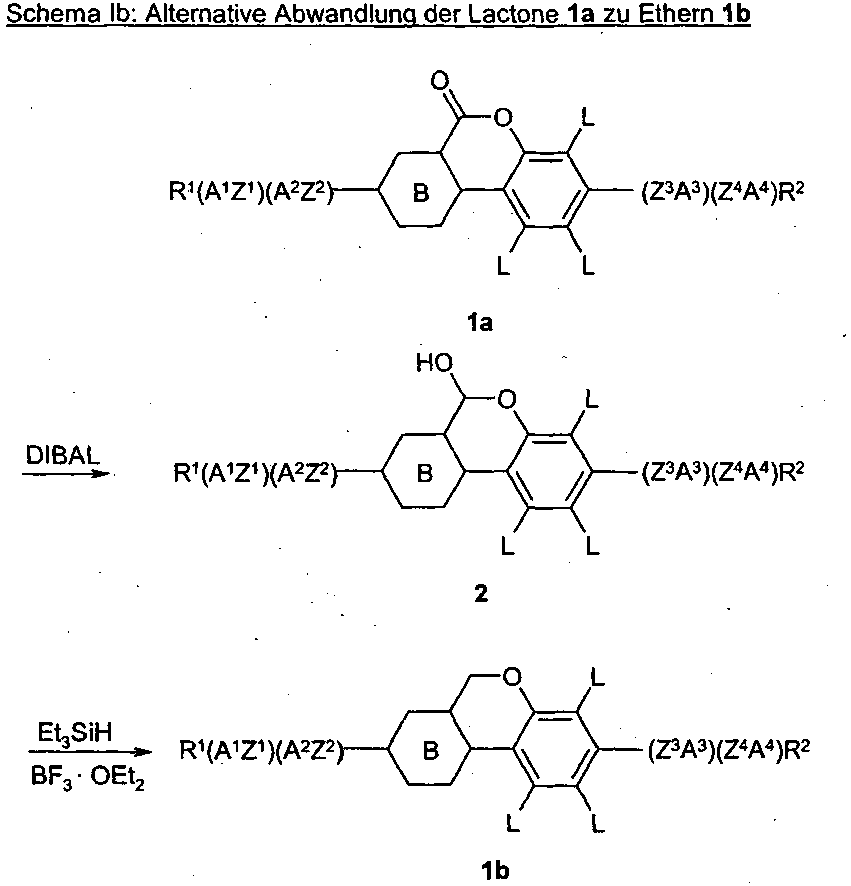

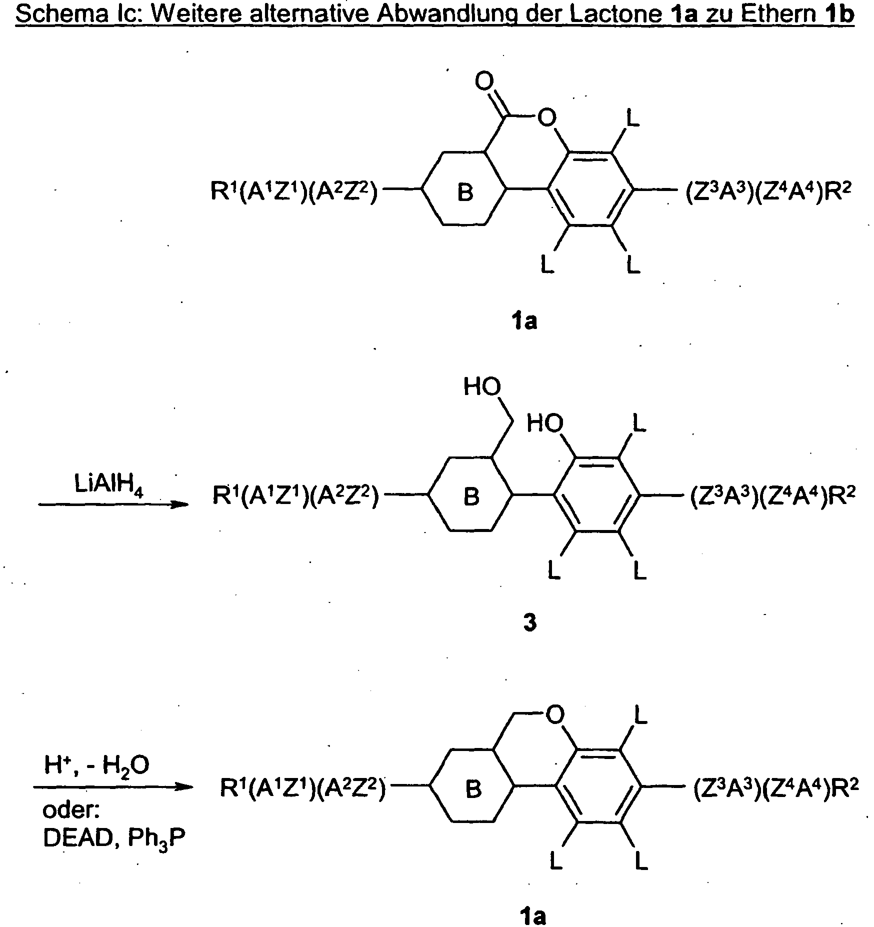

Die Difluorether **1c** erhält man z. B. entweder durch Umsetzung der Lactone **1a** mit Lawessons Reagenz zu **4** und anschließende Behandlung mit DAST oder mit NBS in Gegenwart von Ohlas Reagenz (W. H. Bunnelle, B. R. McKinnis, B.A. Narayanan, J. Org. Chem. 1990, 55, S. 768-770) (siehe Schema II), oder in Analogie zu dem in A. Taugerbeck, M. Klasen-Memmer, Anmeldung Aktenzeichen 10 2005 031 554.2 beschriebenen Verfahren durch Fluorodesulfurierung von Dithioorthoestern vom Typ **5** mit einem Oxidationsmittel wie z.B. Brom, NBS, DBH u.a. in Gegenwart einer Fluoridionenquelle wie HF-Pyridinkomplex, Triethylamin-trishydrogenfluorid, etc. (siehe Schema III). 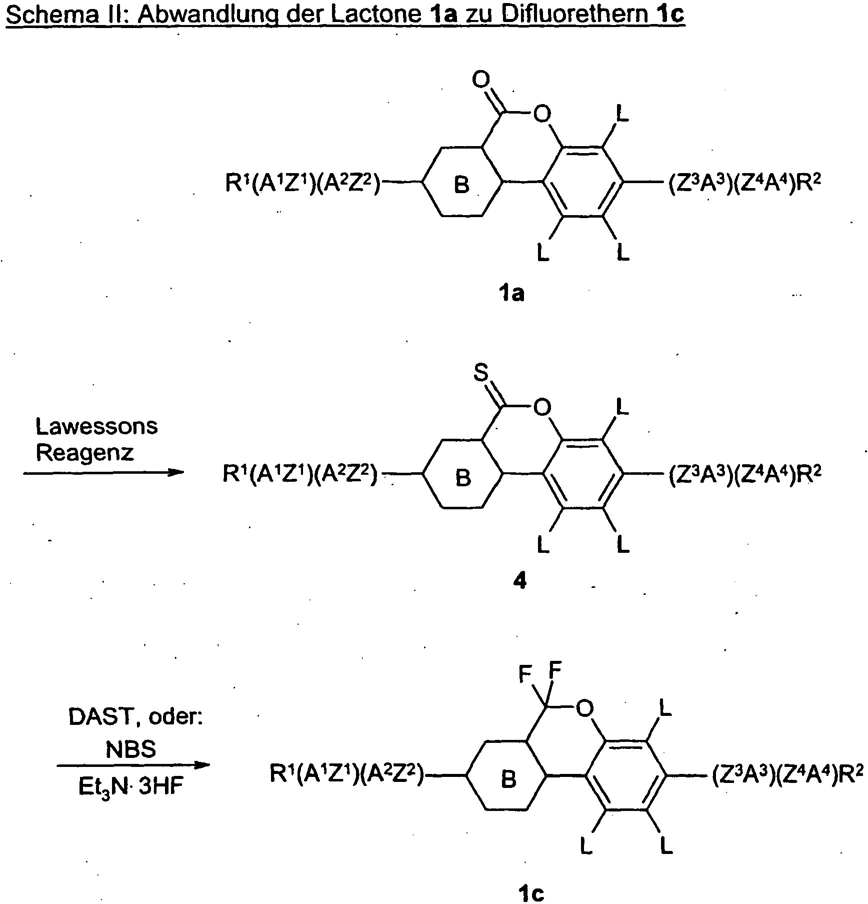 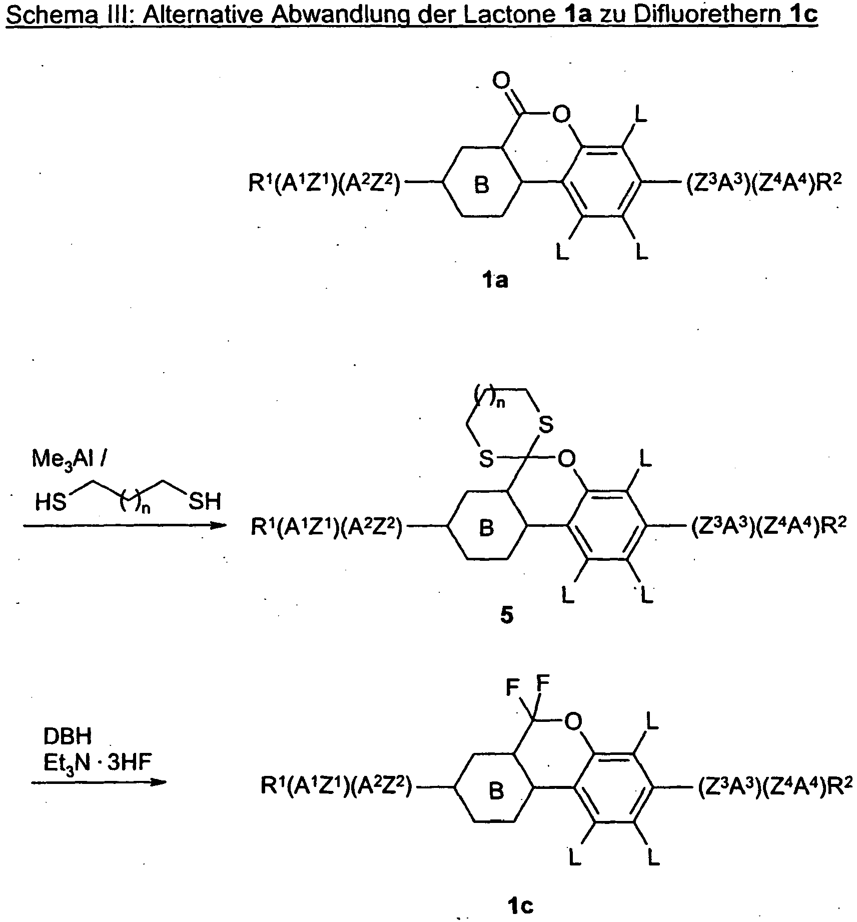 worin n = 0 oder 1 bedeutet.

Die Herstellung der Lactone **1a** kann gemäß S. Sethna, R. Phadke, Org. React. 1953, 7, S. 1 durch Pechmann-Kondensation von Phenolderivaten oder Resorcinen mit β-Ketoestern vom Typ 6 (V. H. Wallingford, A. H. Homeyer, D. M. Jones, J. Am. Chem. Soc. 1941, 63, S. 2252-2254) und nachfolgende Hydrierung (Schema IV) erfolgen.

Eine alternative Reduktion der Verbindungen **8** mit Lithium in Ammoniak ist in D. J. Collins, A. G. Ghingran, S. B. Rutschmann, Aust. J. Chem. 1989, 42, S. 1769-1784 beschrieben.

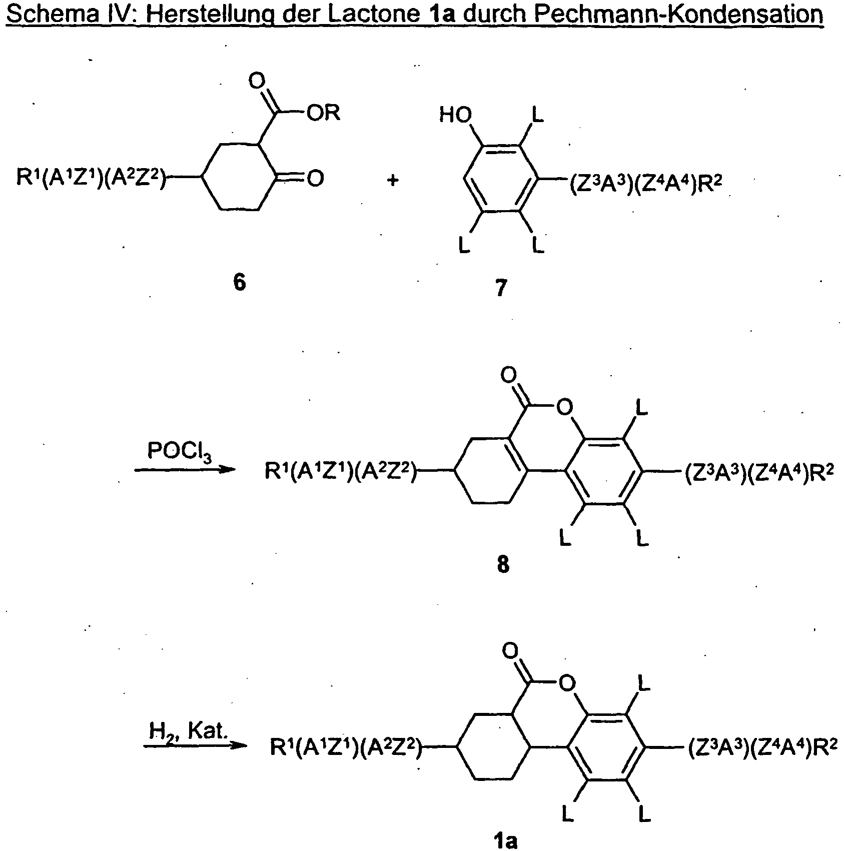

Die Verbindungen **8** sind auch nach P. Sellès, U. Mueller, Org. Lett. 2004, 6, S. 277-279 durch Suzuki-Kupplung aus Enoltriflaten 9 erhältlich (siehe Schema V). Die Verbindungen **9** können aus den oben beschriebenen Ketoestern **6** durch Behandeln mit Trifluormethansulfonsäureanhydrid in Gegenwart einer Base wie z.B. Collidin erhalten werden (E. Piers, H. L. A.Tse, Tetrahedron Lett. 1984, 25, 3155-3158). Die Boronsäuren **10** sind z.B. Aus den entsprechenden, in A. Taugerbeck, M. Klasen-Memmer, DE102004004228 beschriebenen Alkylbromiden durch Brom-Lithium-Austausch und anschließende Umsetzung mit Trimethylborat zugänglich.

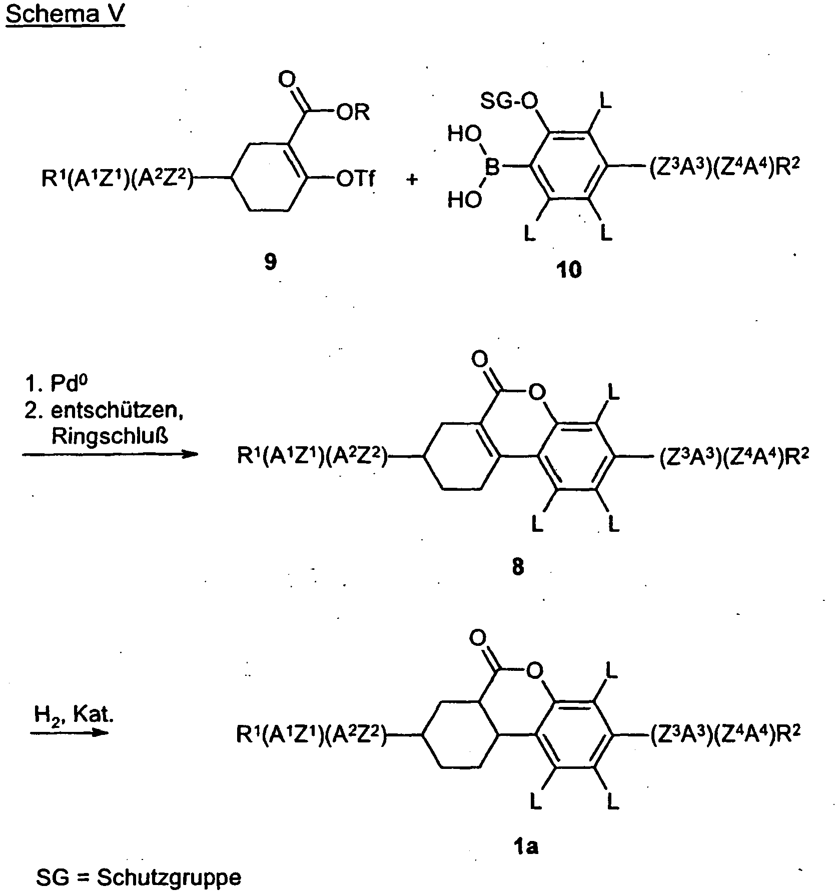

Die Verbindungen **1a** fallen nach Hydrierung als Isomerengemisch an, das durch die üblichen Verfahren, Kristallisation und/oder Chromatographie getrennt werden kann. Verbindungen mit 6a*R**,8*R**,10a*S**-Konfiguration können gemäß Schema VI in zwei zusätzlichen Syntheseschritten und nach D. J. Collins, A. G. Ghingran, S. B. Rutschmann, Aust. J. Chem. 1989, 42, S. 1769-1784 durch basenkatalysierte lsomerisierung erhalten werden, wobei es vorteilhaft sein kann, in Analogie zu J. M. Fevig et al., Bioorg. Med. Chem. Lett. 1996, 6, S. 295-300 den Lactonring zunächst durch Verseifen zu öffnen und nach erfolgter basenkatalysierter Isomerisierung wieder zu schließen.

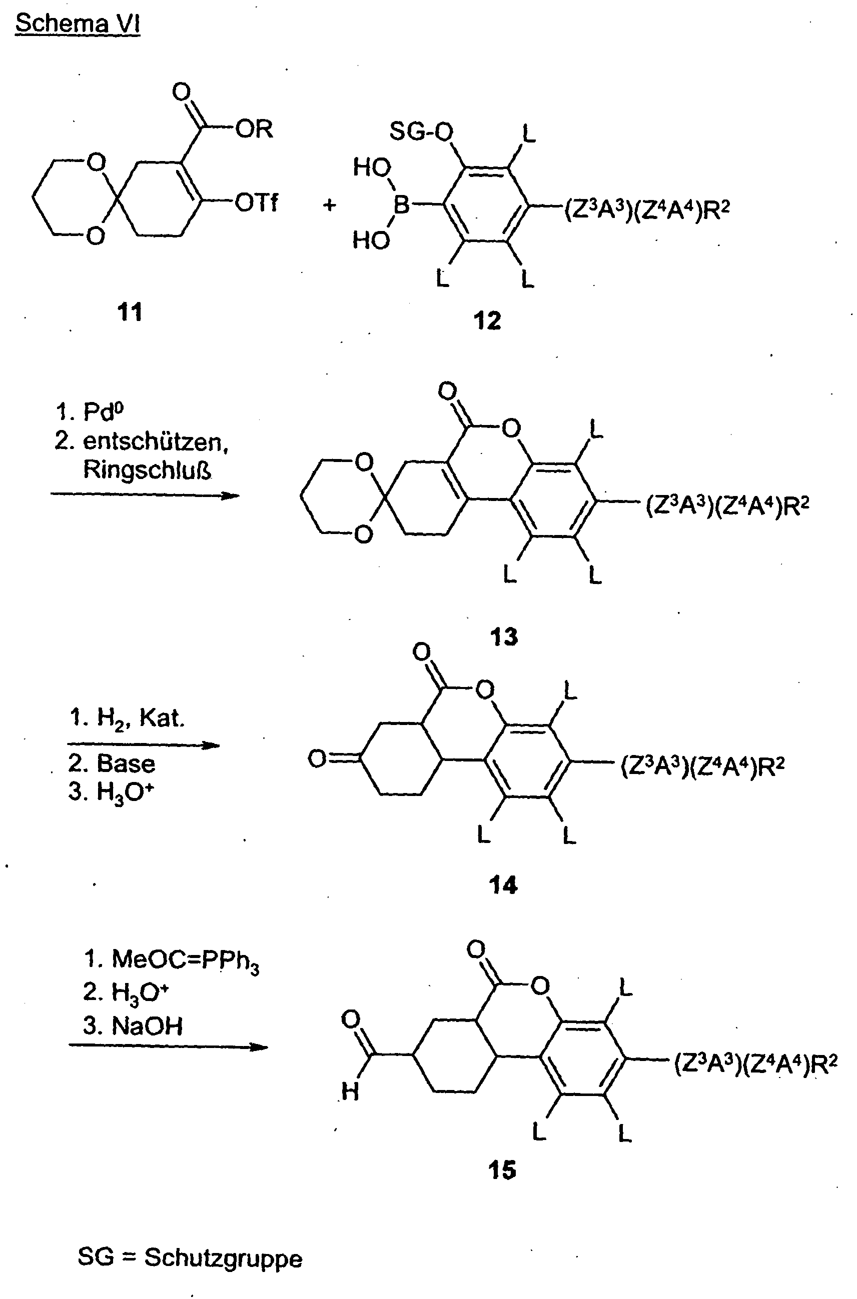

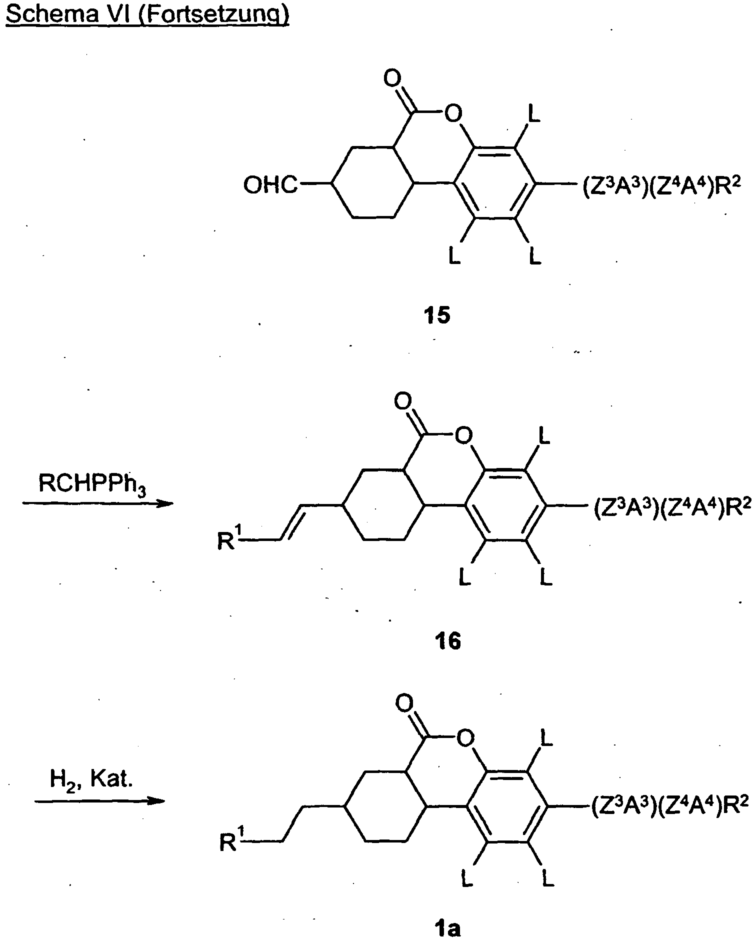

In Analogie zu der in Schema IV dargestellten Synthese lassen sich höher ungesättigte oder aromatische Verbindungen **1a** erhalten (siehe Schema VII). Ein entsprechender Zugang zu dielektrisch negativen Verbindungen ist in A. Taugerbeck, M. Klasen-Memmer, Anmeldung Aktenzeichen DE 10 2005 031 554.2 offengelegt.

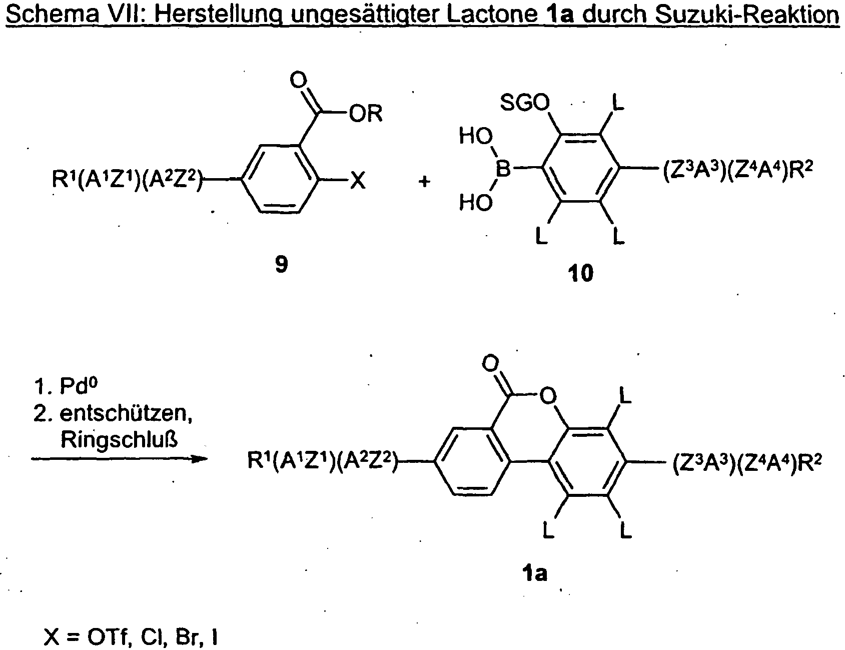

Eine alternative Synthesestrategie ist in den Schemata VIII und IX dargestellt, wobei ausgehend von in geeigneter Weise substituierten Vorstufen 9 oder 17 zunachst die Ether- oder Esterfunktion gebildet wird und in einem zweiten Schritt das Biphenylsytem durch z. B. Suzuki-Kupplung (Schema VIII) oder Heck-Reaktion (Schema IX) aufgebaut wird.

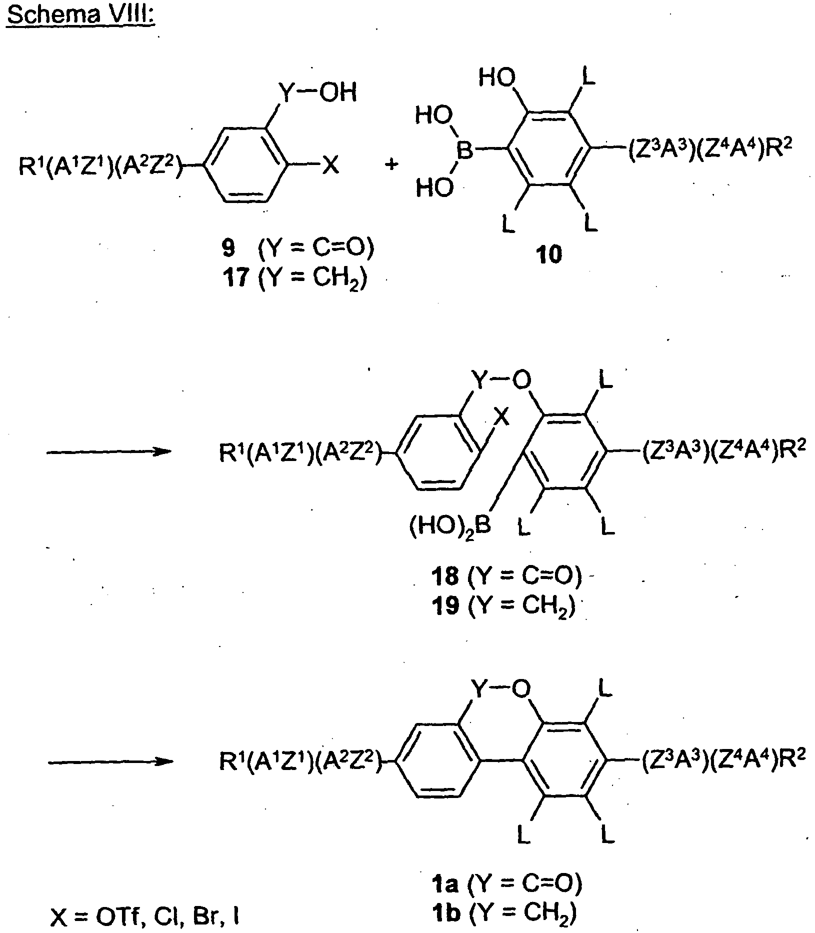

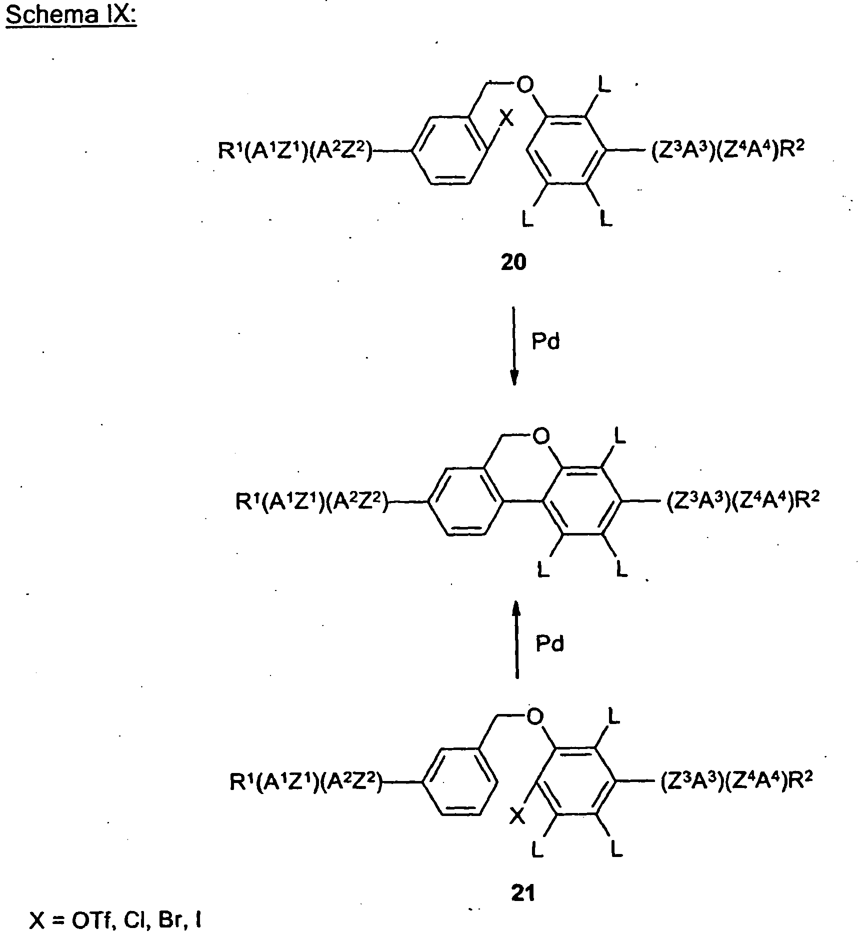

Im Folgenden werden Beispiele für Strukturen bevorzugter Verbindungen der Formel I und entsprechender Vergleichsverbindungen gegeben, worin R die unter Formel I für R¹ gegebene Bedeutung hat und bevorzugt Alkyl mit 1 bis 12 C-Atomen, besonders bevorzugt mit 1 bis7 C-Atomen oder Alkenyl mit 2 bis 7 C-Atomen und ganz besonders bevorzugt n-Alkyl, inklusive Methyl, oder 1-E-Alkenyl, inklusive Vinyl, bedeutet.

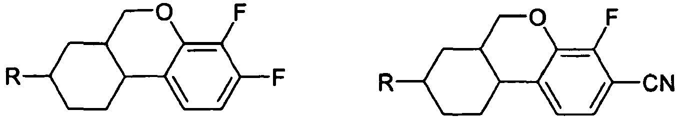

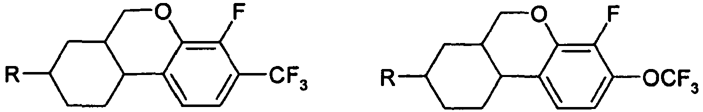

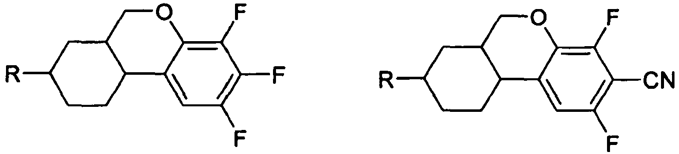

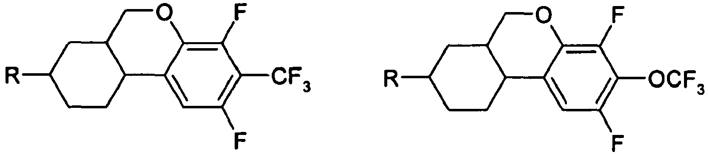

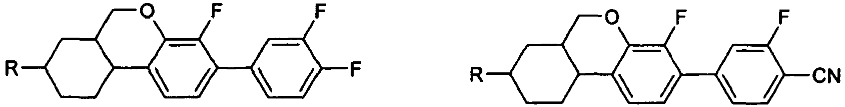

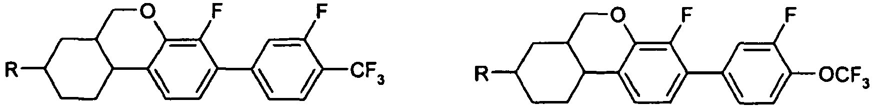

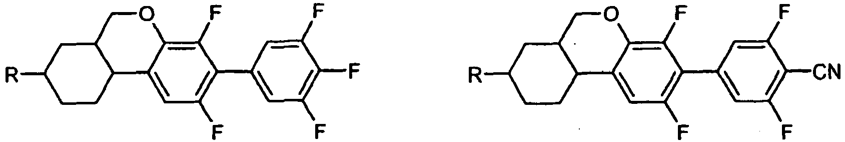

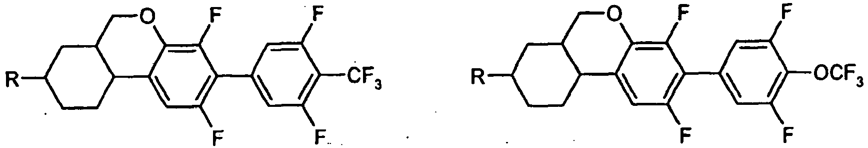

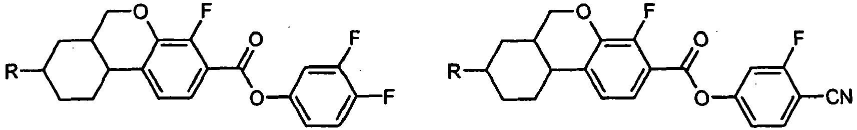

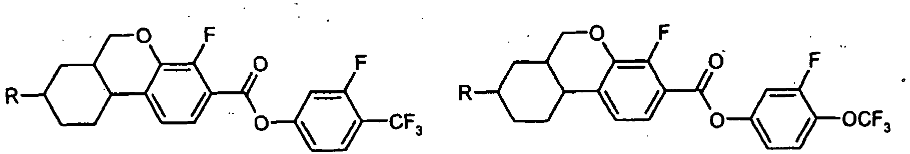

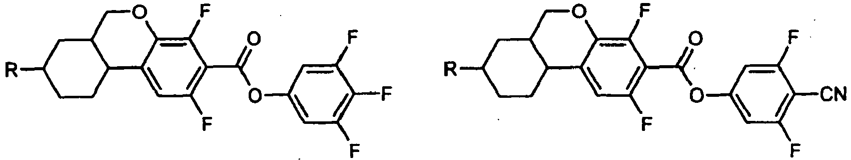

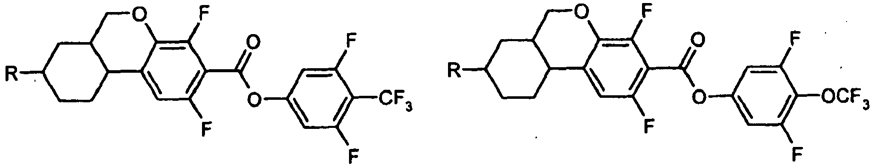

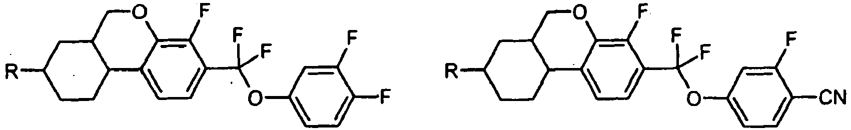

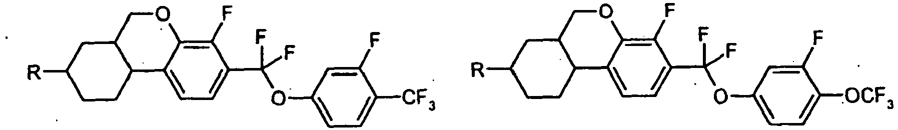

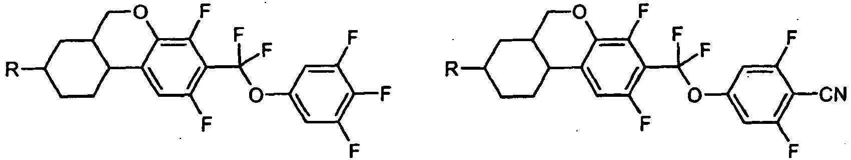

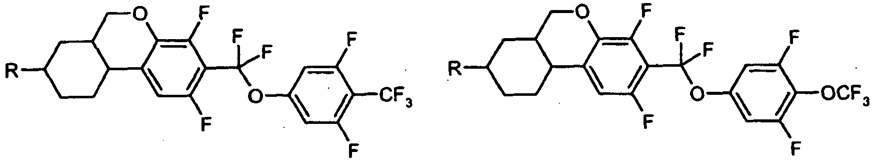

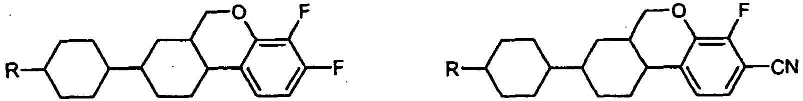

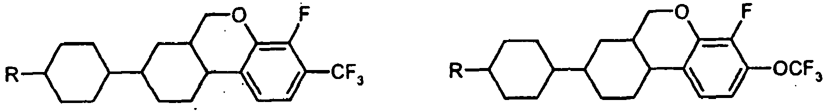

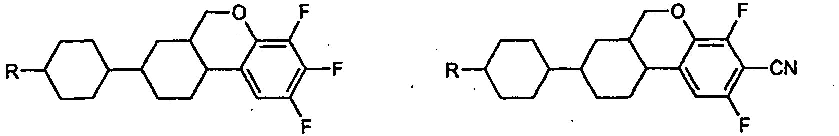

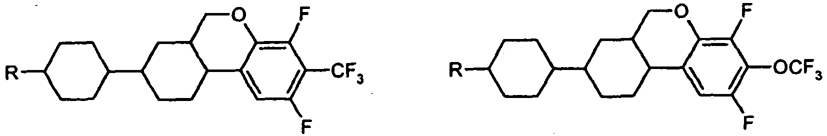

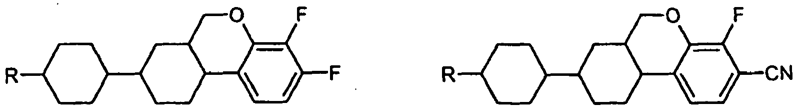

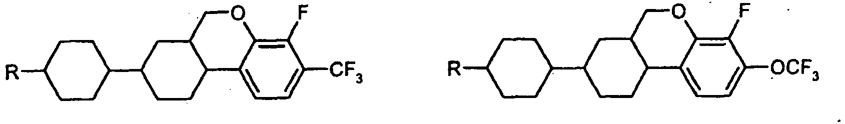

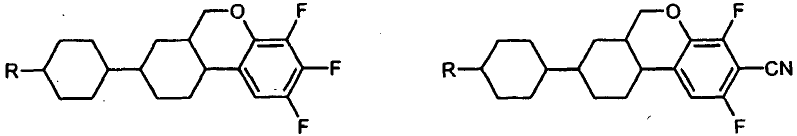

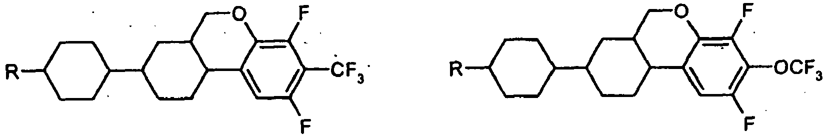

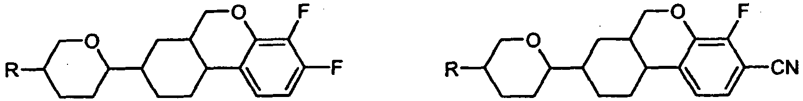

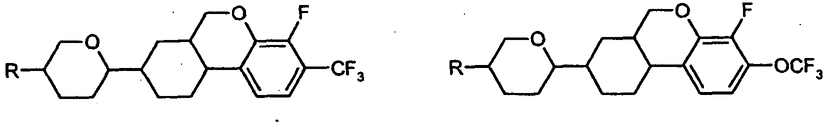

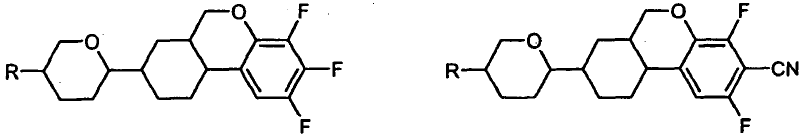

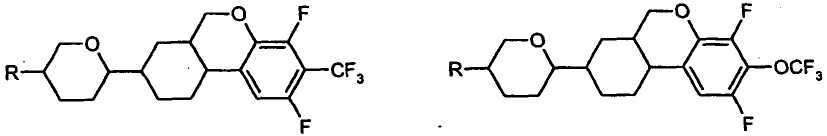

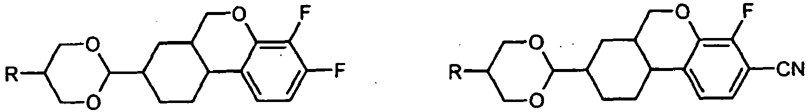

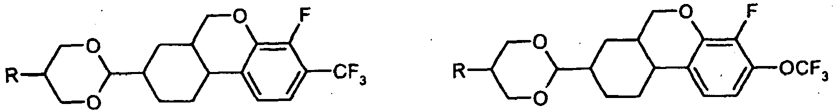

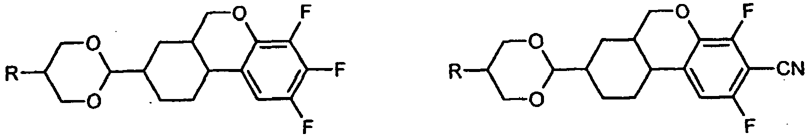

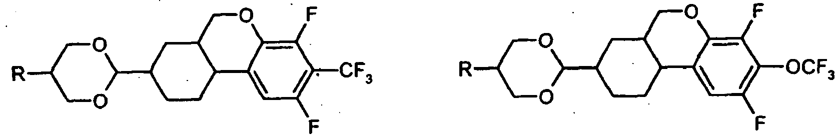

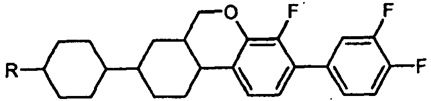

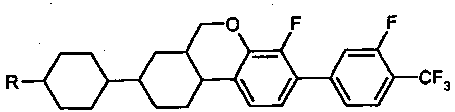

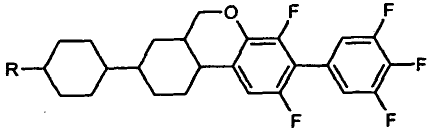

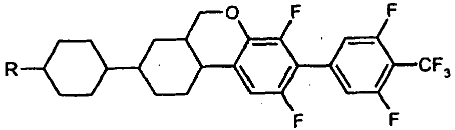

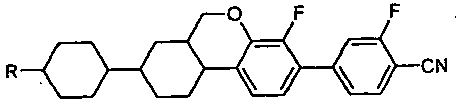

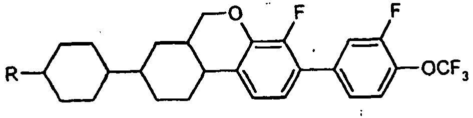

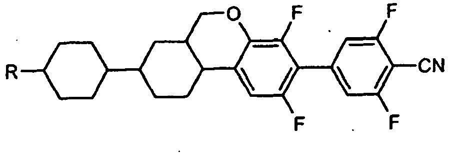

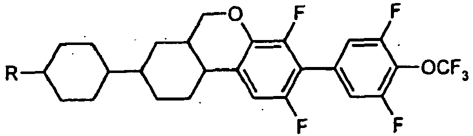

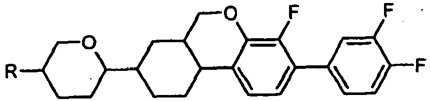

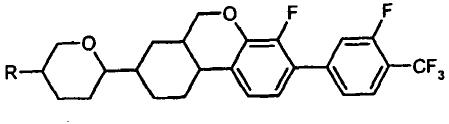

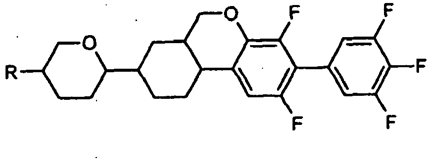

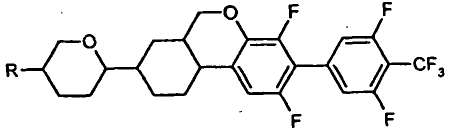

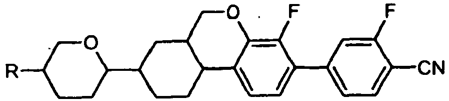

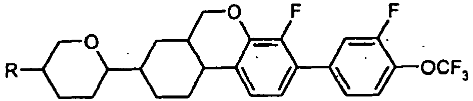

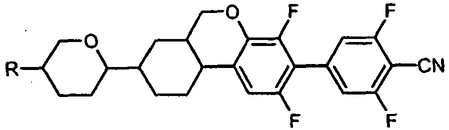

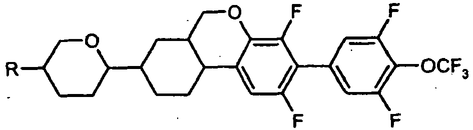

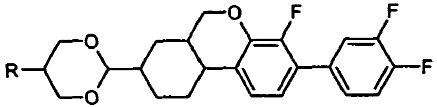

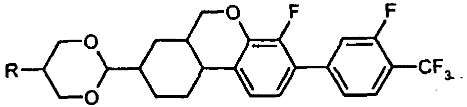

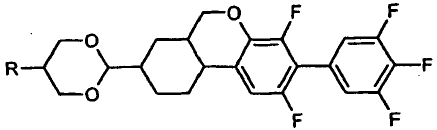

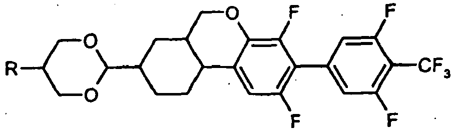

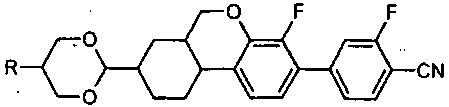

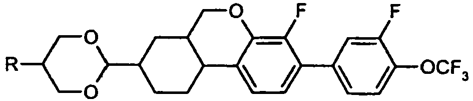

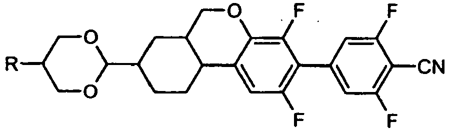

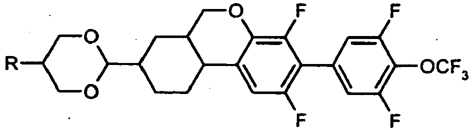

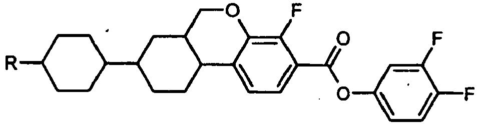

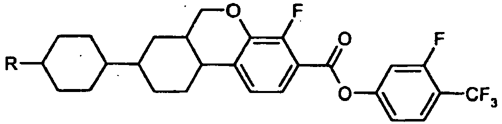

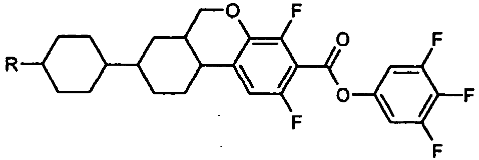

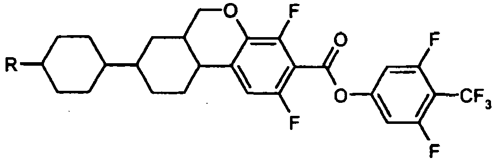

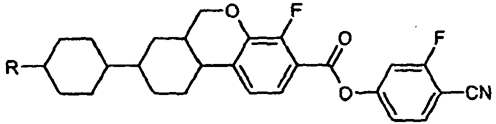

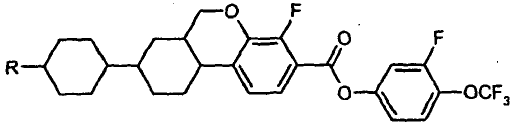

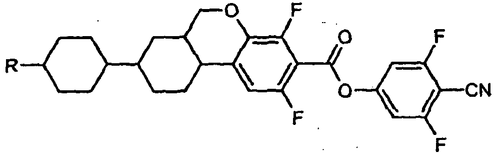

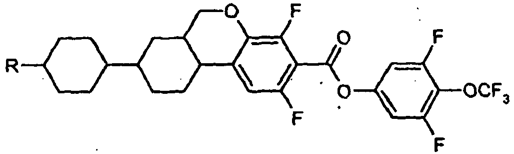

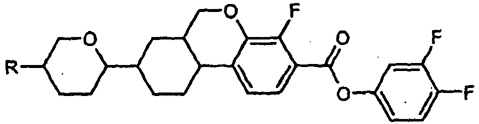

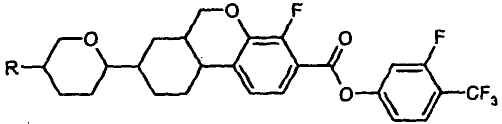

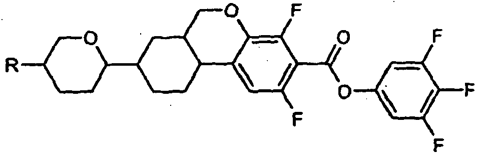

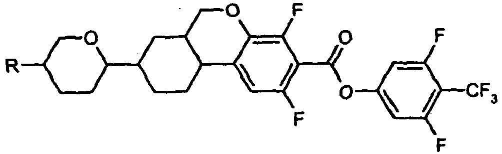

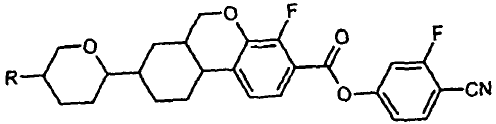

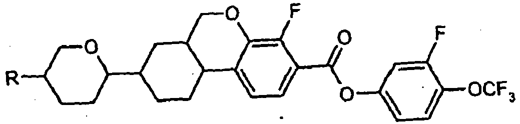

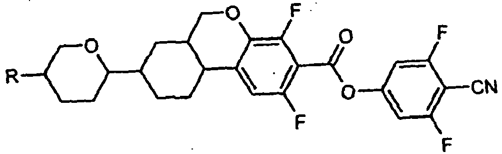

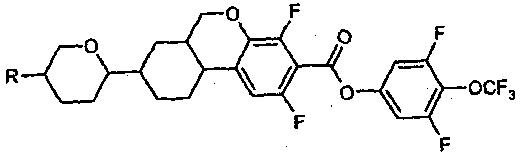

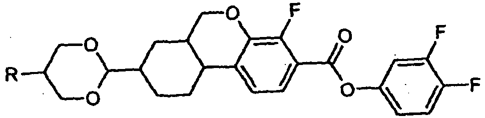

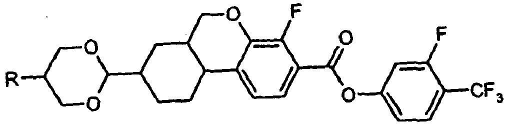

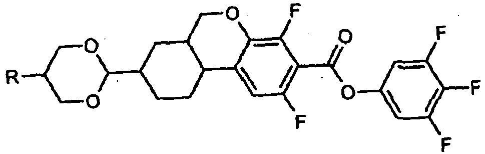

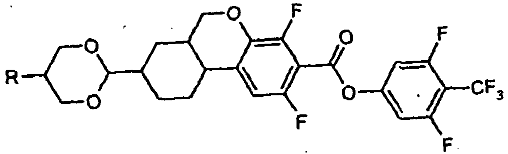

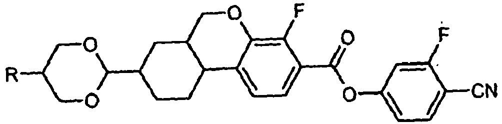

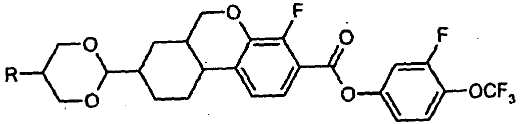

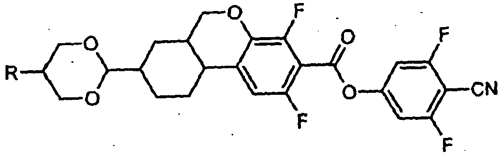

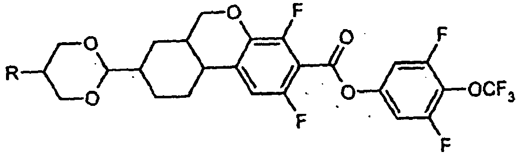

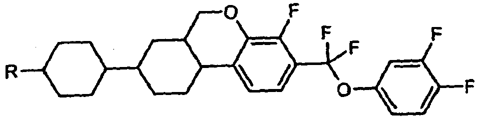

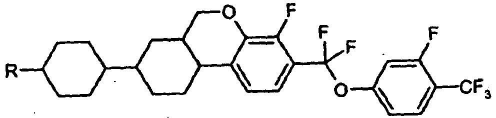

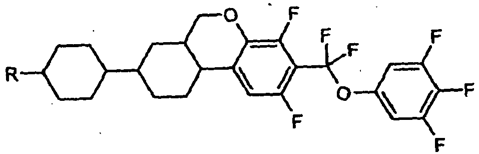

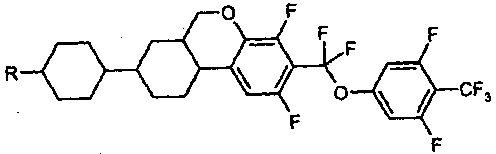

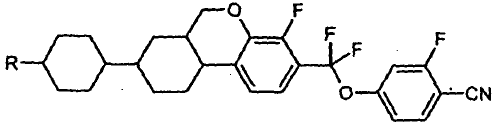

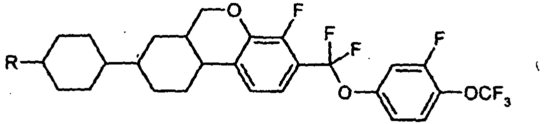

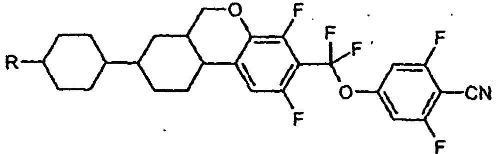

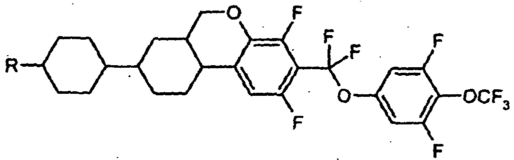

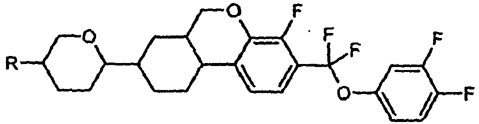

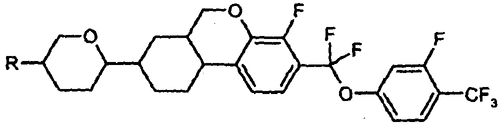

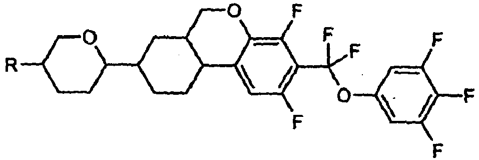

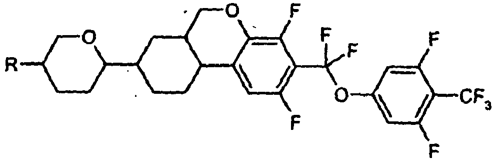

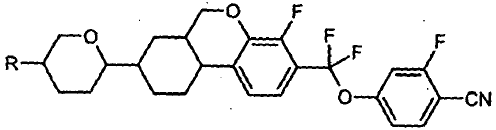

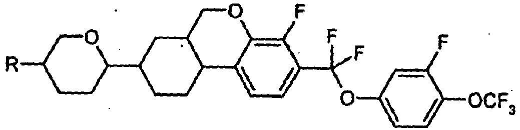

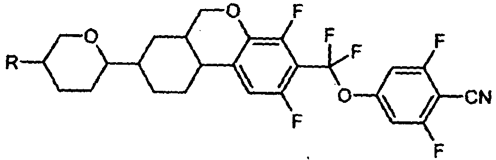

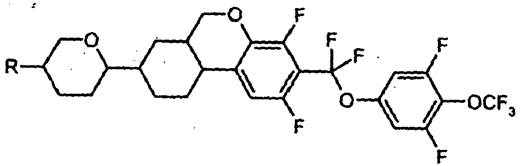

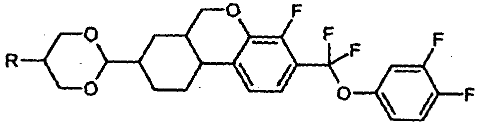

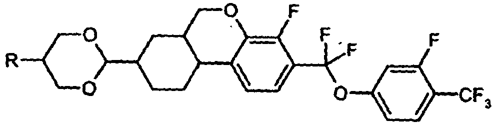

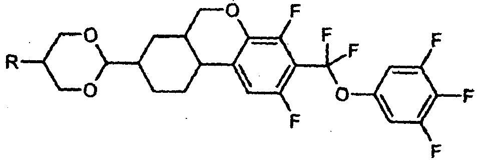

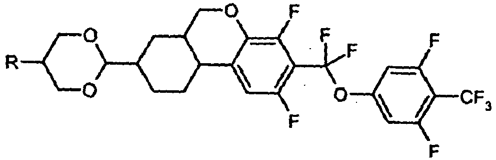

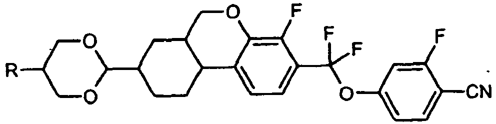

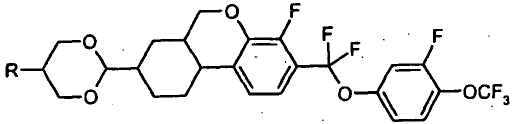

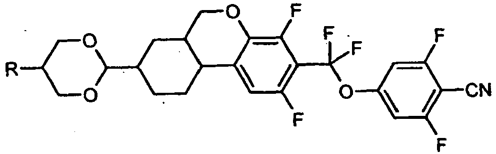

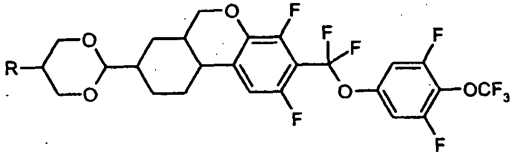

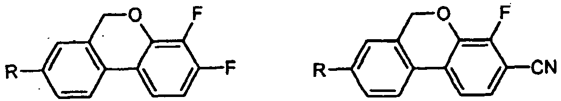

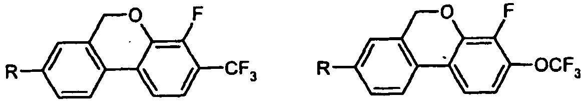

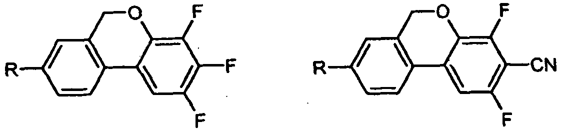

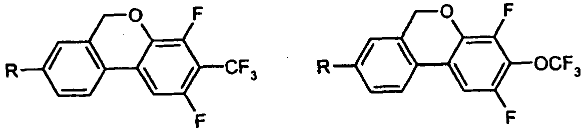

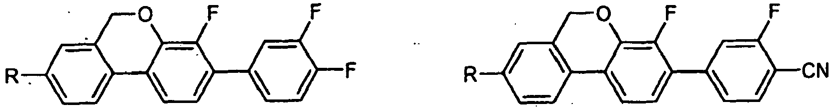

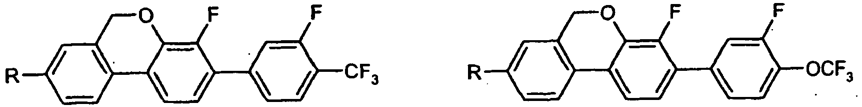

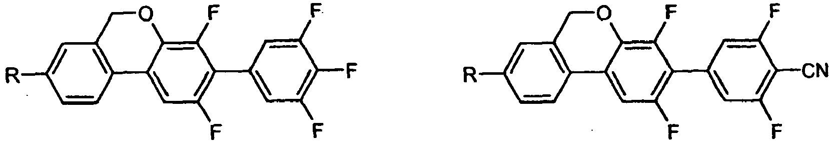

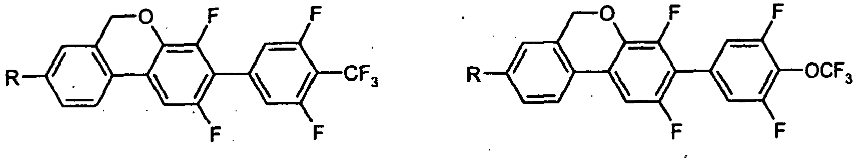

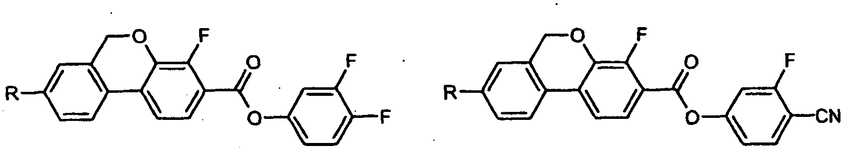

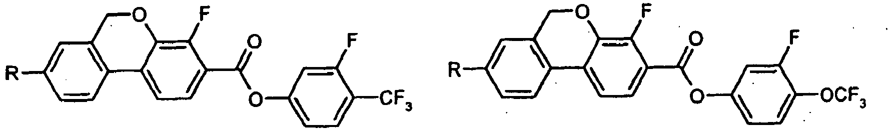

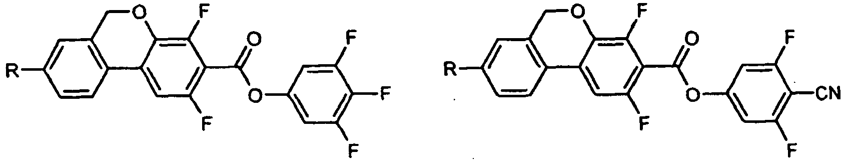

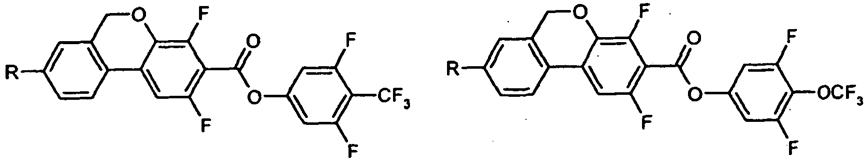

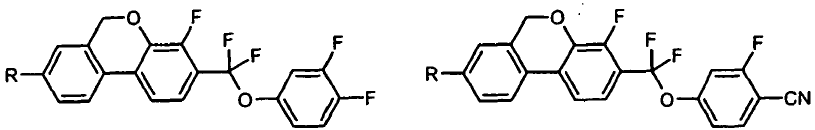

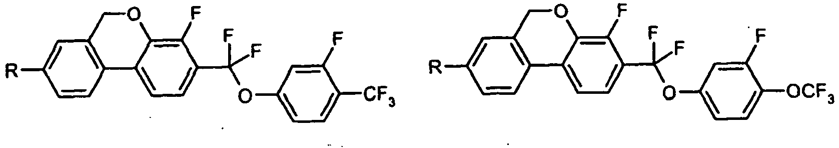

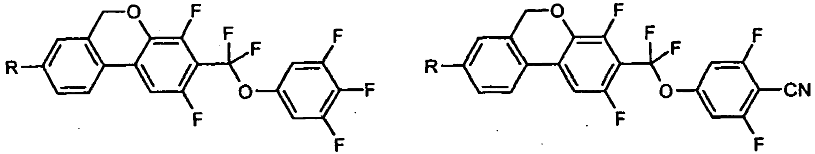

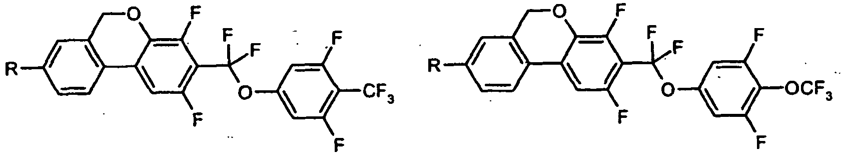

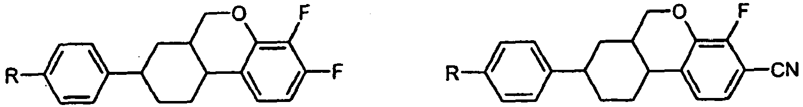

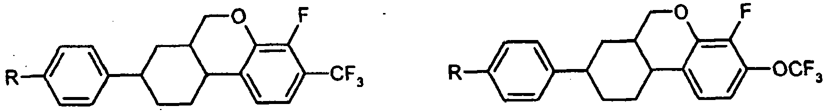

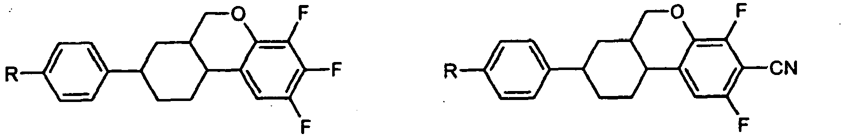

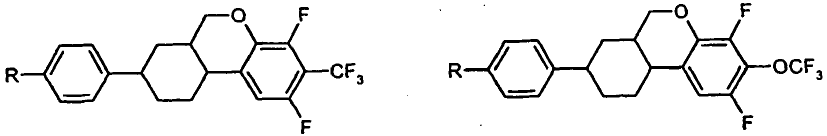

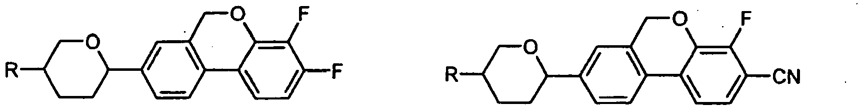

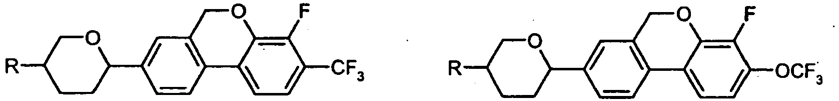

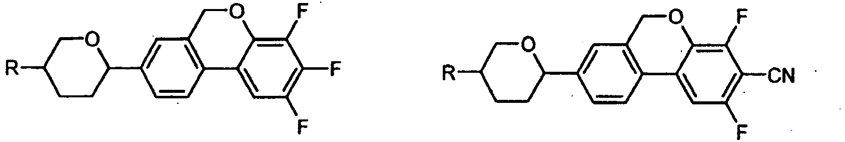

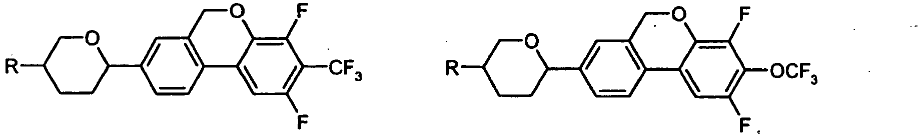

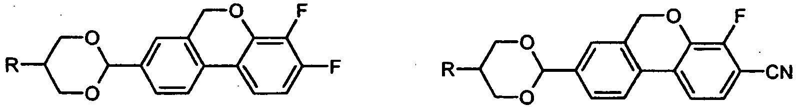

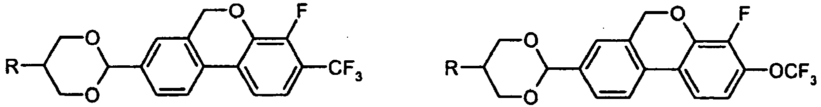

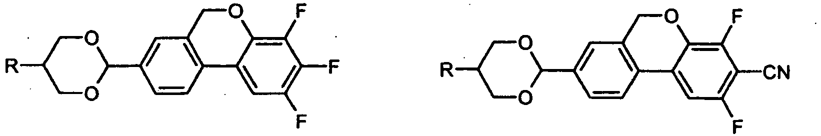

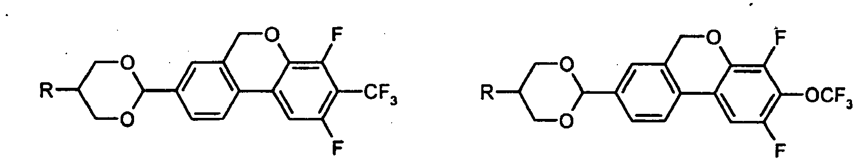

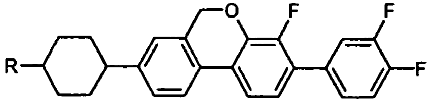

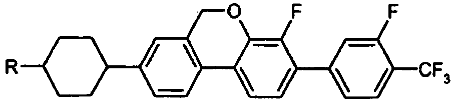

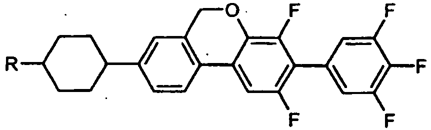

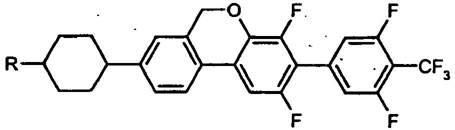

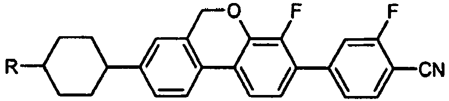

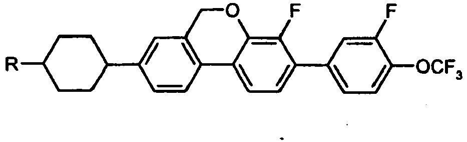

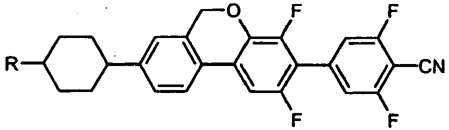

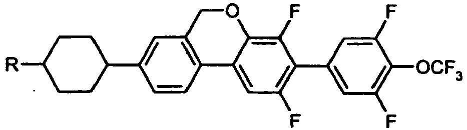

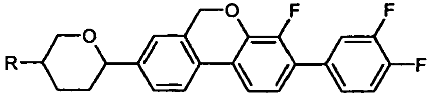

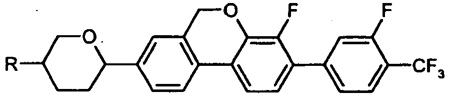

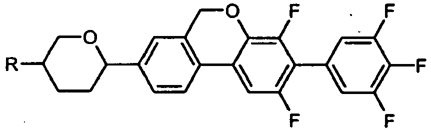

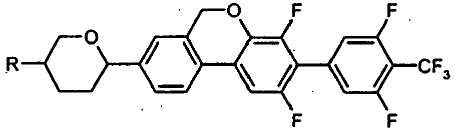

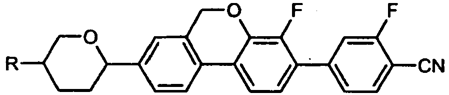

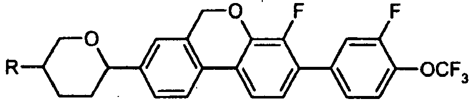

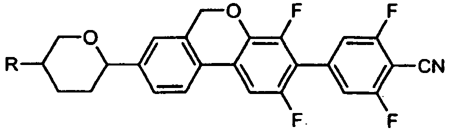

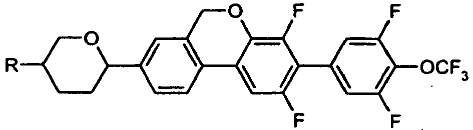

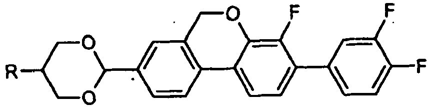

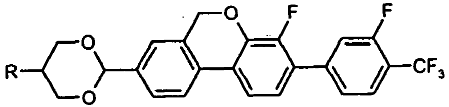

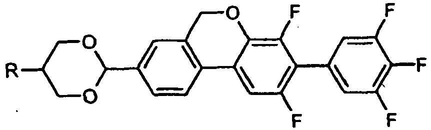

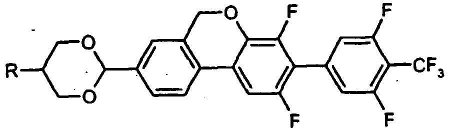

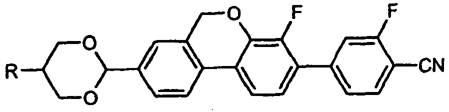

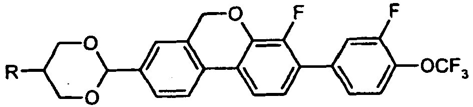

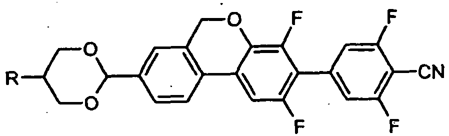

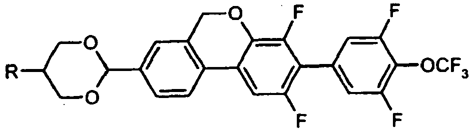

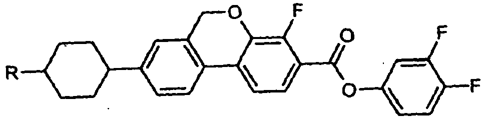

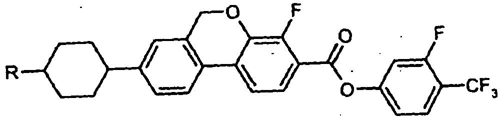

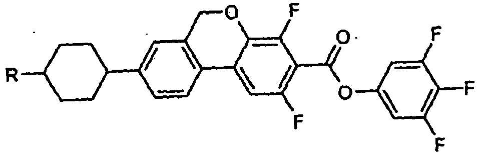

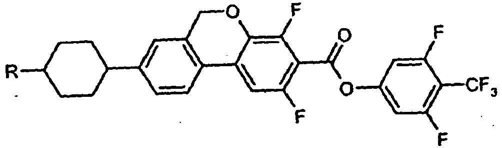

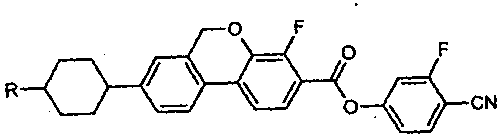

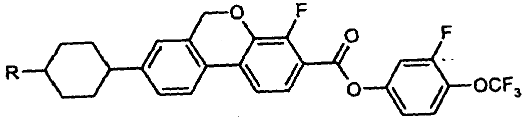

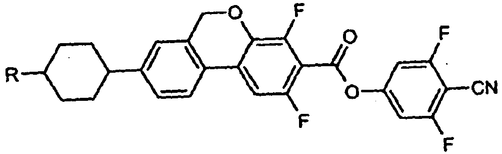

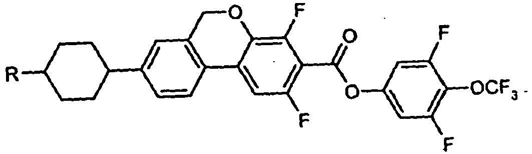

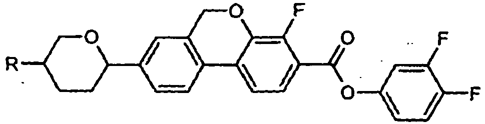

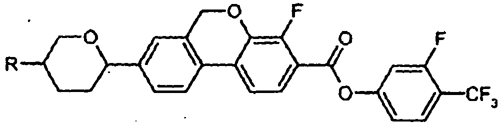

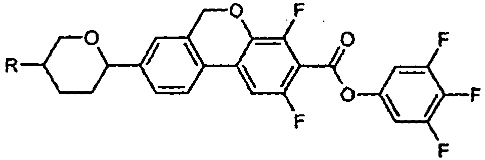

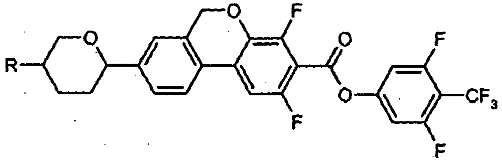

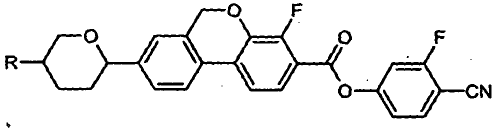

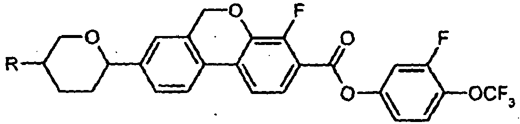

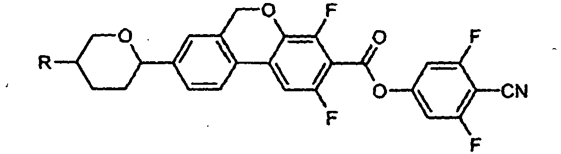

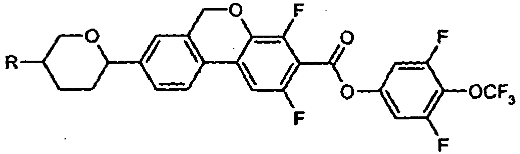

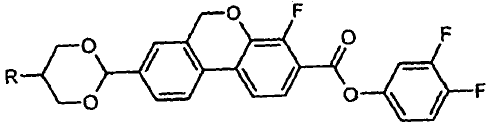

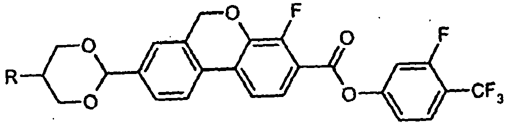

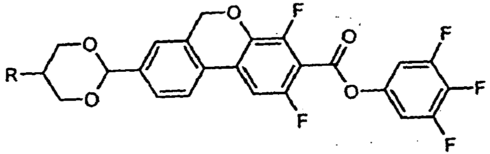

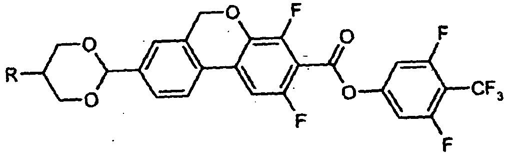

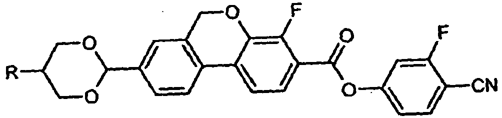

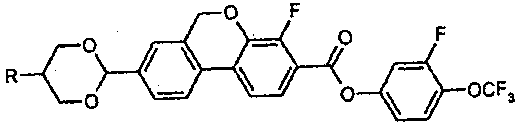

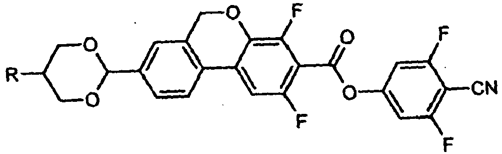

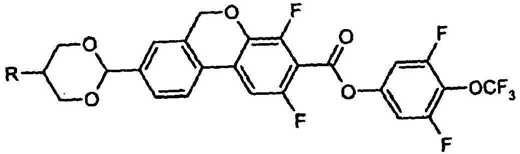

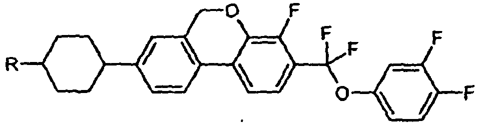

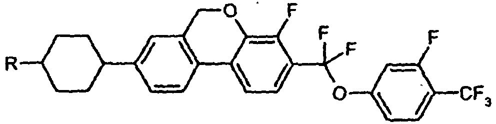

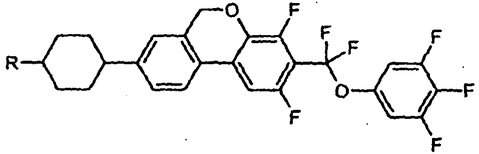

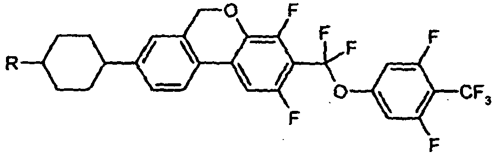

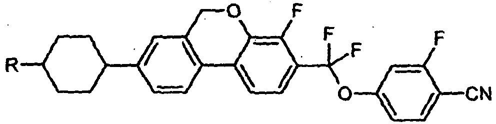

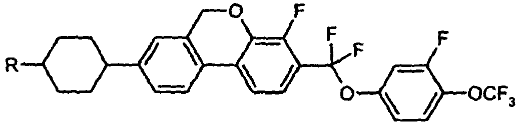

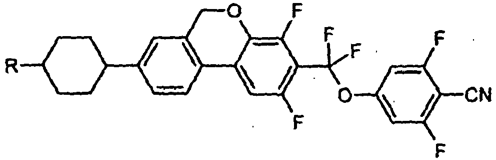

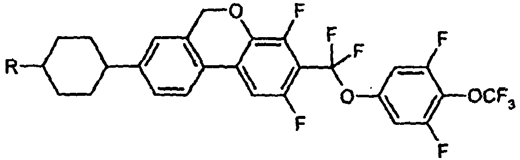

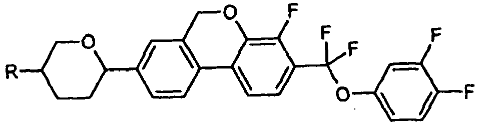

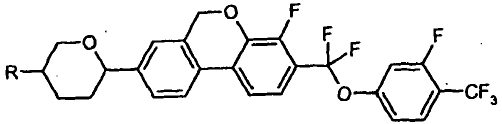

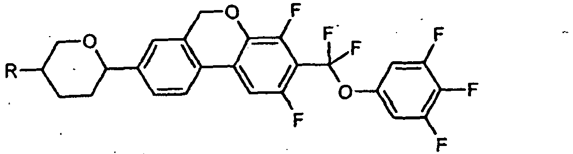

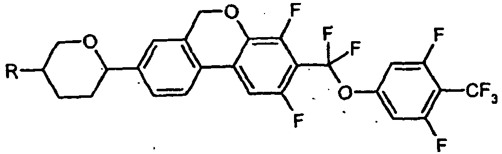

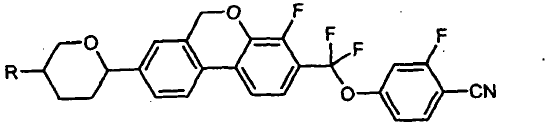

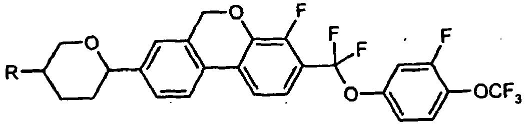

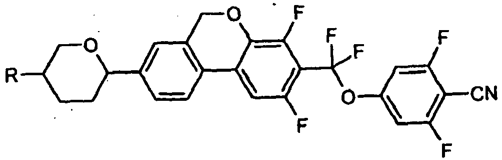

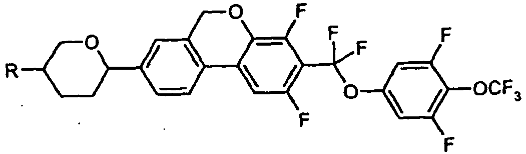

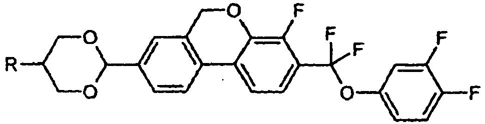

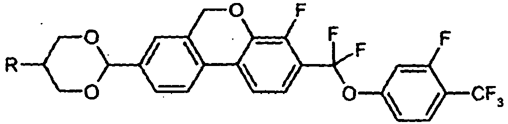

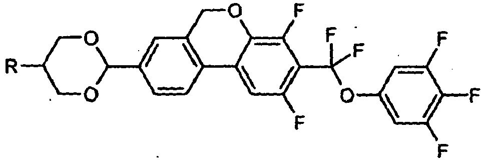

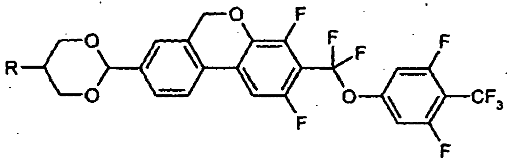

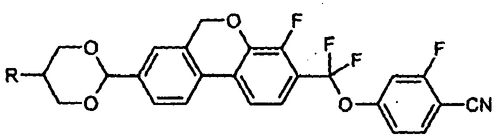

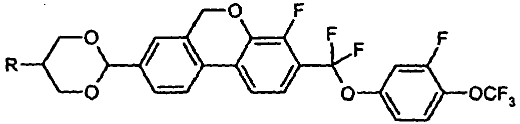

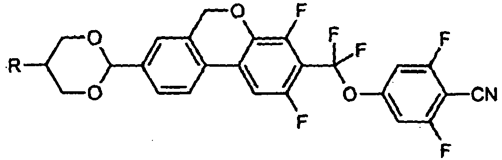

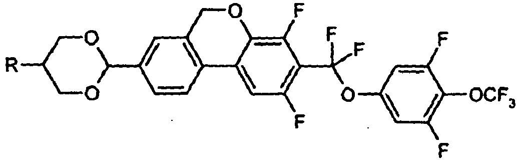

Im Folgenden werden weitere Beispiele für Strukturen bevorzugter Verbindungen der Formel I gegebene, worin R die unter Formel I für R¹ gegebene Bedeutung hat und bevorzugt Alkyl mit 1 bis 12 C-Atomen, besonders bevorzugt mit 1 bis 7 C-Atomen oder Alkenyl mit 2 bis 7 C-Atomen und ganz besonders bevorzugt n-Alkyl, inklusive Methyl, oder 1-E-Alkenyl, inklusive Vinyl, bedeutet.

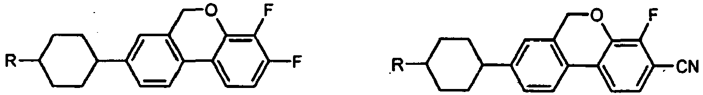

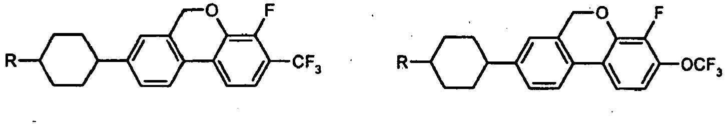

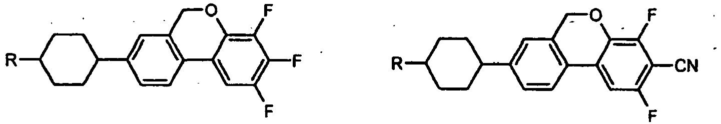

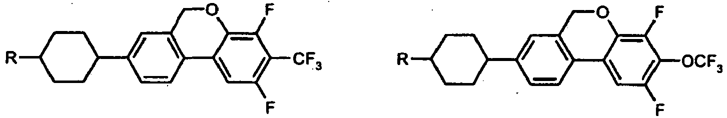

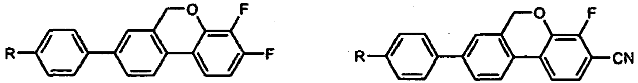

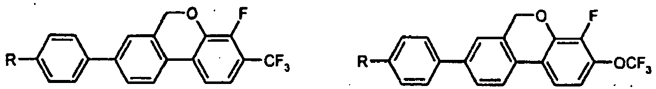

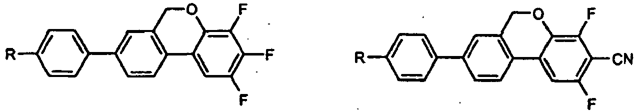

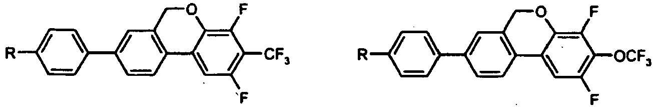

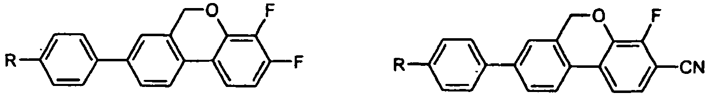

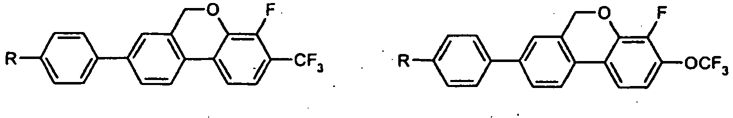

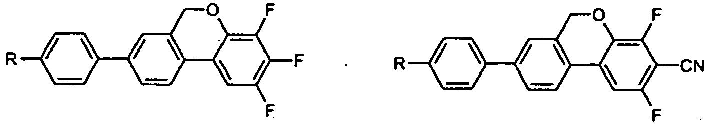

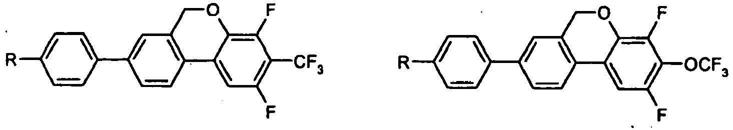

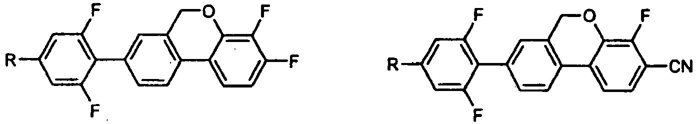

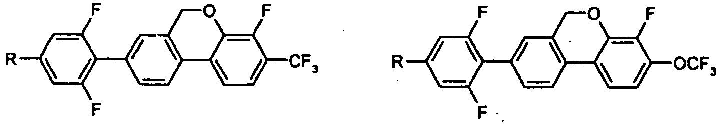

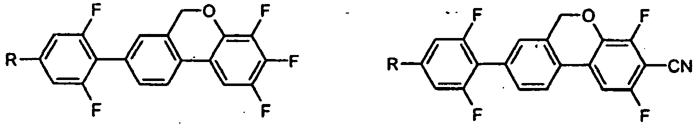

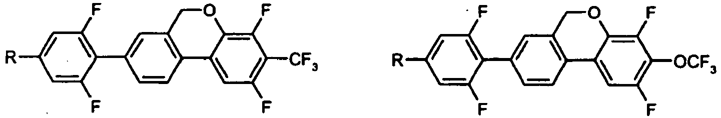

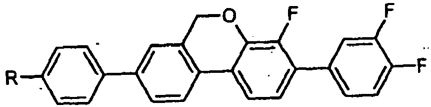

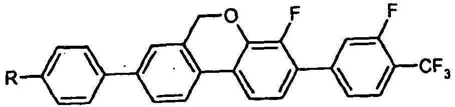

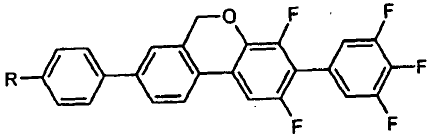

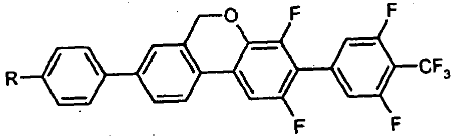

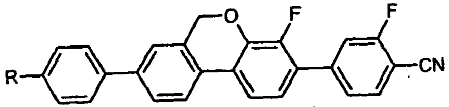

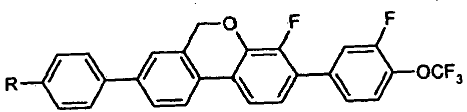

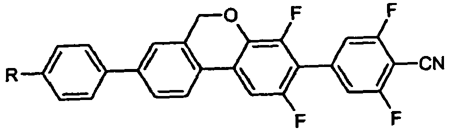

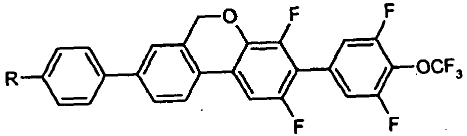

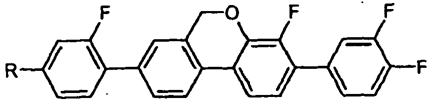

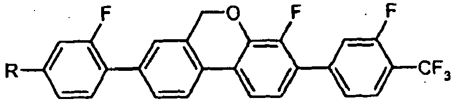

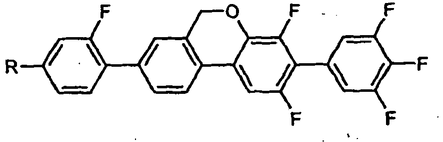

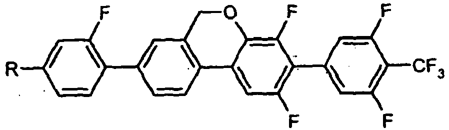

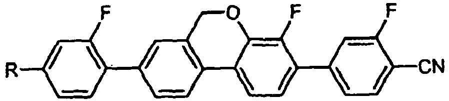

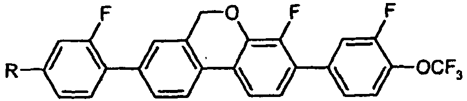

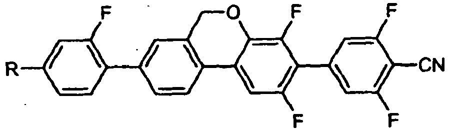

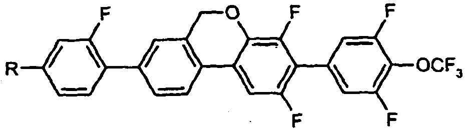

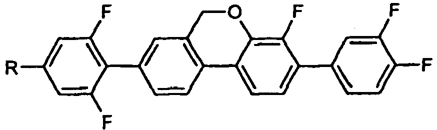

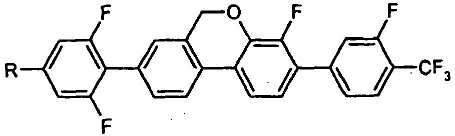

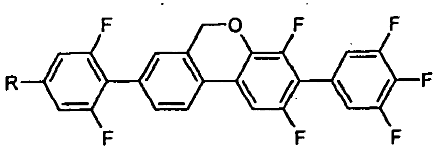

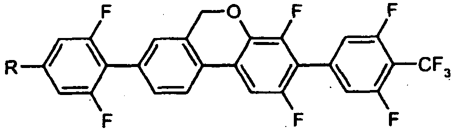

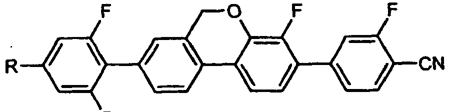

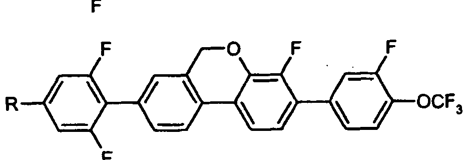

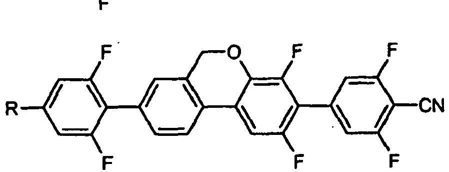

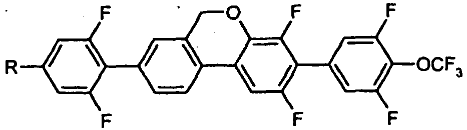

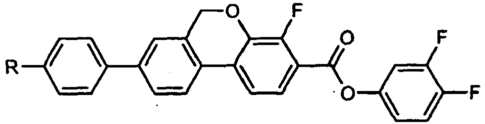

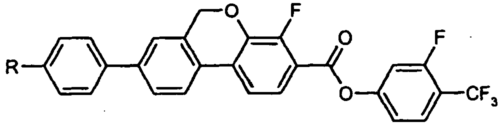

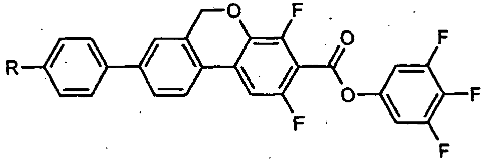

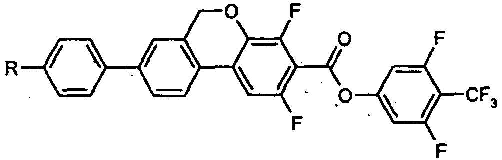

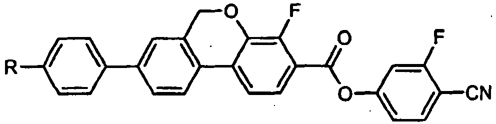

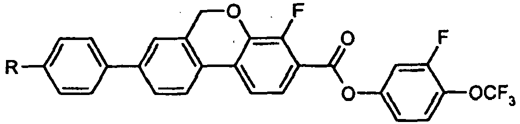

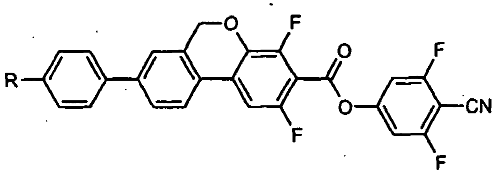

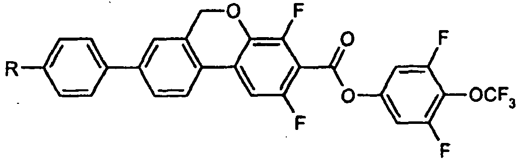

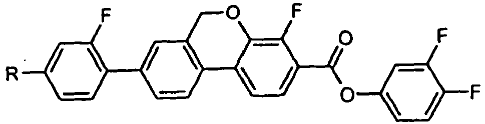

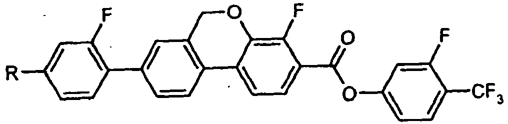

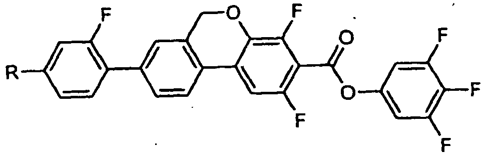

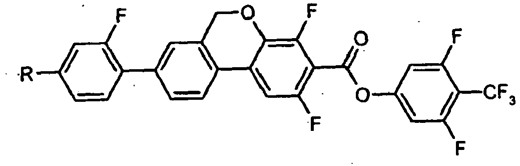

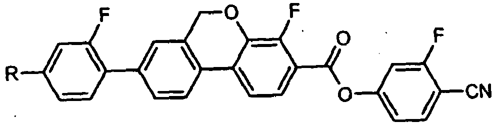

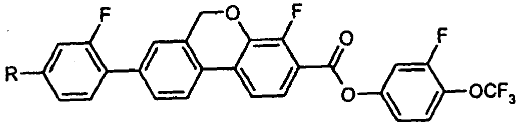

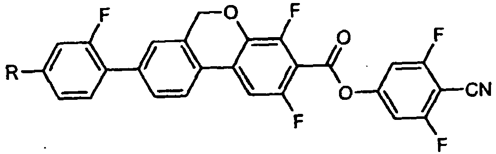

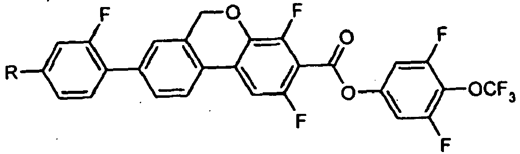

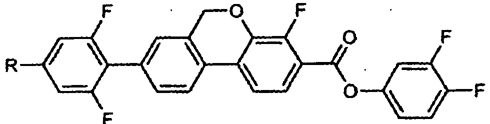

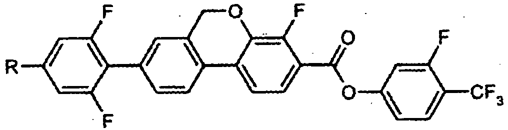

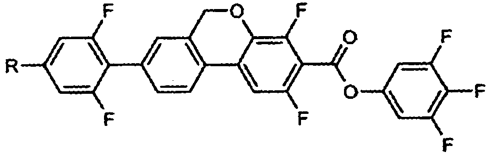

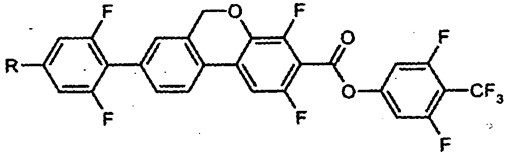

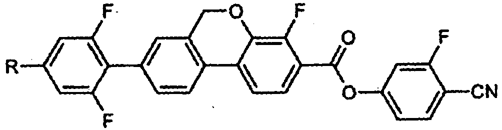

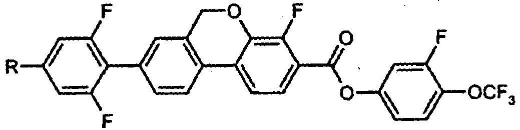

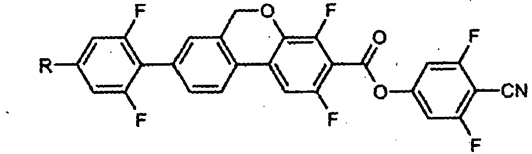

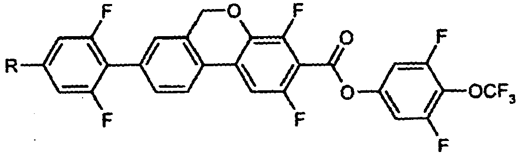

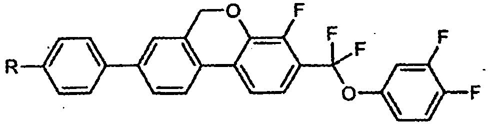

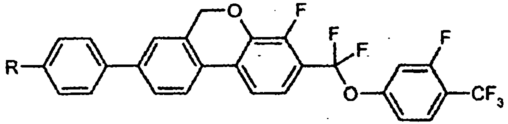

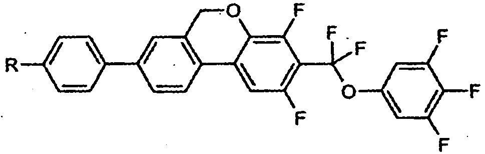

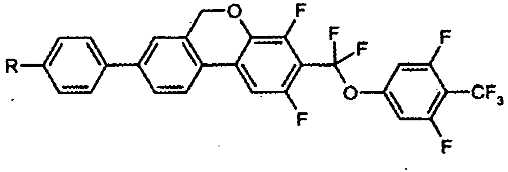

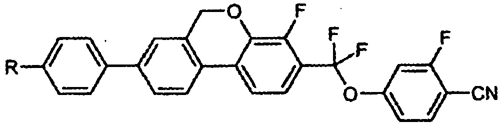

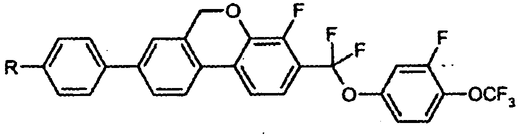

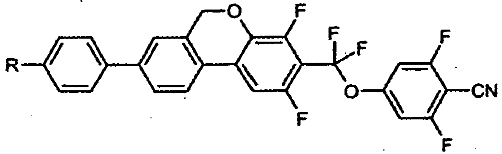

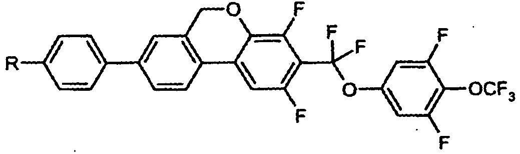

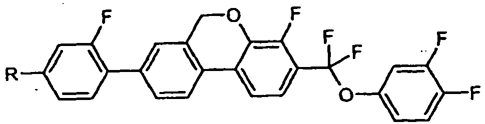

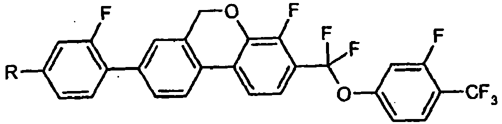

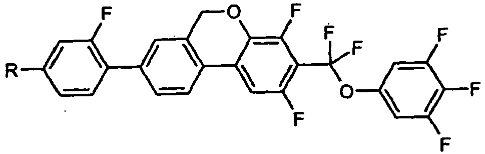

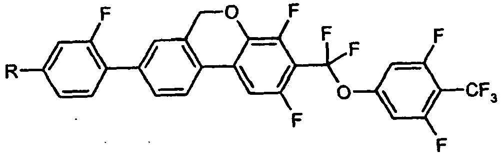

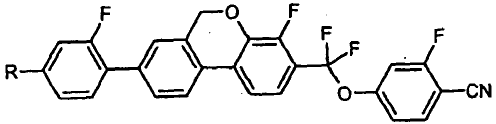

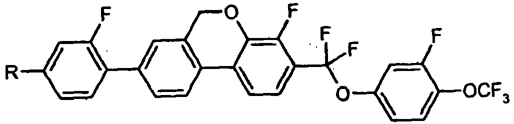

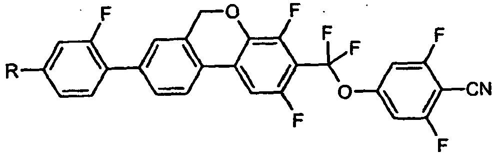

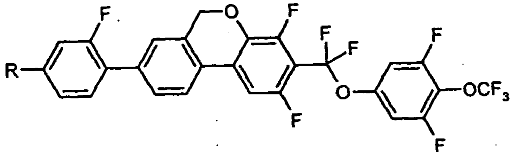

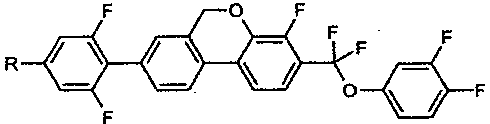

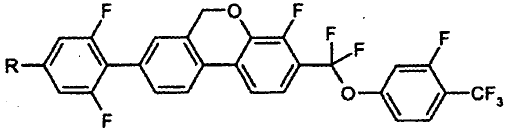

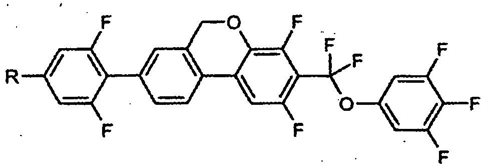

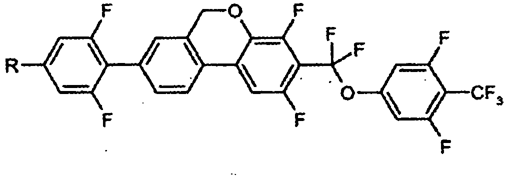

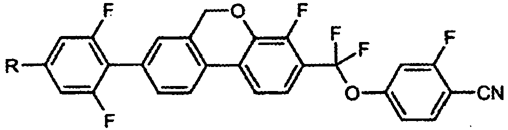

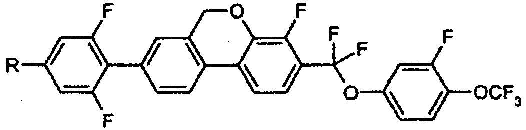

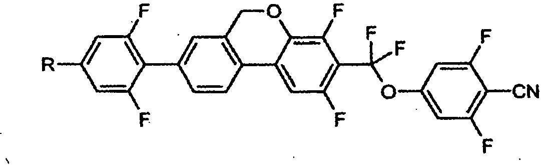

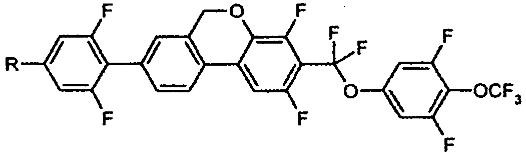

Erfindungsgemäße Verbindungen der Formel I können aufgrund ihrer Molekülstruktur chiral sein und können dementsprechend in verschiedenen enantiomeren Formen auftreten. Sie können daher in racemischer oder in optisch aktiver Form vorliegen.

Da sich die pharmazeutische Wirksamkeit der Racemate bzw. der Stereoisomeren der erfindungsgemäßen Verbindungen unterscheiden kann, kann es wünschenswert sein, die Enantiomere zu verwenden. In diesen Fällen kann das Endprodukt oder aber bereits die Zwischenprodukte in enantiomere Verbindungen, durch dem Fachmann bekannte chemische oder physikalische Maßnahmen, aufgetrennt oder bereits als solche bei der Synthese eingesetzt werden.

Im Falle racemischer Amine werden aus dem Gemisch durch Umsetzung mit einem optisch aktiven Trennmittel Diastereomere gebildet. Als Trennmittel eignen sich z.B. optisch aktiven Säuren, wie die R- und S-Formen von Weinsäure, Diacetylweinsäure, Dibenzoylweinsäure, Mandelsäure, Äpfelsäure, Milchsäure, geeignet N-geschützte Aminosäuren (z.B. N-Benzoylprolin oder N-Benzolsulfonylprolin) oder die verschiedenen optisch aktiven Camphersulfonsäuren. Vorteilhaft ist auch eine chromatographische Enantiomerentrennung mit Hilfe eines optisch aktiven Trennmittels (z.B. Dinitrobenzoylphenylglycin, Cellulosetriacetat oder andere Derivate von Kohlenhydraten oder auf Kieselgel fixierte chiral derivatisierte Methacrylatpolymere). Als Laufmittel eignen sich hierfür wäßrige oder alkoholische Lösungsmittelgemische wie z.B. Hexan/Isopropanol/ Acetonitril z.B. im Verhältnis 82:15:3.

Erfindung umfaßt nicht nur die genannten Verbindungen, sondern auch Mischungen und Zubereitungen, welche neben diesen erfindungsgemäßen Verbindungen auch andere pharmakologische Wirkstoffe oder Adjuvantien enthalten, die die primäre pharmakologische Wirkung der erfindungsgemäßen Verbindungen in gewünschter Weise beeinflussen können.

Die erfindungsgemäßen Verbindungen können als Arzneimittelwirkstoffe in der Human- oder Veterinärmedizin eingesetzt werden, insbesondere zur Prophylaxe oder Therapie von Krankheiten, die durch die zentralnervöse Wirkung der Verbindungen beeinflusst werden können.

Besonders bevorzugt können die erfindungsgemäßen Verbindungen zur Behandlung sexueller Störungen bzw. Steigerung der sexuellen Leistungsfähigkeit, von Durchfallerkrankungen, Nikotinabhängigkeit, entzündlicher ZNS-Erkrankungen (Demyelinisierung, virale Gehirnhautentzündung, Multiple Sklerose, Guillain-Barré-Syndrom) und unfallbedingter Gehirnverletzungen oder Schädeltraumata, Appetenzstörungen, d. h. Abhängigkeiten verschiedener Art (Drogen, Alkohol, Zucker), Bulimie und ihre eventuellen Folgen (Fettleibigkeit, Diabetes) eingesetzt werden.

Sie sind weiterhin wirksam gegen Bluthochdruck oder wirken gegen Angstzustände und/oder Depressionen, als Sedativum, Tranquilizer, Analgeticum, Antiemeticum oder sie wirken entzündungshemmend.

Die zentralnervöse Wirkung kann durch die Verabreichung an Ratten in Dosierungen von 0.1-1000 mg/kg, bevorzugt von 1-100 mg/kg gezeigt werden. Es werden Effekte wie reduzierte spontane motorische Aktivität beobachtet, wobei die erforderliche Dosis sowohl von der Wirksamkeit der Verbindung als auch von Körpergewicht des Versuchtieres abhängt.

Gegenstand der Erfindung ist dementsprechend Verbindungen der oben und unten sowie in den Ansprüchen definierten Formeln einschließlich ihrer physiologisch unbedenklichen Salze als Arzneimittel, Diagnostika oder Reagenzien.

Gegenstand sind auch entsprechende pharmazeutische Zubereitungen, welche mindestens ein Arzneimittel der Formel I sowie gegebenenfalls Träger- und/oder Hilfsstoffe enthalten. Als Trägerstoffe kommen organische oder anorganische Substanzen in Frage, die sich für die enterale (z. B. orale), parenterale, topische Applikation oder für eine Applikation in Form eines Inhalations-Sprays eignen und mit den neuen Verbindungen nicht reagieren, beispielsweise Wasser, pflanzliche Öle, Benzylalkohole, Alkylenglykole, Polyethylenglykole, Glyzerintriacetat, Gelatine, Kohlenhydrate wie Lactose oder Stärke, Magnesiumstearat, Talk, Vaseline. Zur oralen Anwendung dienen insbesondere Tabletten, Pillen, Dragees, Kapseln, Pulver, Granulate, Sirupe, Säfte oder Tropfen, zur rektalen Anwendung Suppositorien, zur parenteralen Anwendung Lösungen, vorzugsweise ölige oder wässrige Lösungen, ferner Suspensionen, Emulsionen oder Implantate, für die topische Anwendung Salben, Cremes oder Puder. Die neuen Verbindungen können auch lyophilisiert und die erhaltenen Lyophilisate z. B. zur Herstellung von Injektionspräparaten verwendet werden. Die angegebenen Zubereitungen können sterilisiert sein und/oder Hilfsstoffe wie Gleit-, Konservierungs-, Stabilisierungs- und/oder Netzmittel, Emulgatoren, Salze zur Beeinflussung des osmotischen Druckes, Puffersubstanzen, Farb-, Geschmacks- und/oder mehrere weitere Wirkstoffe enthalten, z. B. ein oder mehrere Vitamine.

Für die Applikation als Inhalations-Spray können Sprays verwendet werden, die den Wirkstoff entweder gelöst oder suspendiert in einem Treibgas oder Treibgasgemisch (z. B. CO₂) enthalten. Zweckmäßig verwendet man den Wirkstoff dabei in mikronisierter Form, wobei ein der mehrere zusätzliche physiologisch verträgliche Lösungsmittel zugegen sein können, z. B. Ethanol. Inhalationslösungen können mit Hilfe üblicher Inhalatoren verabreicht werden.

Die erfindungsgemäßen Substanzen können in der Regel in Analogie zu anderen, im Handel befindlichen THC-Analoga verabreicht werden, vorzugsweise in Dosierungen zwischen etwa 0,05 und 500 mg, insbesondere zwischen 0,5 und 100 mg pro Dosierungseinheit. Die tägliche Dosierung liegt vorzugsweise zwischen etwa 0,01 und 20 mg/kg Körpergewicht. Die spezielle Dosis für jeden Patienten hängt jedoch von den verschiedensten Faktoren ab, beispielsweise von der Wirksamkeit der eingesetzten speziellen Verbindung, vom Alter, Körpergewicht, allgemeinen Gesundheitszustand, Geschlecht, von der Kost, vom Verabreichungszeitpunkt und -weg, von der Ausscheidungsgeschwindigkeit, Arzneistoffkombination und Schwere der jeweiligen Erkrankung, welcher die Therapie gilt.

Ferner können die neuen Verbindungen der Formel I in der analytischen Biologie und Molekularbiologie verwendet werden.

Die spezifische Ligandenbindung an die Rezeptoren wird definiert als die Differenz zwischen vollständier Bindung und nicht spezifischer Bindung, die in Gegenwart von einem Überschuss ungelabellten Liganden ermittelt wird (siehe z. B. MUNRO, S., THOMAS, K.L. and ABU-SHAAR, M. (1993), Molecular characterization of a peripheral receptor for cannabinoids. Nature, 365 : 61-65. RINALDI-CARMONA, M., CALANDRA, B., SHIRE, D., BOUABOULA, M., OUSTRIC, D., BARTH, F., CASELLAS, P., FERRARA, P. and LE FUR, G. (1996), Characterization of two cloned human CB1 cannabinoid receptors isoform; J. Pharmacol. Exp. Ther., 278: 871-878.

Gegenstand der vorliegenden Erfindung sind auch Flüssigkristallmedien, die eine oder mehrere Verbindung(en) der Formel I enthalten.

In einer bevorzugten Ausführungsform enthalten die Flüssigkristallmedien gemäß der vorliegenden Erfindung
a) eine oder mehrere dielektrisch negative Verbindung(en) der Formel I 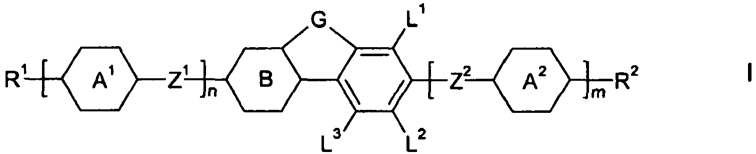 worin die Parameter die oben angegebenen Bedeutungen besitzen,
b) eine oder mehrere dielektrisch positive Verbindung(en) der Formel II 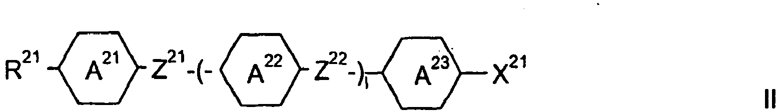 worin
   - R²¹: die oben bei Formel I für R¹ gegebene Bedeutung hat,
   - X²¹: Halogen, -CN, -SCN, -NCS, -OCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 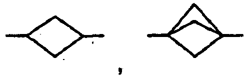 -CO-, -CO-O-, -O-CO- oder -O-CO-O- so ersetzt sein können, dass weder O-noch S-Atome direkt miteinander verknüpft sind, bevorzugt F, Cl, -OCF₃, -OCF₂, oder -CF₃
   - Z²¹ und Z²²: , jeweils unabhängig voneinander, die oben bei Formel I für Z¹ gegebene Bedeutung haben,
   mindestens einer der vorhandenen Ringe 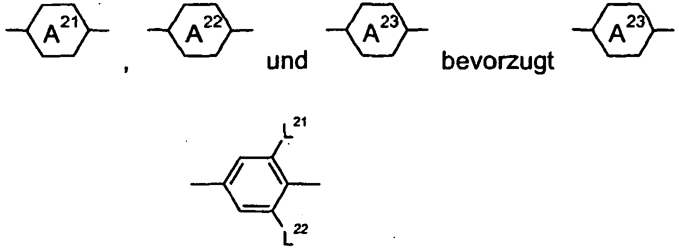 und die anderen, jeweils unabhängig voneinander, 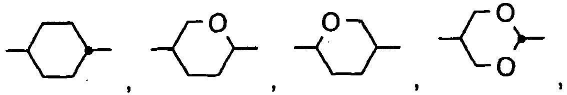 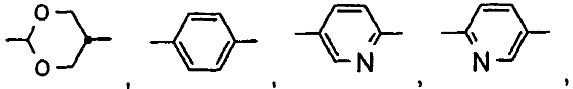 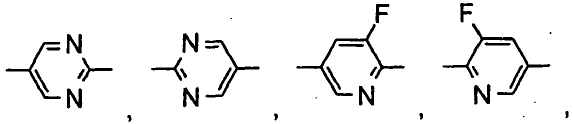 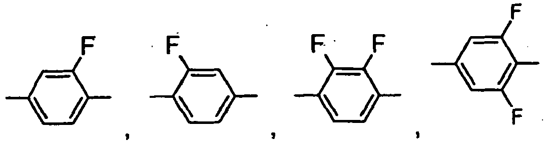 oder 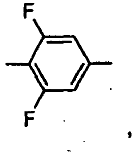 bevorzugt 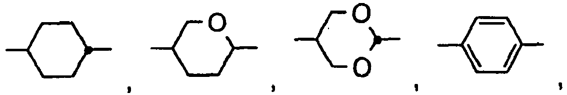 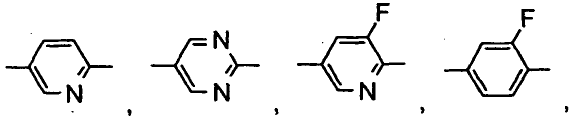 oder 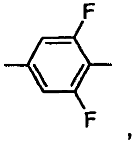 besonders bevorzugt 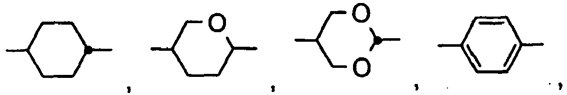 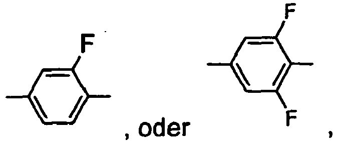 besonders bevorzugt 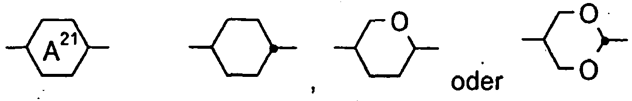 und 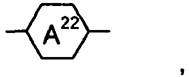 wenn vorhanden, 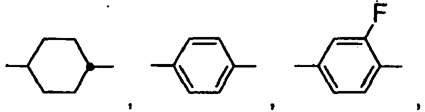 oder 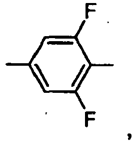
   - L²¹ und L²²: , unabhängig voneinander, H oder F,
   - l: , 1 oder 2, bevorzugt 0 oder 1,
   bedeuten;
   und optional
c) eine oder mehrere dielektrisch neutrale Verbindung der Formel III 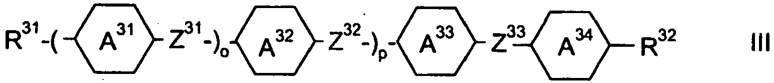 worin
   - R³¹ und R³²: jeweils unabhängig voneinander die oben bei Formel I für R¹ gegebene Bedeutung besitzen und
   - Z³¹, Z³² und Z³³: jeweils unabhängig voneinander -CH₂CH₂-, -CH=CH-, -COO- oder eine Einfachbindung 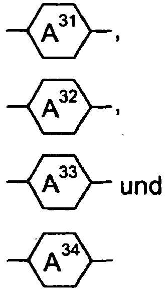
   jeweils unabhängig voneinander 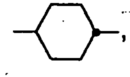 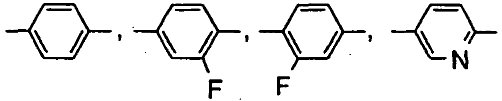 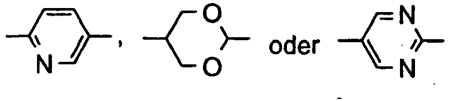 und
   - o und p: unabhängig voneinander 0 oder 1
   bevorzugt jedoch
   - R³¹ und R³²: jeweils unabhängig voneinander Alkyl oder Alkoxy mit 1-5 C-Atomen oder Alkenyl mit 2-5 C-Atomen, 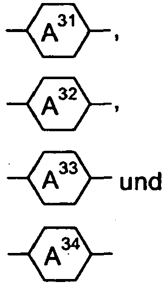 jeweils unabhängig voneinander 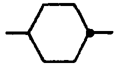 oder 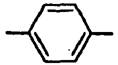
   und ganz besonders bevorzugt mindestens zwei dieser Ringe 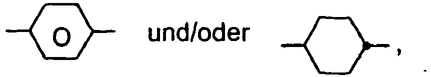 wobei ganz besonders bevorzugt zwei benachbarte Ringe direkt verknüpft sind und zwar bevorzugt 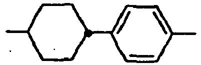 oder 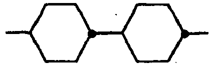
bedeuten, wobei bei dem Phenylenring ein oder mehrere H-Atome, unabhängig voneinander durch F oder CN, bevorzugt durch F und eine oder zwei nicht benachbarte CH₂-Gruppen des Cyclohexylenrings bzw. eines der Cyclohexylenringe durch O-Atome ersetzt sein können.

Bevorzugt enthalten die Flüssigkristallmedien eine oder mehrere Verbindungen der Formel I die keine Biphenyleinheit enthalten.

Besonders bevorzugt enthalten die Flüssigkristallmedien eine oder mehrere Verbindungen der Formel I
worin zwei benachbarte Ringe direkt
verknüpft sind und zwar bevorzugt 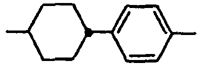
oder 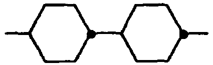
bedeuten, wobei bei dem Phenylenring ein oder mehrere H-Atome, unabhängig voneinander durch F oder CN, bevorzugt durch F und eine oder zwei nicht benachbarte CH₂-Gruppen des Cyclohexylenrings bzw. eines der Cyclohexylenringe durch O-Atome ersetzt sein können.

In einer bevorzugten Ausführungsform, die mit den gerade beschriebenen Ausführungsformen identisch sein kann, enthalten die Flüssigkristallmedien eine oder mehrere Verbindungen ausgewählt aus der Gruppe der Verbindungen ausgewählt aus der Gruppe der Verbindungen der Formel I-3.

Bevorzugt enthält das Flüssigkristallmedium eine oder mehrere Verbindungen ausgewählt aus der Gruppe der Verbindungen der Formeln II-1 bis II-4 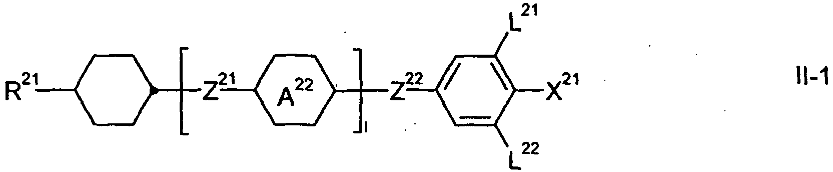 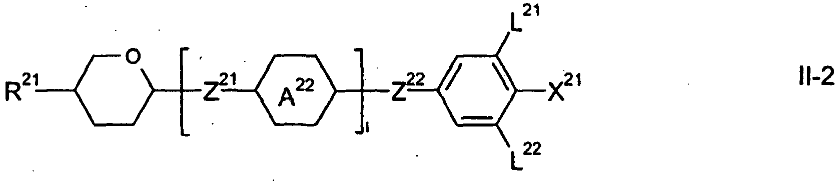 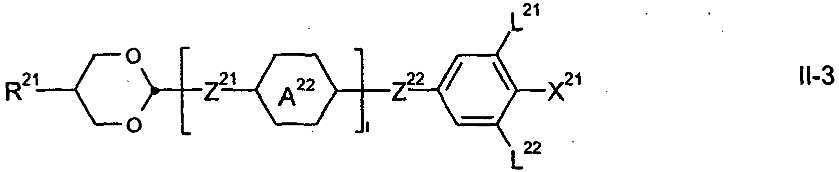 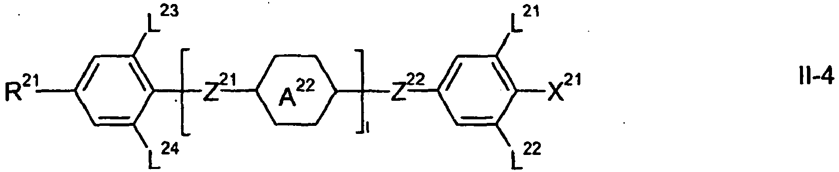 worin
R²¹, X²¹, Z¹², Z²², L²¹, L²², 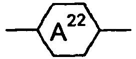 und I jeweils die oben bei Formel II gegebene Bedeutung besitzen und
- L²³ und L²⁴,: unabhängig voneinander, H oder F bedeuten und bei Formel II-4 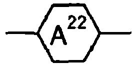 bevorzugt einen aromatischen Ring bedeutet.

Besonders bevorzugt ist
- R²¹: Alkyl oder Alkoxy, bevorzugt mit 1-5 C-Atomen, bevorzugt Akyl, und
im Fall I = 0
- Z²²: -CF₂O-, -CO-O- oder eine Einfachbindung besonders bevorzugt eine Einfachbindung,
im Fall I = 1 oder 2
- Z²¹ und Z²²: beide eine Einfachbindung oder Z²² oder einer der vorhandenen Z²¹ -CO-O-, -CF₂O- oder -CH=CH-, bevorzugt -CO-O- oder -CF₂O-, besonders bevorzugt -CF₂O-, und die anderen eine Einfachbindung.

Insbesondere bevorzugt enthält das Flüssigkristallmedium eine oder mehrere Verbindungen ausgewählt aus der Gruppe der Verbindungen der Formeln II-1a bis II-1h, II-2a bis II-2d, II-3a und III-3b und II-4a bis II-4c 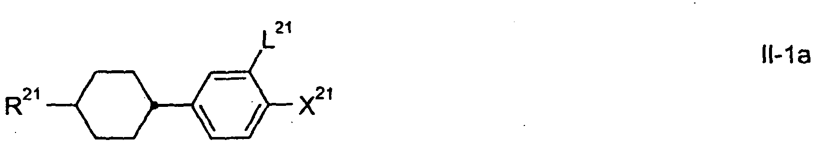 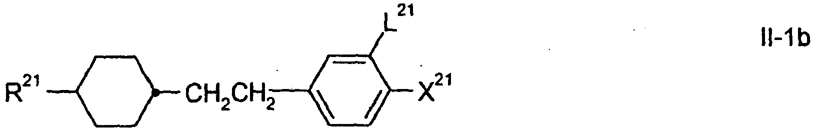 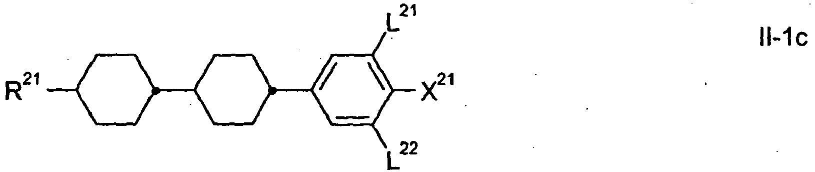 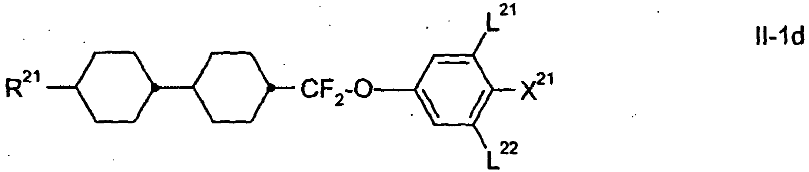 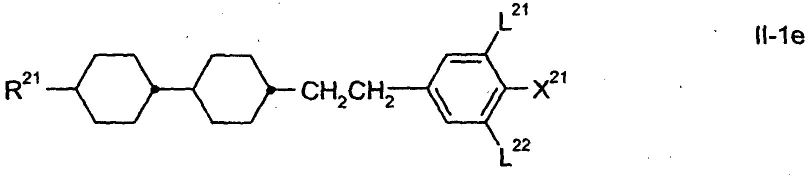 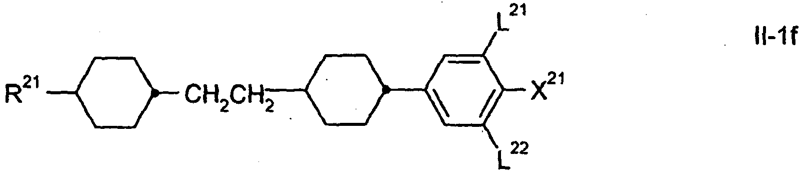 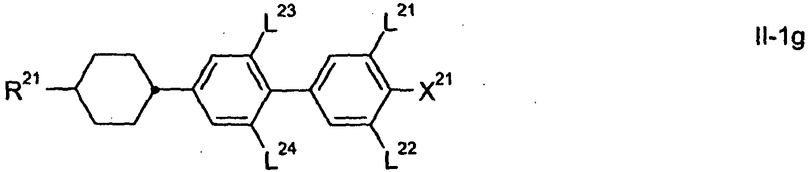 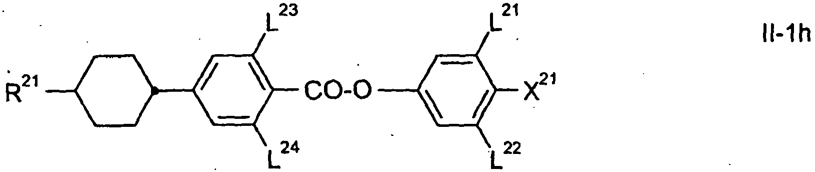 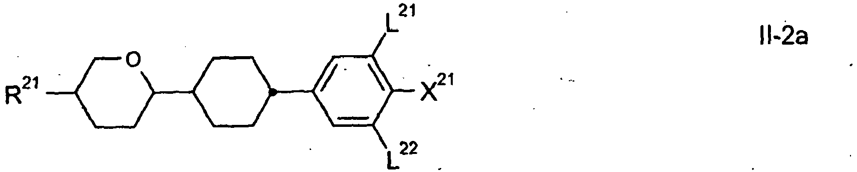 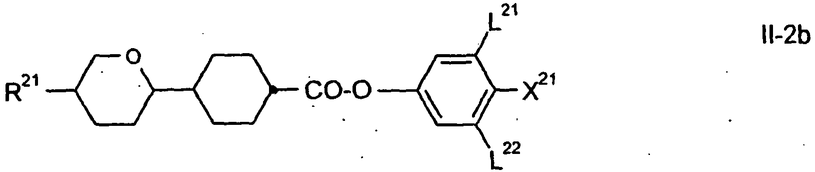 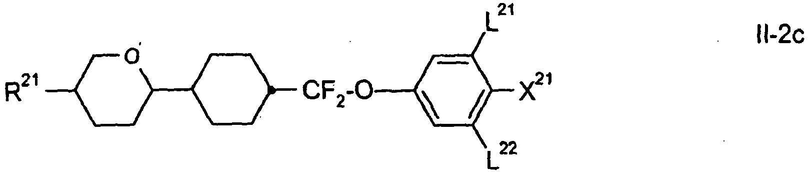 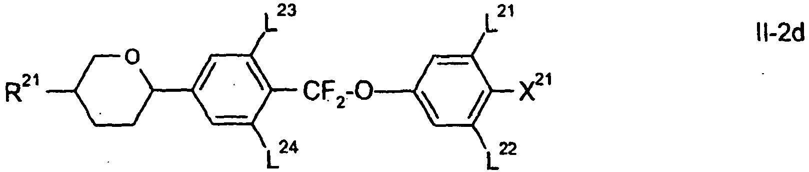 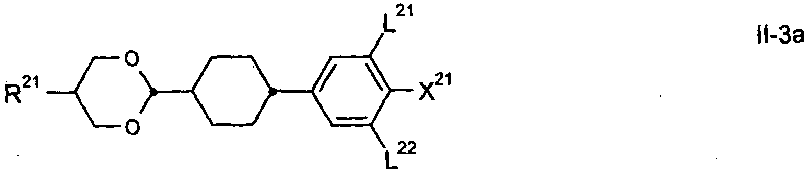 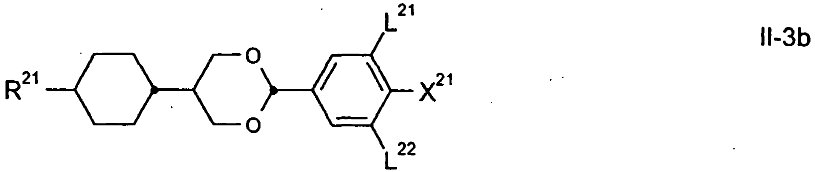 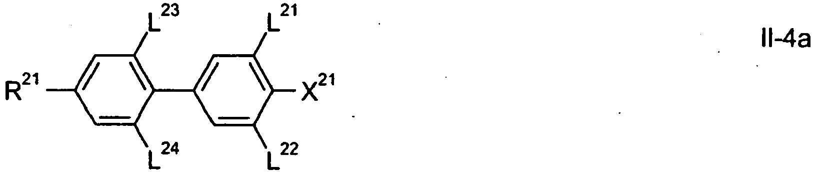 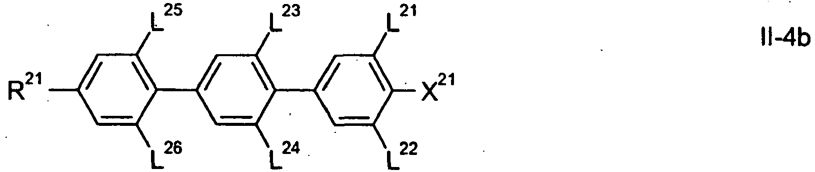 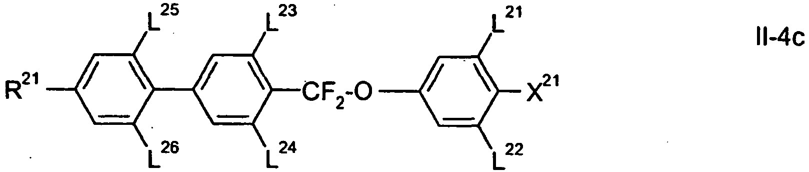 worin
R²¹ und X²¹ jeweils die oben bei Formel II gegebene Bedeutung besitzen und
- L²³ bis L²⁶: , unabhängig voneinander, H oder F bedeuten.

Besonders bevorzugt enthält das Flüssigkristallmedium eine oder mehrere Verbindungen ausgewählt aus der Gruppe der Verbindungen der Formeln III-1 bis III-3: 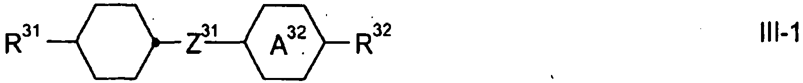 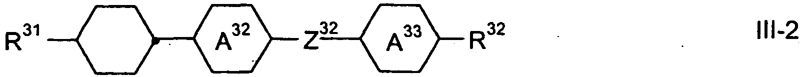 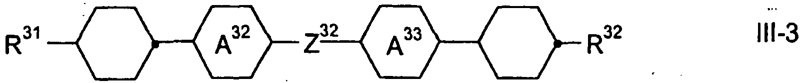 worin R³¹, R³², Z³¹, Z³², 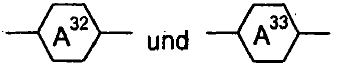 jeweils die oben bei Formel III angegebene Bedeutung haben.

Insbesondere bevorzugt enthält das Flüssigkristallmedium eine oder mehrere Verbindungen ausgewählt aus der Gruppe der Verbindungen der Formeln III-1a bis III-1d, III-1e, III-2a bis III-2g, III-3a bis III-3d und III-4a: 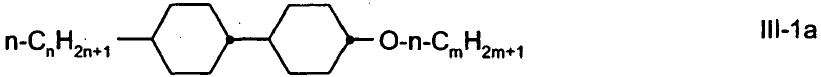 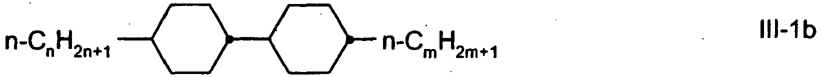 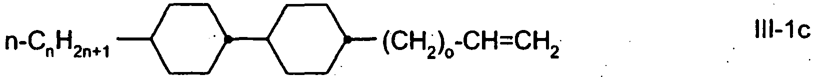 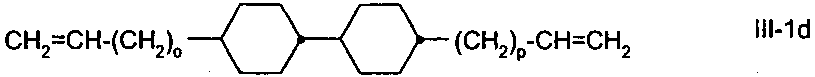 worin n und m jeweils unabhängig voneinander 1 bis 5 und o und p jeweils sowohl davon als auch voneinander unabhängig 0 bis 3 bedeuten, 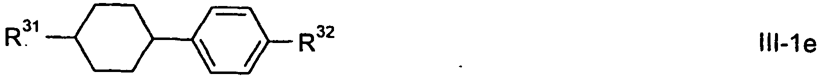 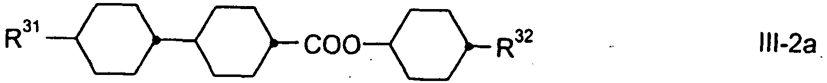 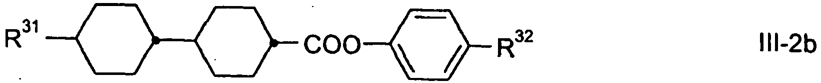 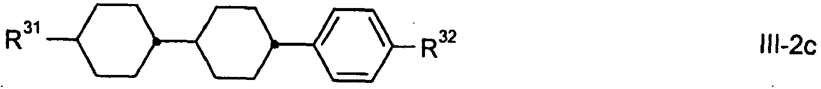 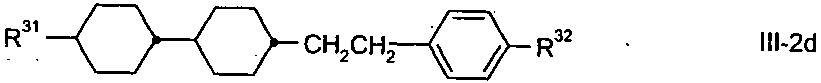 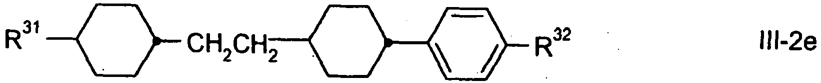 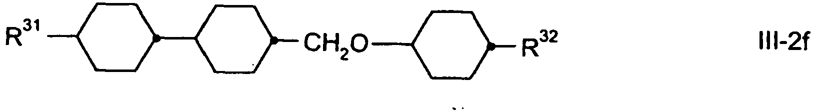 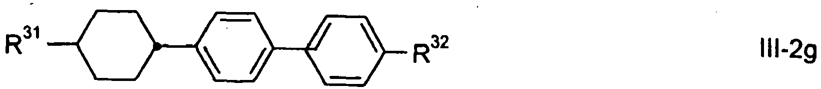 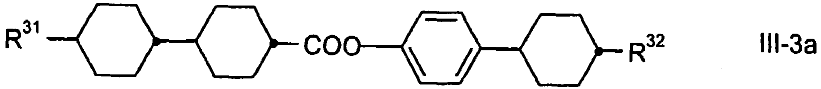 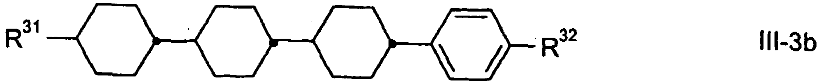 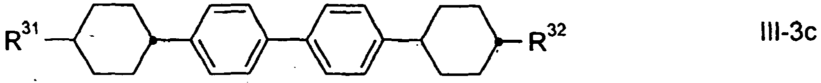 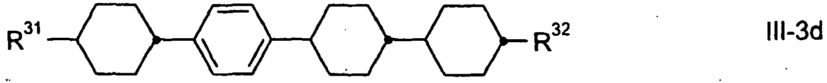 worin R³¹ und R³³ jeweils die oben unter Formel III, bevorzugt die unter Formel III-1, angegebene Bedeutung besitzen und die Phenylringe, insbesondere bei den Verbindungen III-2g und III-3c optional fluoriert sein können, jedoch nicht so, dass die Verbindungen mit denen der Formel II und ihren Unterformeln identisch sind. Bevorzugt ist R³¹ n-Alkyl mit 1 bis 5 C-Atomen, insbesondere bevorzugt mit 1 bis 3 C-Atomen und R³² n-Alkyl oder n-Alkoxy mit 1 bis 5 C-Atomen oder Alkenyl mit 2 bis 5 C-Atomen. Hiervon sind insbesondere Verbindungen der Formeln III-1a bis III-1d bevorzugt.

Bevorzugte fluorierte Verbindungen der Formeln III-2g und III-3c sind die Verbindungen der Formeln III-2g' und III-3c' 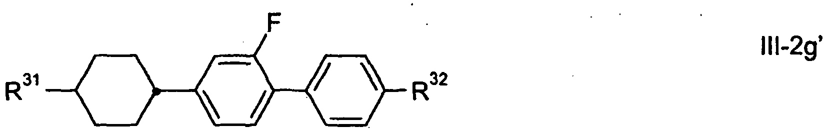 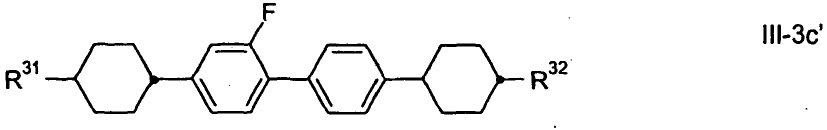 worin R³¹ und R³³ jeweils die oben unter Formel III, bevorzugt die unter Formel III-2g, bzw. III-3c angegebene Bedeutung haben.

In der vorliegenden Anmeldung bedeutet, wenn nicht ausdrücklich anders angegeben, der Begriff Verbindungen sowohl eine Verbindung, als auch mehrere Verbindungen.

Die erfindungsgemäßen Flüssigkristallmedien weisen bevorzugt nematische Phasen von jeweils mindestens von -20°C bis 80°C, bevorzugt von -30°C bis 85°C und ganz besonders bevorzugt von -40°C bis 100°C auf. Hierbei bedeutet der Begriff eine nematische Phase aufweisen einerseits, daß bei tiefen Temperaturen bei der entsprechenden Temperatur keine smektische Phase und keine Kristallisation beobachtet wird und andererseits, daß beim Aufheizen aus der nematischen Phase noch keine Klärung auftritt. Die Untersuchung bei tiefen Temperaturen wird in einem Fließviskosimeter bei der entsprechenden Temperatur durchgeführt sowie durch Lagerung in Testzellen, einer der elektrooptischen Anwendung entsprechenden Schichtdicke, für mindestens 100 Stunden überprüft. Bei hohen Temperaturen wird der Klärpunkt nach üblichen Methoden in Kapillaren gemessen.

Ferner sind die erfindungsgemäßen Flüssigkristallmedien durch niedrige optische Anisotropien gekennzeichnet.

Der Ausdruck "Alkyl" umfaßt vorzugsweise geradkettige und verzweigte Alkylgruppen mit 1 bis 7 Kohlenstoffatomen, insbesondere die geradkettigen Gruppen Methyl, Ethyl, Propyl, Butyl, Pentyl, Hexyl und Heptyl. Gruppen mit 2 bis 5 Kohlenstoffatomen sind im allgemeinen bevorzugt.

Der Ausdruck "Alkenyl" umfaßt vorzugsweise geradkettige und verzweigte Alkenylgruppen mit 2 bis 7 Kohlenstoffatomen, insbesondere die geradkettigen Gruppen. Besonders bevorzugte Alkenylgruppen sind C₂ bis C₇-1E-Alkenyl, C₄ bis C₇-3E-Alkenyl, C₅ bis C₇-4-Alkenyl, C₆ bis C₇-5-Alkenyl und C₇ 6-Alkenyl, insbesondere C₂ bis C₇-1 E-Alkenyl, C₄ bis C₇-3E-Alkenyl und C₅ bis C₇-4-Alkenyl. Beispiele weiterer bevorzugter Alkenylgruppen sind Vinyl, 1E-Propenyl, 1E-Butenyl, 1 E-Pentenyl, 1 E-Hexenyl, 1 E-Heptenyl, 3-Butenyl, 3E-Pentenyl, 3E-Hexenyl, 3E-Heptenyl, 4-Pentenyl, 4Z-Hexenyl, 4E-Hexenyl, 4Z-Heptenyl, 5-Hexenyl, 6-Heptenyl und dergleichen. Gruppen mit bis zu 5 Kohlenstoffatomen sind im allgemeinen bevorzugt.

Der Ausdruck "Fluoralkyl" umfaßt vorzugsweise geradkettige Gruppen mit endständigem Fluor, d.h. Fluormethyl, 2-Fluorethyl, 3-Fluorpropyl, 4-Fluorbutyl, 5-Fluorpentyl, 6-Fluorhexyl und 7-Fluorheptyl. Andere Positionen des Fluors sind jedoch nicht ausgeschlossen.

Der Ausdruck "Oxaalkyl", bzw. Alkoxyalkyl umfaßt vorzugsweise geradkettige Reste der Formel CₙH₂ₙ₊₁-O-(CH₂)ₘ, worin n und m jeweils unabhängig voneinander 1 bis 6 bedeuten. Vorzugsweise ist n 1 und m 1 bis 6.

Verbindungen mir einer Vinyl-Endgruppe und Verbindungen mit einer Methyl-Endgruppe haben eine geringe Rotationsviskosität.

In der vorliegenden Anmeldung bedeuten die Begriffe dielektrisch positive Verbindungen solche Verbindungen mit einem Δε > 1,5, dielektrisch neutrale Verbindungen solche mit -1,5 ≤ Δε ≤ 1,5 und dielektrisch negative Verbindungen solche mit Δε < -1,5. Hierbei wird die dielektrische Aniso-tropie der Verbindungen bestimmt indem 10 % der Verbindungen in einem flüssigkristallinen Host gelöst werden und von dieser Mischung die Kapazität in mindestens jeweils einer Testzelle mit ca. 20 µm Schichtdicke mit homeotroper und mit homogener Oberflächenorientierung bei 1 kHz bestimmt wird. Die Meßspannung beträgt typischerweise 0,5 V bis 1,0 V, jedoch stets weniger als die kapazitive Schwelle der jeweiligen Flüssigkristallmischung.

Als Wirtsmischung für die Bestimmung der anwendungsrelevanten physikalischen Parameter wird ZLI-4792, von Merck KGaA, Deutschland, verwendet. Als Ausnahme wird bei der Bestimmung der dielektrischen Anisotropie von dielektrisch negativen Verbindungen ZLI-2857, ebenfalls von Merck KGaA, Deutschland, verwendet. Aus der Änderung der Eigenschaften, z.B. der Dielektrizitätskonstanten, der Wirtsmischung nach Zugabe der zu untersuchenden Verbindung und Extrapolation auf 100 % der eingesetzten Verbindung werden die Werte für die jeweilige zu untersuchende Verbindung erhalten.

Die eingesetzte Konzentration der zu untersuchenden Verbindung beträgt 10 %. Ist die Löslichkeit der zu untersuchenden Verbindung hierzu nicht ausreichend wird ausnahmsweise die eingesetzte Konzentration solange halbiert, also auf 5 %, 2,5% usw. verringert, bis die Löslichkeitsgrenze unterschritten ist.

Der Begriff Schwellenspannung bezieht sich üblicherweise auf die optische Schwelle für 10 % relativen Kontrast (V₁₀). In Bezug auf die Flüssigkristallmischungen mit negativer dielektrischer Anisotropie, wird der Begriff Schwellenspannung in der vorliegenden Anmeldung jedoch für die kapazitive Schwellenspannung (V₀), auch Freedericksz-Schwelle genannt, verwendet, sofern nicht explizit anders angegeben.

Alle Konzentrationen in dieser Anmeldung sind, soweit nicht explizit anders vermerkt, in Massenprozent angegeben und beziehen sich auf die entsprechende Gesamtmischung. Alle physikalischen Eigenschaften werden und wurden nach "Merck Liquid Crystals, Physical Properties of Liquid Crystals", Status Nov. 1997, Merck KGaA, Deutschland bestimmt und gelten für eine Temperatur von 20 °C, sofern nicht explizit anders angegeben. Δn wird bei 589 nm und Δε bei 1 kHz bestimmt.

Bei den Flüssigkristallmedien mit negativer dielektrischer Anisotropie wurde die Schwellenspannung als kapazitive Schwellung V₀ in Zellen mit durch Lecithin homeotrop orientierter Flüssigkristallschicht bestimmt.

Die erfindungsgemäßen Flüssigkristallmedien können bei Bedarf auch weitere Zusatzstoffe und gegebenenfalls auch chirale Dotierstoffe in den üblichen Mengen enthalten. Die eingesetzte Menge dieser Zusatzstoffe beträgt insgesamt 0 % bis 10 % bezogen auf die Menge der gesamten Mischung bevorzugt 0,1 % bis 6 %. Die Konzentrationen der einzelnen eingesetzten Verbindungen betragen jeweils bevorzugt 0,1 bis 3 %. Die Konzentration dieser und ähnlicher Zusatzstoffe wird bei der Angabe der Konzentrationen sowie der Konzentrationsbereiche der Flüssigkristallverbindungen in den Flüssigkristallmedien nicht berücksichtigt.

Die Zusammensetzungen bestehen aus mehreren Verbindungen, bevorzugt aus 3 bis 30, besonders bevorzugt aus 6 bis 20 und ganz besonders bevorzugt aus 10 bis 16 Verbindungen, die auf herkömmliche Weise gemischt werden. In der Regel wird die gewünschte Menge der in geringerer Menge verwendeten Komponenten in den Komponenten gelöst, die den Hauptbestandteil ausmachen, zweckmäßigerweise bei erhöhter Temperatur. Liegt die gewählte Temperatur über dem Klärpunkt des Hauptbestandteils, so ist die Vervollständigung des Lösungsvorgangs besonders leicht zu beobachten. Es ist jedoch auch möglich, die Flüssigkristallmischungen auf anderen üblichen Wegen, z.B. unter Verwendung von Vormischungen oder aus einem sogenannten "multi bottle" Systemen herzustellen.

Mittels geeigneter Zusatzstoffe können die erfindungsgemäßen Flüssigkristallphasen derart modifiziert werden, dass sie in jeder bisher bekannt gewordenen Art von Anzeige und insbesondere von TN-Anzeigen, sowie IPS-Anzeigen einsetzbar sind.

Die nachstehenden Beispiele dienen zur Veranschaulichung der Erfindung, ohne sie zu beschränken. In den Beispielen sind der Schmelzpunkt T(C,N), der Übergang von der smektischen (S) zur nematischen (N) Phase T(S,N) und Klärpunkt T(N,I) einer Flüssigkristallsubstanz in Grad Celsius angegeben. Die verschiedenen smektischen Phasen werden durch entsprechende Suffixe gekennzeichnet.

Die Prozentangaben sind, soweit nicht explizit anders gekennzeichnet, vorund nachstehend Massenprozente und die physikalischen Eigenschaften sind die Werte bei 20 °C, sofern nicht explizit anders angegeben.

Alle angegebenen Werte für Temperaturen in dieser Anmeldung sind °C und alle Temperaturdifferenzen entsprechend Differenzgrad, sofern nicht explizit anders angegeben.

Bei den Synthesebeispielen und -schemata bedeuten:
- DAST: Diethylaminoschwefeltrifluorid,
- DBH: Dibromdimethylhydantoin,
- DEAD: Diethylazodicarboxylat,
- DIBAL: Diisobutylaluminiumhydrid
- MTB-Ether: Methyl-tert-butylether
- NBS: N-Bromsuccinimid,
- Tf: Trifluormethansulfonyl
- THF: Tetrahydrofuran.

In der vorliegenden Anmeldung und in den folgenden Beispielen sind die Strukturen der Flüssigkristallverbindungen durch Abkürzungen, auch Acronyme genannt, angegeben, wobei die Transformation in chemische Formeln gemäß folgender Tabellen A und B erfolgt. Alle Reste CₙH₂ₙ₊₁ und CₘH₂ₘ₊₁ sind geradkettige Alkylreste mit n bzw. m C-Atomen. Die Codierung gemäß Tabelle B versteht sich von selbst. In Tabelle A ist nur das Acronym für den Grundkörper angegeben. Im Einzelfall folgt getrennt vom Acronym für den Grundkörper mit einem Strich ein Code für die Substituenten R¹, R², L¹, L² und L³:

| Code für R¹, R², L¹, L², L³ | R¹ . | R² | L¹ | L² | L³ |
|---|---|---|---|---|---|
| nm | CₙH₂ₙ₊₁ | CₘH₂ₘ₊₁ | H | H | H |
| nOm | CₙH₂ₙ₊₁ | OCₘH₂ₘ₊₁ | H | H | H |
| nO.m | OCₙH₂ₙ₊₁ | CₘH₂ₘ₊₁ | H | H | H |
| nmFF | CnH₂ₙ₊₁ | CₘH₂ₘ₊₁ | F | H | F |
| nOmFF | CₙH₂ₙ₊₁ | OCₘH₂ₘ₊₁ | F | H | F |
| nO.mFF | OCₙH₂ₙ₊₁ | CₘH₂ₘ₊₁ | F | H | F |
| nO.OmFF | OCₙH₂ₙ₊₁ | OCₘH₂ₘ₊₁ | F | H | F |
| n | CₙH₂ₙ₊₁ | CN | H | H | H |
| nN.F | CₙH₂ₙ₊₁ | CN | F | H | H |
| nN.F.F | CₙH₂ₙ₊₁ | CN | F | F | H |
| nF | CₙH₂ₙ₊₁ | F | H | H | H |
| nF.F | CₙH₂ₙ₊₁ | F | F | H | H |
| nF.F.F | CₙH₂ₙ₊₁ | F | F | F | H |
| nCl | CₙH₂ₙ₊₁ | Cl | H | H | H |
| nCl.F | CₙH₂ₙ₊₁ | Cl | F | H | H |
| NCl.F.F | CₙH₂ₙ₊₁ | Cl | F | F | H |
| nmF | CₙH₂ₙ₊₁ | CₘH₂ₘ₊₁ | F | H | H |
| nCF₃ | CₙH₂ₙ₊₁ | CF₃ | H | H | H |
| nOCF₃ | CₙH₂ₙ₊₁ | OCF₃ | H | H | H |
| nOCF₃.F | CₙH₂ₙ₊₁ | OCF₃ | F | H | H |
| nOCF₃.F.F | CₙH₂ₙ₊₁ | OCF₃ | F | F | H |
| nOCF₂ | CₙH₂ₙ₊₁ | OCHF₂ | H | H | H |
| nOCF₂.F.F | CₙH₂ₙ₊₁ | OCHF₂ | F | F | H |
| nS | CₙH₂ₙ₊₁ | NCS | H | H | H |
| rVsN | CᵣH₂ᵣ₊₁-CH=CH-CₛH₂ₛ- | CN | H | H | H |
| nEsN | CᵣH₂ᵣ₊₁-O-CₛH₂ₛ- | CN | H | H | H |
| nAm | CₙH₂ₙ₊₁ | COOCₘH₂ₘ₊₁ | H | H | H |
| nF.Cl | CₙH₂ₙ₊₁ | F | Cl | H | H |

**Tabelle A:**

| | |
|---|---|
| 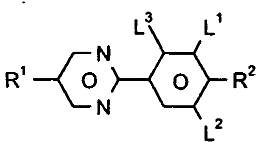 | 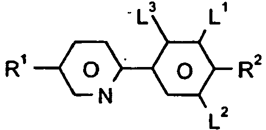 |
| PYP | PYRP |
| 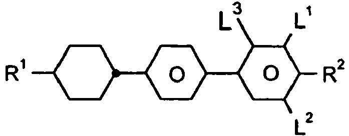 | 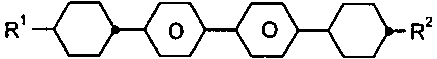 |
| BCH | CBC |
| 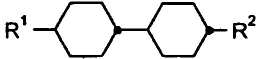 | 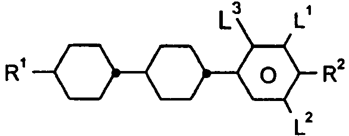 |
| CCH | CCP |
| 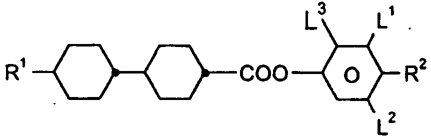 | 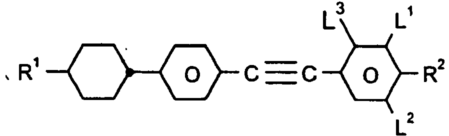 |
| CP | CPTP |
| 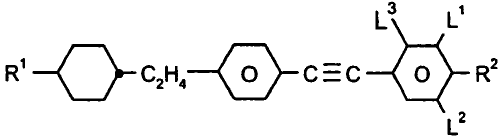 | |
| CEPTP | |
| 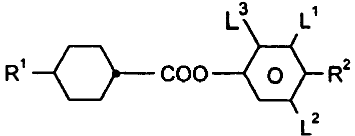 | 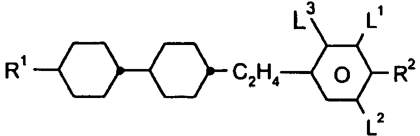 |
| D | ECCP |
| 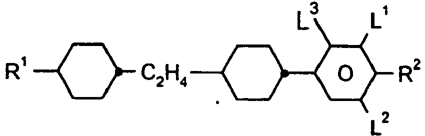 | 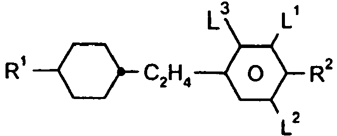 |
| CECP | EPCH |
| 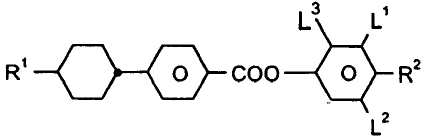 | 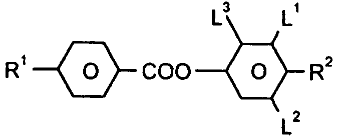 |
| HP | ME |
| 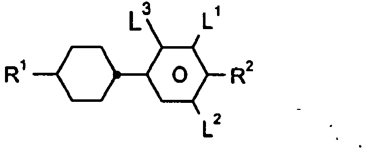 | 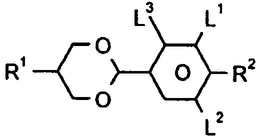 |
| PCH | PDX |
| 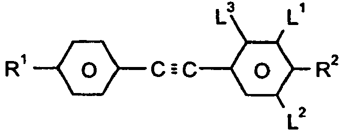 | 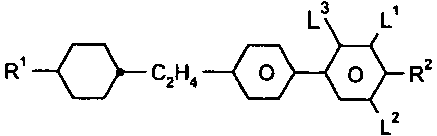 |
| PTP | BECH |
| 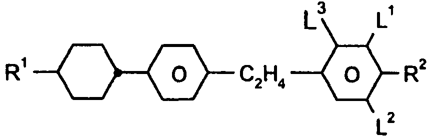 | 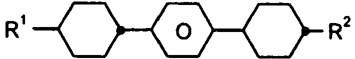 |
| EBCH | CPC |
| 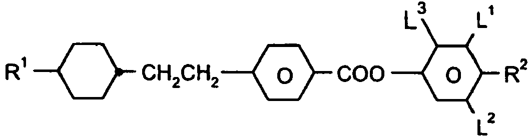 | |
| EHP | |
| 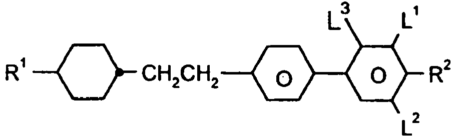 | |
| BEP | |
| 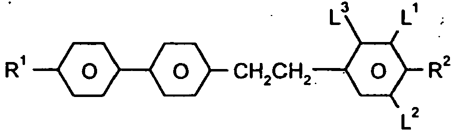 | |
| ET | |

**Tabelle B:**

| | |
|---|---|
| 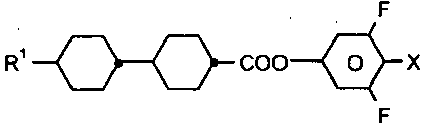 | 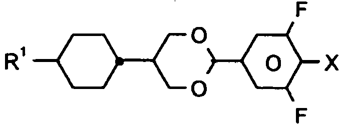 |
| **CCZU-n-X** | **CDU-n-X** |
| (X = F, Cl, -OCF3 = "OT") | (X = F, Cl, -OCF3 = "OT") |
| 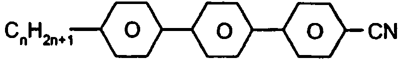 | 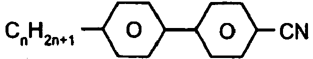 |
| **T3n** | **K3n** |
| 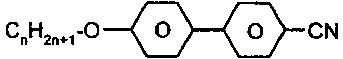 | 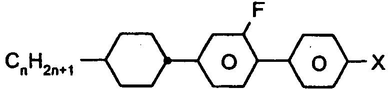 |
| **M3n** | **CGP-n-X** |
| | (X = F, Cl, -OCF3 = "OT") |
| 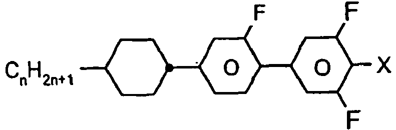 | 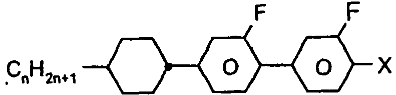 |
| **CGU-n-X** | **CGG-n-X** |
| (X = F, Cl, -OCF3 = "OT") | (X = F, Cl, -OCF3 = "OT") |
| 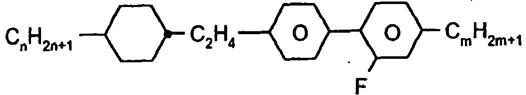 | |
| **Inm** | |
| 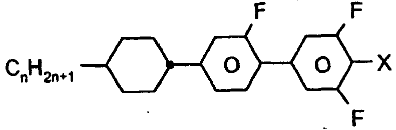 | |
| **CGU-n-X** | |
| (X = F, Cl, -OCF3 = "OT") | |
| 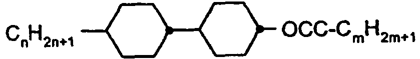 | 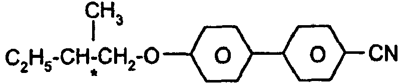 |
| **C-nm** | **C15** |
| 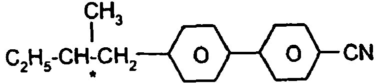 | |
| **CB15** | |
| 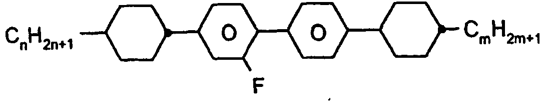 | |
| **CBC-nmF** | |
| 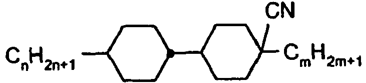 | 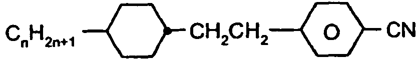 |
| **CCN-nm** | **G3n** |
| 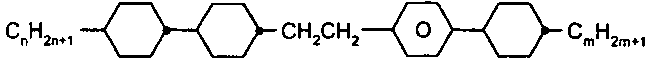 | |
| **CCEPC-nm** | |
| 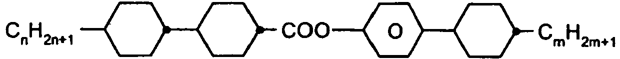 | |
| **CCPC-nm** | |
| 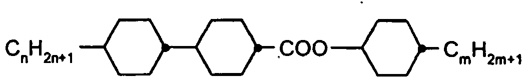 | |
| **CH-nm** | |
| 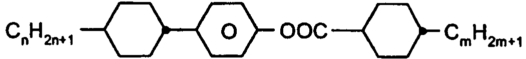 | |
| **HD-nm** | |
| 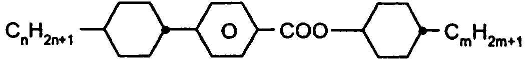 | |
| **HH-nm** | |
| 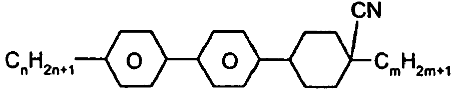 | |
| **NCB-nm** | |
| 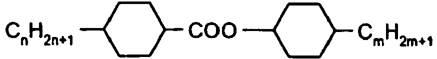 | |
| **OS-nm** | |
| 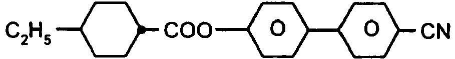 | |
| **CHE** | |
| 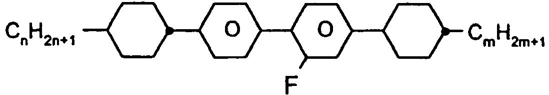 | |
| **CBC-nmF** | |
| 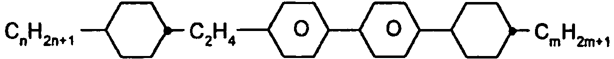 | |
| **ECBC-nm** | |
| 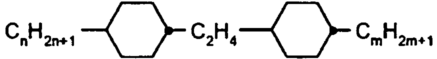 | 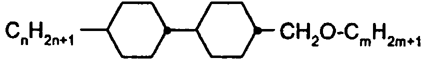 |
| **ECCH-nm** | **CCH-n1EM** |
| 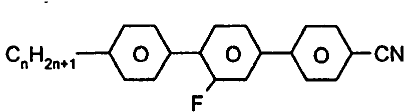 | 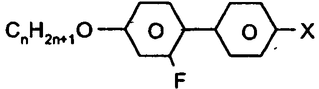 |
| **T-nFN** | **GP-nO-X** |
| | (X = F, Cl, -OCF3 = "OT") |
| 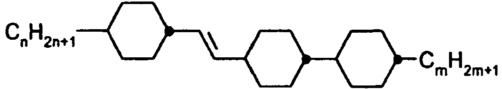 | |
| **CVCC-n-m** | |
| 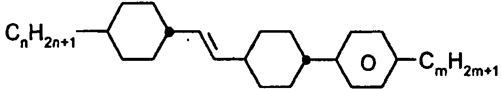 | |
| **CVCP-n-m** | |
| 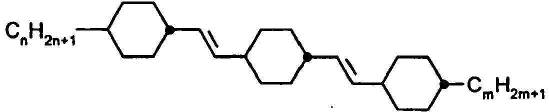 | |
| **CVCVC-n-m** | |
| 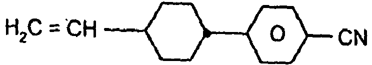 | |
| **CP-V-N** | |
| 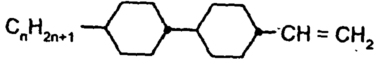 | |
| **CC-n-V** | |
| 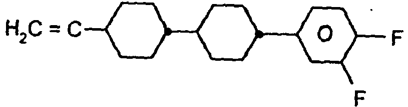 | |
| **CCG-V-F** | |
| 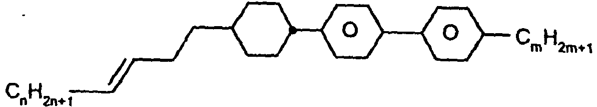 | |
| **CPP-nV2-m** | |
| 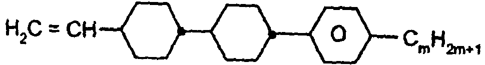 | |
| **CCP-V-m** | |
| 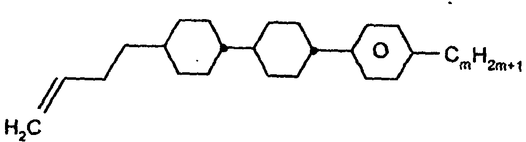 | |
| **CCP-V2-m** | |
| 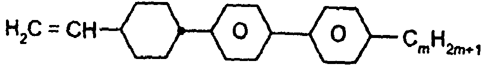 | |
| **CPP-V-m** | |
| 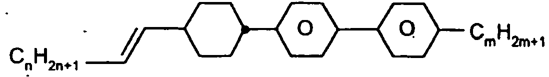 | |
| **CPP-nV-m** | |
| 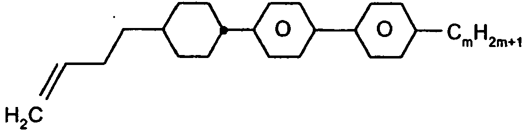 | |
| **CPP-V2-m** | |
| 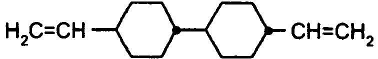 | |
| **CC-V-V** | |
| 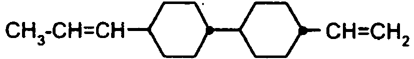 | |
| **CC-1V-V** | |
| 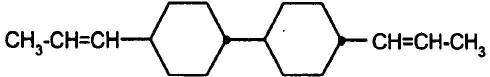 | |
| **CC-1V-V1** | |
| 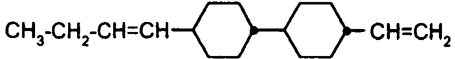 | |
| **CC-2V-V** | |
| 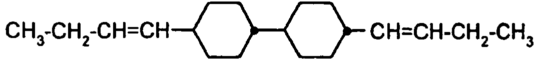 | |
| **CC-2V-V2** | |
| 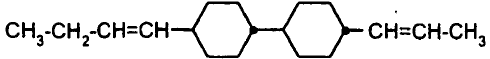 | |
| **CC-2V-V1** | |
| 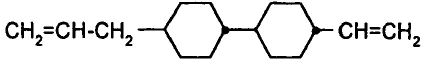 | |
| **CC-V1-V** | |
| 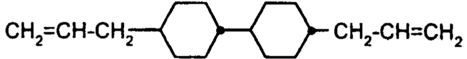 | |
| **CC-V1-1V** | |
| 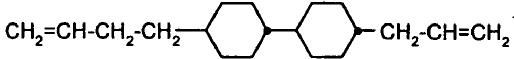 | |
| **CC-V2-1V** | |

### Beispiele

Die folgenden Beispiele sollen die Erfindung erläutern, ohne sie zu begrenzen. Vor- und nachstehend bedeuten Prozentangaben Gewichtsprozent. Alle Temperaturen sind in Grad Celsius angegeben. Δn bedeutet optische Anisotropie (589 nm, 20 °C), Δε die dielektrische Anisotropie (1 kHz, 20 °C), H.R. die "voltage holding ratio" (bei 100 °C, nach 5 Minuten im Ofen, 1 V), V₁₀, V₅₀ und V₉₀ (die Schwellenspannung, Mittgrauspannung bzw. Sättigungsspannung), sowie V₀ (die kapazitive Schwellenspannung) wurden jeweils bei 20°C bestimmt.

### Substanzbeispiele

### Vergleichsbeispiel 1: (8-Propyl-3-(3,4,5-trifluorphenyl)-7,8,9,10-tetrahydrobenzo[c]chromen-6-on)

### 1.1. Herstellung von Trifluormethansulfonsäure-6-oxo-8-propyl-7,8,9,10-tetrahydro-6H-benzo[c]chromen-3-ylester

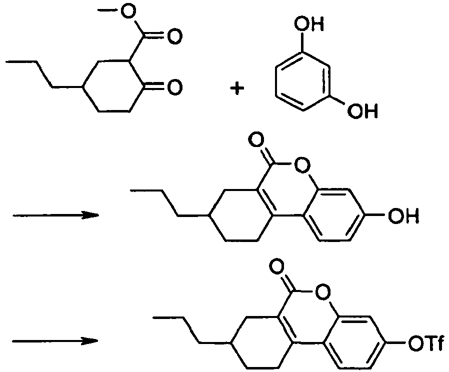

16,6 g (78,5 mmol) 2-Oxo-5-propylcyclohexancarbonsäuremethylester, 7,65 g (69,5 mmol) Resorcin und 5,6 ml (6,1 mmol) Phosphorylchlorid werden in 55 ml Toluol gelöst und 3 h unter Rückfluß erhitzt. Nach Hydrolyse mit Wasser wird der ausgefallene Niederschlag abgesaugt, mit Toluol gewaschen und getrocknet.

Das erhaltene 3-Hydroxy-8-propyl-7,8,9,10-tetrahydro-benzo[c]chromen-6-on wird in Dichlormethan gelöst, 29 ml (0,21 mol) Triethylamin hinzugegeben und bei -78 °C 25.7 ml (0,153 mol) Trifluormethansulfonsäureanhydrid zutropfen gelassen. Die Kühlung wird entfernt, der Ansatz 2 h bei Raumtemp. gerührt und auf eiskalte 1 M Salzsäure gegeben. Die wäßrige Phase wird abgetrennt und mit Dichlormethan extrahiert. Die vereinigten org. Phasen werden mit Wasser neutral gewaschen und über Natriumsulfat getrocknet. Nach Entfernung des Lösungsmittels im Vakuum erhält man Trifluormethansulfonsäure-6-oxo-8-propyl-7,8,9,10-tetrahydro-6H-benzo[c]chromen-3-ylester, der ohne weitere Aufreinigung umgesetzt wird.

### 1.2. Herstellung von 8-Propyl-3-(3,4,5-trifluorphenyl)-7,8,9,10-tetrahydrobenzo[c]chromen-6-on

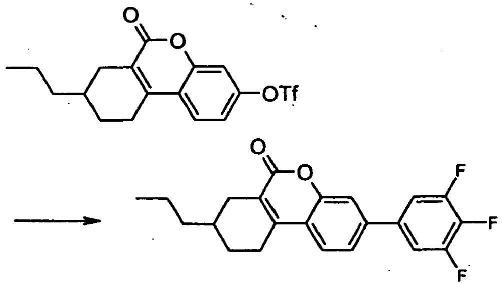

10 g (25,6 mmol) Trifluormethansulfonsäure-6-oxo-8-propyl-7,8,9,10-tetrahydro-6H-benzo[c]chromen-3-ylester, 4.50 g (25.6 mmol) 3,4,5-Trifluorbenzolboronsäure, 10,6 g (38,4 mmol) Natriummetaborat-Octahydrat, 360 mg (0,51 mmol) Bis(triphenylphosphin)palladiumchlorid und 50 µl Hydraziniumhydroxyd werden in 15 ml Wasser und 250 ml THF gelöst und über Nacht unter Rückfluß erhitzt. Der Ansatz wird mit Wasser versetzt und dreimal mit Dichlormethan extrahiert. Die vereinigten org. Phasen werden über Natriumsulfat getrocknet, das Lösungsmittel im Vakuum entfernt und der Rückstand über Kieselgel filtriert und umkristallisiert. Man erhält 8-Propyl-3-(3,4,5-trifluorphenyl)-7,8,9,10-tetrahydro-benzo[c]chromen-6-on.

### Vergleichsbeispiel 2: (8-Propyl-3-(3,4,5-trifluorphenyl)-6a,7,8,9,10,10a-hexahydro-6H-benzo[c]chromen)

### 2.1. Herstellung von 5-Propyl-2-(3',4',5'-trifluor-3-hydroxy-biphenyl-4-yl)-cyclohexancarbonsäureethylester

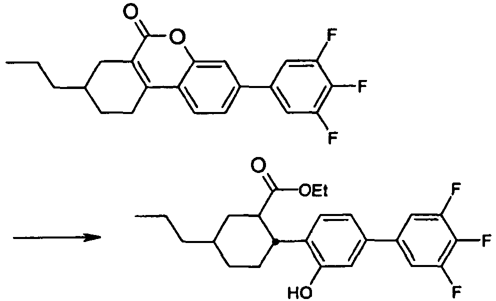

10 g (26,9 mmol) 8-Propyl-3-(3,4,5-trifluorphenyl)-7,8,9,10-tetrahydrobenzo[c]chromen-6-on aus Beispiel 1 (1.2.) werden in THF gelöst und in Gegenwart von Palladium-Aktivkohlekatalysator bis zum Stillstand hydriert. Anschließend wird filtriert, das Lösungsmittel im Vakuum entfernt und der Rückstand in abs. Ethanol gelöst und nach Zugabe von 5,5 g (80,7 mmol) Natriumethanolat über Nacht unter Rückfluß erhitzt. Nach Zugabe von Wasser wird angesäuert, die Lösung mit MTB-Ether extrahiert und über Natriumsulfat getrocknet. Das Lösungsmittel wird im Vakuum entfernt und das Rohprodukt durch Kristallisation gereinigt. Man erhält 5-Propyl-2-(3',4',5'-trifluor-3-hydroxybiphenyl-4-yl)-cyclohexancarbonsäureethylester.

### 2.2. Herstellung von 8-Propyl-3-(3,4,5-trifluorphenyl)-6a,7,8,9,10,10a-hexahydro-6H-benzo[c]chromen

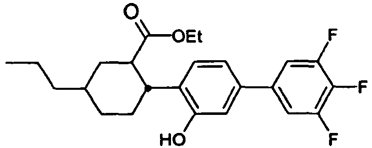

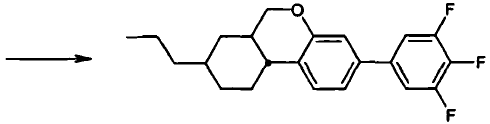

903 mg (23,8 mmol) Lithiumaluminiumhydrid werden in 20 ml THF vorgelegt und unter Eiskühlung eine Lösung von 10 g (23,8 mmol) 5-Propyl-2-(3',4',5'-trifluor-3-hydroxy-biphenyl-4-yl)-cyclohexancarbonsäureethylester in 50 ml THF zutropfen gelassen. Die Kühlung wird entfernt und der Ansatz 3 h bei Raumtemp. gerührt, 1 h unter Rückfluß erhitzt und auf Eis gegeben. Nach dem Ansäuern mit 2 M Schwefelsäure wird dreimal mit MTB-Ether extrahiert, die vereinigten org. Phasen mit Wasser gewaschen und über Natriumsulfat getrocknet. Das Lösungsmittel wird im Vakuum entfernt und der Rückstand durch Chromatographie an Kieselgel gereinigt. Das erhaltene 3',4',5'-Trifluor-4-(2-hydroxymethyl-4-propyl-cyclohexyl)-biphenyl-3-ol wird in 100 ml THF gelöst, 6,24 g (23,8 mmol) Triphenylphosphin hinzugegeben und unter Eiskühlung eine Lösung von 5,3 g (26,2 mmol) Diisopropylazodicarboxylat in 50 ml THF hinzutropfen gelassen. Die Kühlung wird entfernt und der Ansatz über Nacht bei Raumtemp. rühren gelassen. Nach Zugabe von Wasser wird die organisch Phase abgetrennt und die wäßrige Phase dreimal mit MTB-Ether extrahiert. Die vereinigten org. Phasen werden mit Wasser und gesättigter. Natriumchloridlösung gewaschen und über Natriumsulfat getrocknet. Das Lösungsmittel wird im Vakuum entfernt und der Rückstand durch Chromatographie an Kieselgel gereinigt. Man erhält 8-Propyl-3-(3,4,5-trifluorphenyl)-6a,7,8,9,1 0,1 Oa-hexahydro-6H-benzo[c]chromen als farblose Kristalle.

### Beispiele 1 bis 30

Analog zu den Vergleischsbeispielen werden hergestellt Verbindungen der Formel: 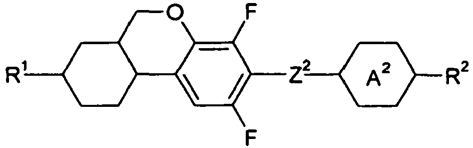 worin 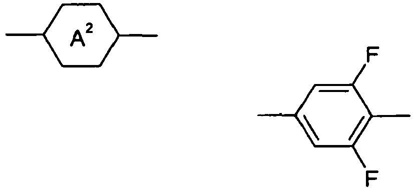 und
- Z²: eine Einfachbindung
bedeutet.

Bemerkung: *aus 10%-iger Lösung in ZLI-4792 extrapolierte Werte.

| Nr. | R¹ | R² | Phasensequenz Δε* T/°C |
|---|---|---|---|
| 1 | CH₃ | F | |
| 2 | CH₃ | Cl | |
| 3 | CH₃ | CF₃ | |
| 4 | CH₃ | OCF₃ | |
| 5 | C₂H₅ | F | |
| 6 | C₂H₅ | Cl | |
| 7 | C₂H₅ | CF₃ | |
| 8 | C₂H₅ | OCF₃ | |
| 9 | *n*-C₃H₇ | F | |
| 10 | *n*-C₃H₇ | Cl | |
| 11 | *n*-C₃H₇ | CF₃ | |
| 12 | *n*-C₃H₇ | OCF₃ | |
| 13 | *n*-C₄H₉ | F | |
| 14 | *n*-C₄H₉ | Cl | |
| 15 | *n*-C₄H₉ | CF₃ | |
| 16 | *n*-C₄H₉ | OCF₃ | |
| 17 | *n*-C₅H₁₁ | F | |
| 18 | *n*-C₅H₁₁ | Cl | |
| 19 | *n*-C₅H₁₁ | CF₃ | |
| 20 | *n*-C₅H₁₁ | OCF₃ | |
| 21 | *n*-C₇H₁₅ | F | |
| 22 | *n*-C₇H₁₅ | Cl | |
| 23 | *n*-C₇H₁₅ | CF₃ | |
| 24 | *n*-C₇H₁₅ | OCF₃ | |
| 25 | CH₂=CH | F | |
| 26 | CH₂=CH | Cl | |
| 27 | CH₂=CH | CF₃ | |
| 28 | CH₂=CH | OCF₃ | |
| 29 | *E*-CH₃-CH=CH | F | |
| 30 | *E*-CH₃-CH=CH | Cl | |

### Beispiele 31 bis 60

Analog zu den vorhergehenden Beispielen werden hergestellt Verbindungen der Formel: 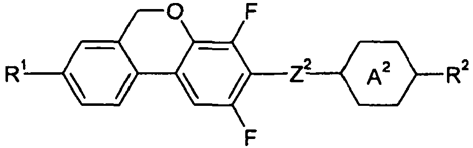 worin 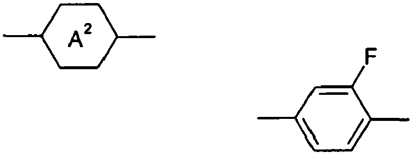 und
- Z²: eine Einfachbindung
bedeutet.

Bemerkung: * aus 10%-iger Lösung in ZLI-4792 extrapolierte Werte.

| Nr. | R¹ | R² | Phasensequenz Δε* T/°C |
|---|---|---|---|
| 31 | CH₃ | F | |
| 32 | CH₃ | Cl | |
| 33 | CH₃ | CF₃ | |
| 34 | CH₃ | OCF₃ | |
| 35 | C₂H₅ | F | |
| 36 | C₂H₅ | Cl | |
| 37 | C₂H₅ | CF₃ | |
| 38 | C₂H₅ | OCF₃ | |
| 39 | *n*-C₃H₇ | F | |
| 40 | *n*-C₃H₇ | Cl | |
| 41 | *n*-C₃H₇ | CF₃ | |
| 42 | *n*-C₃H₇ | OCF₃ | |
| 43 | *n*-C₄H₉ | F | |
| 44 | *n*-C₄H₉ | Cl | |
| 45 | *n*-C₄H₉ | CF₃ | |
| 46 | *n*-C₄H₉ | OCF₃ | |
| 47 | *n*-C₅H₁₁ | F | |
| 48 | *n*-C₅H₁₁ | Cl | |
| 49 | *n*-C₅H₁₁ | CF₃ | |
| 50 | *n*-C₅H₁₁ | OCF₃ | |
| 51 | *n*-C₇H₁₅ | F | |
| 52 | *n*-C₇H₁₅ | Cl | |
| 53 | *n*-C₇H₁₅ | CF₃ | |
| 54 | *n*-C₇H₁₅ | OCF₃ | |
| 55 | CH₂=CH | F | |
| 56 | CH₂=CH | Cl | |
| 57 | CH₂=CH | CF₃ | |
| 58 | CH₂=CH | OCF₃ | |
| 59 | *E*-CH₃-CH=CH | F | |
| 60 | *E*-CH₃-CH=CH | Cl | |

### Beispiele 61 bis 90

Analog zu den vorherigen Beispielen werden hergestellt Verbindungen der Formel: 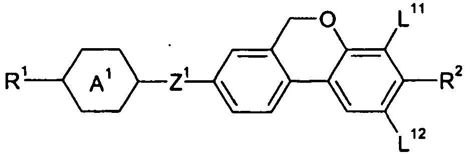 worin 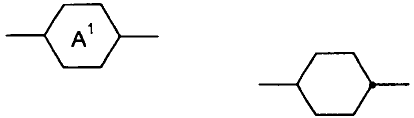 L¹¹ und L¹² F und
- Z¹: eine Einfachbindung
bedeuten.

Bemerkung: * aus 10%-iger Lösung in ZLI-4792, extrapolierte Werte.

| | | | |
|---|---|---|---|
| Nr. | R¹ | R² | Phasensequenz Δε* T/°C |
| 61 | CH₃ | F | |
| 62 | CH₃ | Cl | |
| 63 | CH₃ | CF₃ | |
| 64 | CH₃ | OCF₃ | |
| 65 | C₂H₅ | F | |
| 66 | C₂H₅ | Cl | |
| 67 | C₂H₅ | CF₃ | |
| 68 | C₂H₅ | OCF₃ | |
| 69 | *n*-C₃H₇ | F | |
| 70 | *n*-C₃H₇ | Cl | |
| 71 | *n*-C₃H₇ | CF₃ | |
| 72 | *n*-C₃H₇ | OCF₃ | |
| 73 | *n*-C₄H₉ | F | |
| 74 | *n*-C₄H₉ | Cl | |
| 75 | *n*-C₄H₉ | CF₃ | |
| 76 | *n*-C₄H₉ | OCF₃ | |
| 77 | *n*-C₅H₁₁ | F | |
| 78 | *n*-C₅H₁₁ | Cl | |
| 79 | *n*-C₅H₁₁ | CF₃ | |
| 80 | *n*-C₅H₁₁ | OCF₃ | |
| 81 | *n*-C₇H₁₅ | F | |
| 82 | *n*-C₇H₁₅ | Cl | |
| 83 | *n*-C₇H₁₅ | CF₃ | |
| 84 | *n*-C₇H₁₅ | OCF₃ | |
| 85 | CH₂=CH | F | |
| 86 | CH₂=CH | Cl | |
| 87 | CH₂=CH | CF₃ | |
| 88 | CH₂=CH | OCF₃ | |
| 89 | *E*-CH₃-CH=CH | F | |
| 90 | *E*-CH₃-CH=CH | Cl | |

### Mischungsbeispiele

Es werden flüssigkristalline Gemische hergestellt und auf ihre anwendungstechnischen Eigenschaften untersucht.

### Vergleichsbeispiel M 1

Es wurde eine Flüssigkristallmischung mit der in der folgenden Tabelle angegebenen Zusammensetzung hergestellt und untersucht. Sie hat die ebenfalls in der Tabelle gezeigten Eigenschaften.

| Zusammensetzung | | | Physikalische Eigenschaften | | |
|---|---|---|---|---|---|
| Verbindung | | Konz. | T(N,I) | = | 92,2 °C |
| # | Abkürzunq | /Massen-% | | | |
| 1 | CCP-3OCF3 | 7 | Δn(20°C,589nm) | = | 0.1053 |
| 2 | CCG-V-F | 6 | | | |
| 3 | CCP-3F.F.F | 7 | Δε(20°C, 1kHz) | = | 6,6 |
| 4 | ECCP-3F.F | 12 | | | |
| 5 | ECCP-5F.F | 10 | γ₁(20°C) | = | 148 mPa·s |
| 6 | BCH-2F.F | 9 | | | |
| 7 | BCH-3F.F.F | 13 | | | |
| 8 | CC-3-V1 | 6 | | | |
| 9 | CC-5-V | 10 | | | |
| 10 | BCH-32F | 7 | | | |
| 11 | BCH-52F | 5 | | | |
| 12 | ***Vergl. Verb.*** | | | | |
| | ***Bsp. 1*** | 6 | | | |
| Σ | | 100,0 | | | |

Das Flüssigkristallmedium hat sehr gute anwendungstechnische Eigenschaften und kann für verschiedene AMD Technologien wie TN- und IPS-Anzeigen eingesetzt werden.

## Patentansprüche

1. Verbindung der Formel I 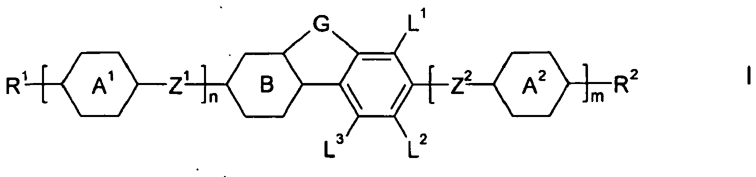 worin
das Strukturelement 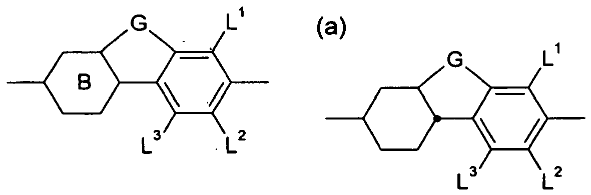 oder 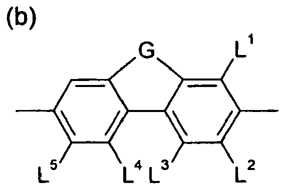 sowie
| | |
|---|---|
| G | -CO-O-, -CH₂-O-, -CF₂-O-, -O-CO-, -O-CH₂-O- oder -O-CF₂-, |
| 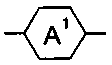 | |
| und | |
| 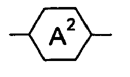 | , jeweils unabhängig voneinander, und wenn mehrfach vorhanden auch diese unabhängig voneinander, |
(a) einen trans-1,4-Cyclohexylenrest, worin auch eine oder zwei nicht benachbarte CH₂-Gruppen durch -O- und/oder -S- ersetzt sein können,
(b) einen 1,4-Cyclohexenylenrest,
(c) einen 1,4-Phenylenrest, worin auch eine oder zwei nicht benachbarte CH-Gruppen durch N ersetzt sein können oder
(d) Naphtalin-2,6-diyl, Decahydronaphthalin-2,6-diyl und 1,2,3,4-Tetrahydronaphthalin-2,6-diyl,
(e) einen Rest ausgewählt aus der Gruppe 1,4-Bicyclo-[2,2,2]-octylen, 1,3-Bicyclo-[1,1,1]-pentylen oder Spiro-[3,3]-heptan-2,6-diyl, 1,3-Cyclobutylen, wobei in
(a) und (b) eine oder mehrere -CH₂- Gruppen, unabhängig voneinander, jeweils durch eine -CHF- oder eine -CF₂- Gruppe ersetzt sein können und in
(c) und (d) eine oder mehrere -CH= Gruppen, unabhängig voneinander, jeweils durch eine -CF=, eine -C(CN)=, eine -C(CH₃)= , eine -C(CH₂F)=, eine -C(CHF₂)=, eine -C(O-CH₃)=, eine -C(O-CHF₂)= oder eine -C(O-CF₃)=Gruppe, bevorzugt eine -CF= Gruppe, ersetzt sein können,
L¹ und L² F,
L³ H,
L⁴ und L⁵, jeweils unabhängig voneinander, H oder F,
R¹ und R², jeweils unabhängig voneinander, Alkyl und Alkoxy mit 1 bis 15 C-Atomen, Alkoxyalkyl, Alkenyl oder Alkenyloxy mit 2 bis 15 C-Atomen, Alkinyl oder Alkinyloxy mit 2 bis 15 C-Atomen, H, Halogen, -CN, -SCN, -NCS, -OCN, -SF₅, -CF₃, -CHF₂, -CH₂F, -OCF₃, -OCHF₂, eine einfach durch -CN oder -CF₃ oder mindestens einfach durch Halogen substituierte Alkylgruppe mit 1 bis 15 C-Atomen, wobei auch eine oder mehrere CH₂-Gruppen, jeweils unabhängig voneinander,
durch -O-, -S-, -CH=CH-, -CF=CF-, -CF=CH-, -CH=CF-, 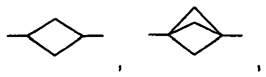 -CO-, -CO-O-, -O-CO- oder -O-CO-O- so ersetzt sein können, dass weder O-noch S-Atome direkt miteinander verknüpft sind,
Z¹ und Z², jeweils unabhängig voneinander, und wenn mehrfach vorhanden auch diese unabhängig voneinander, -CH₂-CH₂-, -(CH₂)₄-, -CF₂-CF₂-, -CF₂-CH₂-, -CH₂-CF₂-, -CH=CH-, -CF=CF-, -CF=CH-, -CH=CF-, -C≡C-, -CO-O-, -O-CO-, -CH₂-O-, -O-CH₂-, -CF₂-O-, -O-CF₂-, oder eine Kombination von zweien dieser Gruppen, wobei keine zwei O-Atome miteinander verbunden sind, oder eine Einfachbindung,
n und m jeweils 0, 1 oder 2, wobei
n + m 0, 1, 2 oder 3,
bedeuten.

2. Verbindung nach nach Anspruch 1, ausgewählt aus der Gruppe der Verbindungen der Unterformeln I-A und I-B 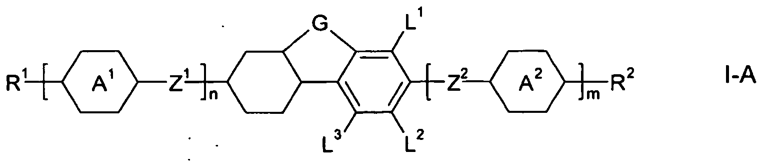 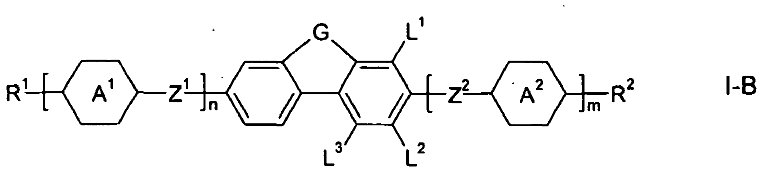 worin die Parameter die in Anspruch 1 gegebene Bedeutung haben.

3. Verbindung nach mindestens einem der Ansprüche 1 und 2, ausgewählt aus der Gruppe der Verbindungen der Unterformeln I-A1 bis I-A3 und I-B1 bis I-B3 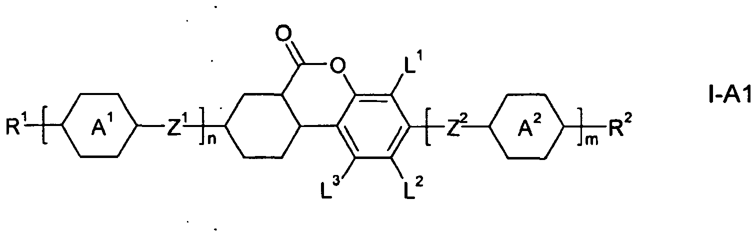 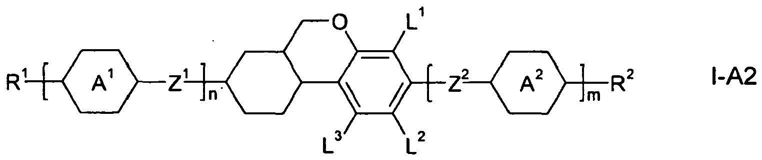 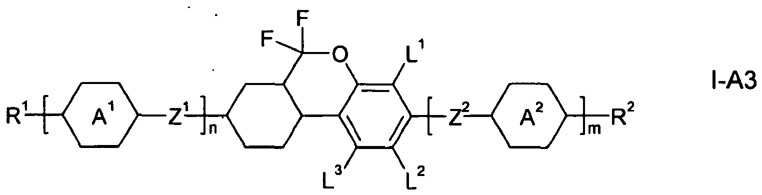 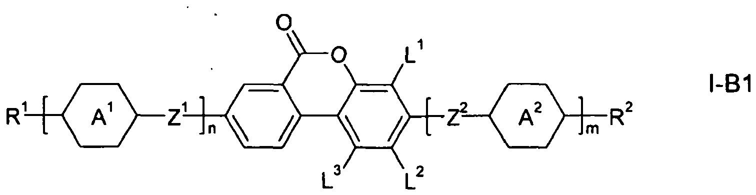 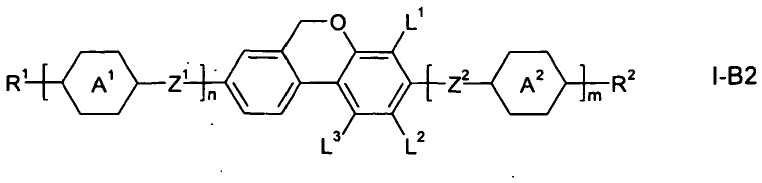 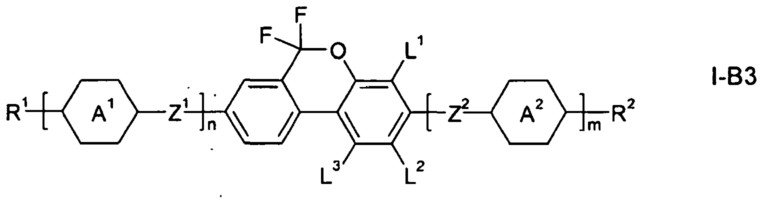 worin die Parameter die in Anspruch 1 gegebene Bedeutung haben.

4. Verbindung nach mindestens einem der Ansprüche 1 bis 3, **dadurch gekennzeichnet, dass** Z¹ und Z² beide eine Einfachbindung bedeuten.

5. Flüssigkristallmedium, **dadurch gekennzeichnet, dass** es eine oder mehrere Verbindungen der Formel I, wie in mindestens einem der Ansprüche 1 bis 4 definiert, enthält.

6. Flüssigkristallmedium nach Anspruch 5, **dadurch gekennzeichnet, dass** es eine nematische Phase aufweist.

7. Flüssigkristallmedium nach Anspruch 6, **dadurch gekennzeichnet, dass** es eine positive dielektrische Anisotropie aufweist.

8. Flüssigkristallmedium nach mindestens einem der Ansprüche 5 bis 7, **dadurch gekennzeichnet, dass** es
eine oder mehrere dielektrisch positive Verbindung(en) der Formel II 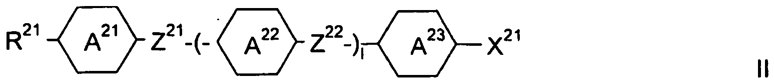 worin
R²¹ die in Anspruch 1 für R¹ gegebene Bedeutung hat,
X²¹ Halogen, -CN, -SCN, -NCS, -OCN, -SF₅, -CF₃, -CHF₂, -CH₂F, -OCF₃, -OCHF₂, eine einfach durch CN oder CF₃ oder mindestens einfach durch Halogen substituierte Alkylgruppe mit 1 bis 15 C-Atomen, wobei auch eine oder mehrere CH₂-Gruppen, jeweils unabhängig voneinander, durch -O-, -S-, -CH=CH-, -CF=CF-, -CF=CH-, -CH=CF-, 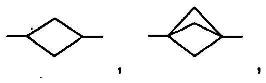 -CO-, -CO-O-, -O-CO- oder -O-CO-O- so ersetzt sein können, dass weder O-noch S-Atome direkt miteinander verknüpft sind,
Z²¹ und Z²² , jeweils unabhängig voneinander, die in Anspruch 1 für Z¹ gegebene Bedeutung haben,
mindestens einer der vorhandenen Ringe 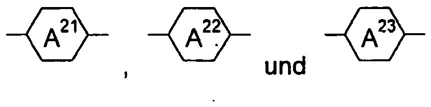 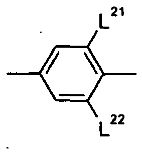 und die anderen, jeweils unabhängig voneinander, 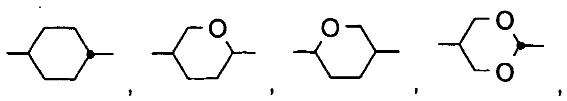 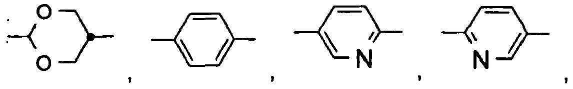 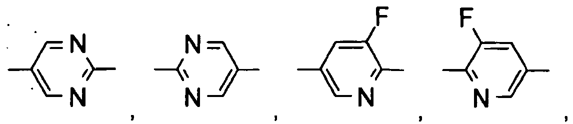 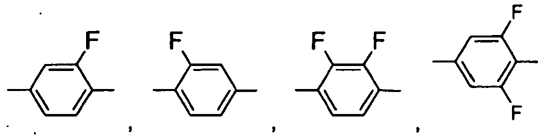 oder 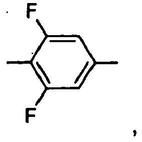
L²¹ und L²² , unabhängig voneinander, H oder F,
I 0, 1 oder 2
bedeuten, enthält.

9. Flüssigkristallmedium nach mindestens einem der Ansprüche 5 bis 8 **dadurch gekennzeichnet, dass** es eine oder mehrere dielektrisch neutrale Verbindung(en) der Formel III 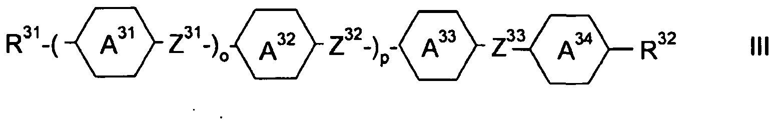 worin
R³¹ und R³² jeweils unabhängig voneinander die oben bei Formel I für R¹ gegebene Bedeutung besitzen und
Z³¹, Z³² und Z³³ jeweils unabhängig voneinander -CH₂CH₂-, -CH=CH-, -COO- oder eine Einfachbindung 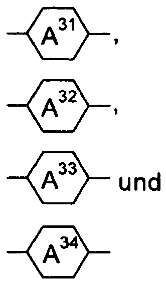 jeweils unabhängig voneinander 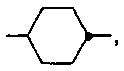 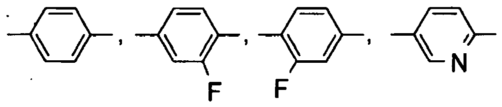 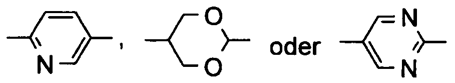 und
o und p unabhängig voneinander 0 oder 1
bedeuten, wobei bei dem Phenylenring ein oder mehrere H-Atome, unabhängig voneinander durch F oder CN, bevorzugt durch F und eine oder zwei nicht benachbarte CH₂-Gruppen des Cyclohexylenrings bzw. eines der Cyclohexylenringe durch O-Atome ersetzt sein können, enthält.

10. Verwendung eines Flüssigkristallmediums nach mindestens einem der Ansprüche 5 bis 9 in einer elektrooptischen Anzeige.

11. Elektrooptische Anzeige enthaltend ein Flüssigkristallmedium nach mindestens einem der Ansprüche 5 bis 9.

12. Anzeige nach Anspruch 11, **dadurch gekennzeichnet, daß** es sich um eine TN- oder eine IPS-Anzeige handelt.

## Claims

1. Compound of the formula I 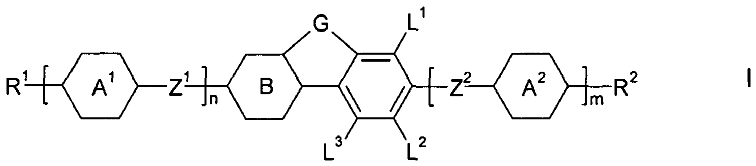 in which
the structural element 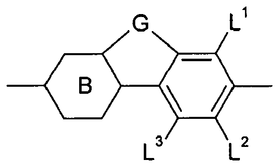 denotes 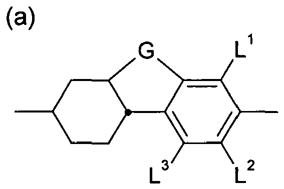 or 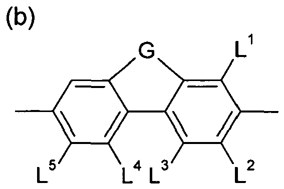 and
| | |
|---|---|
| G | denotes -CO-O-, -CH₂-O-, -CF₂-O-, -O-CO-, -O-CH₂-O- or -O-CF₂-, |
| 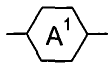 | |
| and | |
| 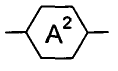 | each, independently of one another and, if present more than once, also these independently of one another, denote |
(a) a trans-1,4-cyclohexylene radical, in which, in addition, one or two non-adjacent CH₂ groups may be replaced by -O- and/or -S-,
(b) a 1,4-cyclohexenylene radical,
(c) a 1,4-phenylene radical, in which, in addition, one or two non-adjacent CH groups may be replaced by N, or
(d) naphthalene-2,6-diyl, decahydronaphthalene-2,6-diyl and 1,2,3,4-tetrahydronaphthalene-2,6-diyl,
(e) a radical selected from the group 1,4-bicyclo[2.2.2]octylene, 1,3-bicyclo[1.1.1]pentylene, spiro[3.3]heptane-2,6-diyl, 1,3-cyclobutylene, where in
(a) and (b), one or more -CH₂- groups may be replaced, in each case independently of one another, by a -CHF- or -CF₂-group, and in
(c) and (d), one or more -CH= groups may be replaced, in each case independently of one another, by a -CF=, -C(CN)=, -C(CH₃)=, -C(CH₂F)=, -C(CHF₂)=, -C(O-CH₃)=, -C(O-CHF₂)= or -C(O-CF₃)= group, preferably a -CF= group, L¹ and L² denote F,
L³ denotes H,
L⁴ and L⁵ each, independently of one another, denote H or F,
R¹ and R² each, independently of one another, denote alkyl or alkoxy having 1 to 15 C atoms, alkoxyalkyl, alkenyl or alkenyloxy having 2 to 15 C atoms, alkynyl or alkynyloxy having 2 to 15 C atoms, H, halogen, -CN, -SCN, -NCS, -OCN, -SF₅, -CF₃, -CHF₂, -CH₂F, -OCF₃, -OCHF₂, an alkyl group having 1 to 15 C atoms which is monosubstituted by -CN or -CF₃ or at least monosubstituted by halogen, where, in addition, one or more CH₂ groups may be replaced, in each case independently of one another, by -O-, -S-, -CH=CH-, -CF=CF-, -CF=CH-, -CH=CF-, 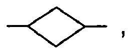 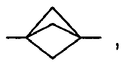 -CO-, -CO-O-, -O-CO- or -O-CO-O- in such a way that neither O nor S atoms are linked directly to one another,
Z¹ and Z² each, independently of one another and, if present more than once, also these independently of one another, denote -CH₂-CH₂-, -(CH₂)₄-, -CF₂-CF₂-, -CF₂-CH₂-, -CH₂-CF₂-, -CH=CH-, -CF=CF-, -CF=CH-, -CH=CF-, -C≡C-, -CO-O-, -O-CO-, -CH₂-O-, -O-CH₂-, -CF₂-O-, -O-CF₂-, or a combination of two of these groups, where no two O atoms are bonded to one another, or a single bond,
n and m each denote 0, 1 or 2, where
n + m denotes 0, 1, 2 or 3.

2. Compound according to Claim 1, selected from the group of the compounds of the sub-formulae I-A and I-B 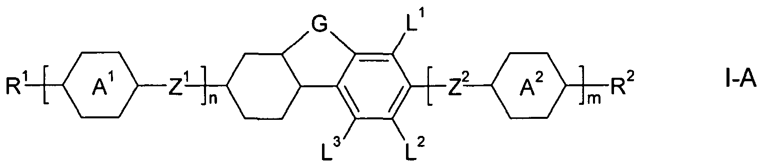 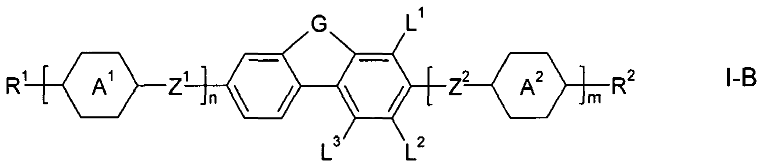 in which the parameters have the meaning given in Claim 1.

3. Compound according to at least one of Claims 1 and 2, selected from the group of the compounds of the sub-formulae I-A1 to I-A3 and I-B1 to I-B3 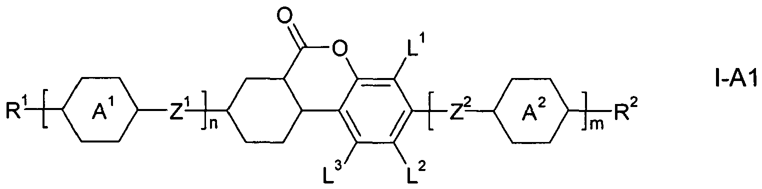 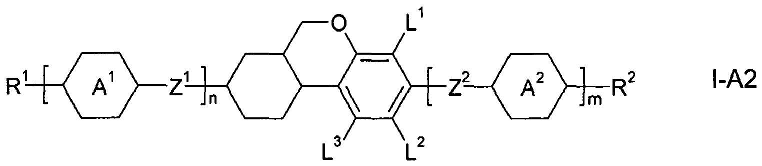 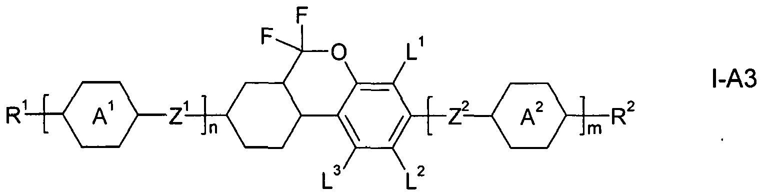 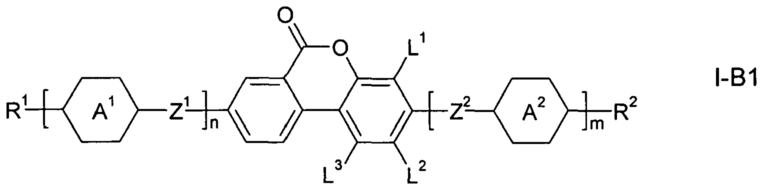 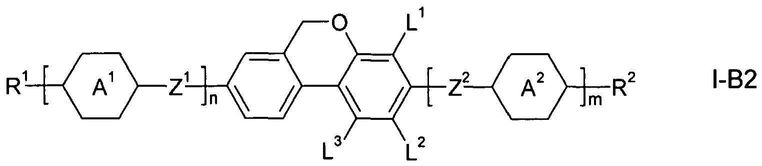 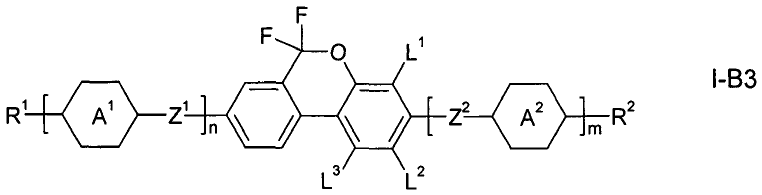 in which the parameters have the meaning given in Claim 1.

4. Compound according to at least one of Claims 1 to 3, **characterised in that** Z¹ and Z² both denote a single bond.

5. Liquid-crystal medium, **characterised in that** it comprises one or more compounds of the formula I as defined in at least one of Claims 1 to 4.

6. Liquid-crystal medium according to Claim 5, **characterised in that** it has a nematic phase.

7. Liquid-crystal medium according to Claim 6, **characterised in that** it has a positive dielectric anisotropy.

8. Liquid-crystal medium according to at least one of Claims 5 to 7, **characterised in that** it comprises
one or more dielectrically positive compound(s) of the formula II 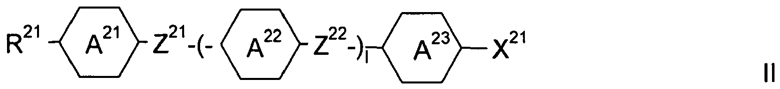 in which
R²¹ has the meaning given for R¹ in Claim 1,
X²¹ denotes halogen, -CN, -SCN, -NCS, -OCN, -SF₅, -CF₃, -CHF₂, -CH₂F, -OCF₃, -OCHF₂, an alkyl group having 1 to 15 C atoms which is monosubstituted by CN or CF₃ or at least monosubstituted by halogen, where, in addition, one or more CH₂ groups may be replaced, in each case independently of one another, by -O-, -S-, -CH=CH-, -CF=CF-, -CF=CH-, -CH=CF-, 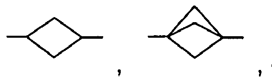 -CO-, -CO-O-, -O-CO- or -O-CO-O- in such a way that neither O nor S atoms are linked directly to one another,
Z²¹ and Z²² each, independently of one another, have the meanring given for Z¹ in Claim 1,
at least one of the rings 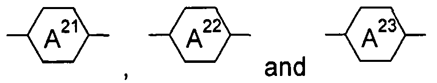 present
denotes 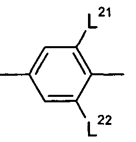 and the others, in each case independently of one another, denote 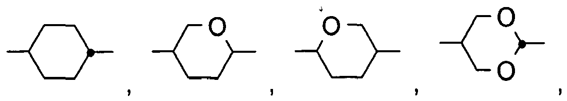 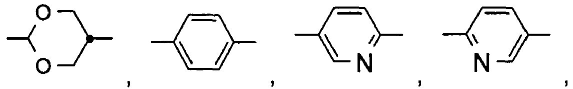 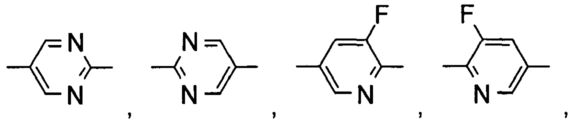 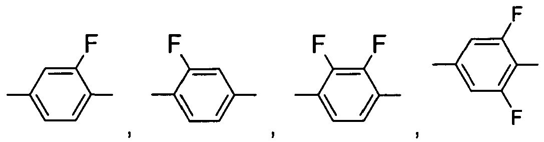 or 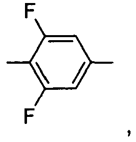
L²¹ and L²², independently of one another, denote H or F,
I denotes 0, 1 or 2.

9. Liquid-crystal medium according to at least one of Claims 5 to 8, **characterised in that** it comprises one or more dielectrically neutral compound(s) of the formula III 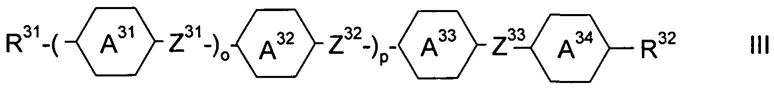 in which
R³¹ and R³² each, independently of one another, have the meaning given above for R¹ in the case of formula **I,** and
Z³¹, Z³² and Z³³ each, independently of one another, denote -CH₂CH₂-, -CH=CH-, -COO- or a single bond, 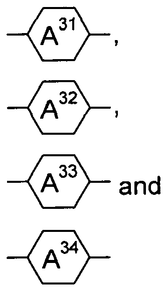 each, independently of one another, denote 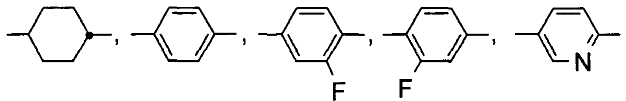 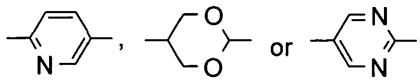 and
o and p, independently of one another, denote 0 or 1,
where in the case of the phenylene ring, one or more H atoms may be replaced, independently of one another, by F or CN, preferably by F, and one or two non-adjacent CH₂ groups of the cyclohexylene ring or of one of the cyclohexylene rings may be replaced by O atoms.

10. Use of a liquid-crystal medium according to at least one of Claims 5 to 9 in an electro-optical display.

11. Electro-optical display containing a liquid-crystal medium according to at least one of Claims 5 to 9.

12. Display according to Claim 11, **characterised in that** it is a TN or IPS display.

## Revendications

1. Composé de la formule I 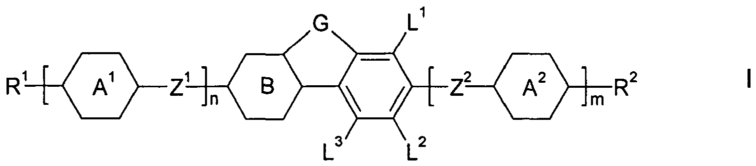 dans laquelle
l'élément structurel 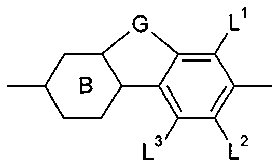 représente 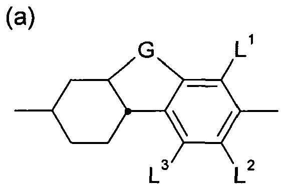 ou 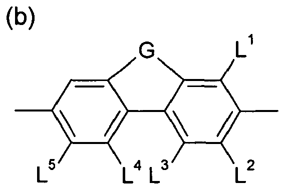 et
G représente -CO-O-, -CH₂-O-, -CF₂-O-, -O-CO-, -O-CH₂-O- ou -O-CF₂-, 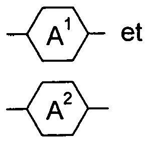 représentent, chacun indépendamment de l'autre, et dans le cas d'une occurrence supérieure à l'unité, également indépendamment les unes des autres
(a) un radical trans-1,4-cyclohexylène, où, en outre, un ou deux groupes CH₂ non adjacents peut/peuvent être remplacé(s) par -O- et/ou -S-,
(b) un radical 1,4-cyclohexénylène,
(c) un radical 1,4-phénylène, où, en outre, un ou deux groupes CH non adjacents peut/peuvent être remplacé(s) par N, ou
(d) naphtalène-2,6-diyle, décahydronaphtalène-2,6-diyle et 1,2,3,4-tétrahydronaphtalène-2,6-diyle,
(e) un radical choisi parmi le groupe 1,4-bicyclo[2.2.2]octylène, 1,3-bicyclo[1.1.1]pentylène, spiro[3.3]heptane-2,6-diyle, 1,3-cyclobutylène, où, dans (a) et (b), un ou plusieurs groupes -CH₂- peut/peuvent être remplacé(s), dans chaque cas indépendamment les uns des autres, par un groupe -CHF- ou -CF₂-, et dans (c) et (d), un ou plusieurs groupes -CH= peut/peuvent être remplacé(s), dans chaque cas indépendamment les uns des autres, par un groupe -CF=, -C(CN)=, -C(CH₃)=, -C(CH₂F)=, -C(CHF₂)=, -C(O-CH₃)=, -C(O-CHF₂)= ou -C(O-CF₃)=, de préférence un groupe a -CF=,
L¹ et L² représentent F,
L³ représente H,
L⁴ et L⁵ représentent, chacun indépendamment l'un de l'autre, H ou F,
R¹ et R² représentent, chacun indépendamment l'un de l'autre, alkyle ou alcoxy comportant de 1 à 15 atomes de C, alcoxyalkyle, alkényle ou alkényloxy comportant de 2 à 15 atomes de C, alkynyle ou alkynyloxy comportant de 2 à 15 atomes de C, H, halogène, -CN, -SCN, -NCS, -OCN, -SF₅, -CF₃, -CHF₂, -CH₂F, -OCF₃, -OCHF₂, un groupe alkyle comportant de 1 à 15 atomes de C, lequel est monosubstitué par -CN ou -CF₃ ou au moins monosubstitué par halogène, où, en outre, un ou plusieurs groupes CH₂ peut/ peuvent être remplacé(s), chacun indépendamment l'un de l'autre, -O-, -S-, -CH=CH-, -CF=CF-, -CF=CH-, -CH=CF-, 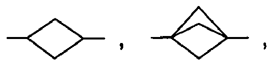 -CO-, -CO-O-, -O-CO- ou -O-CO-O- de telle sorte que ni des atomes de O, ni des atomes de S ne soient liés directement les uns aux autres,
Z¹ et Z² représentent, chacun indépendamment l'un de l'autre, et dans le cas d'une occurrence supérieure à l'unité, également indépendamment les unes des autres -CH₂-CH₂-, -(CH₂)₄-, -CF₂-CF₂-, -CF₂-CH₂-, -CH₂-CF₂-, -CH=CH-, -CF=CF-, -CF=CH-, -CH=CF-, -C≡C-, -CO-O-, -O-CO-, -CH₂-O-, -O-CH₂-, -CF₂-O-, -O-CF₂-, ou une combinaison de deux de ces groupes, où deux atomes de O ne sont jamais liés l'un à l'autre, ou une liaison simple,
n et m représentent chacun 0, 1 ou 2, où
n + m représente 0, 1, 2 ou 3.

2. Composé selon la revendication 1, choisi parmi le groupe des composés des sous-formules I-A et I-B 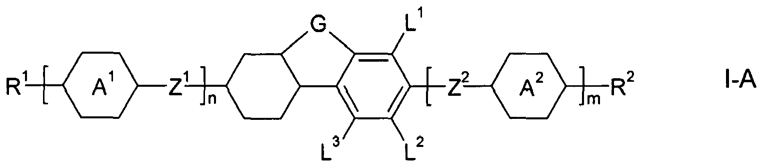 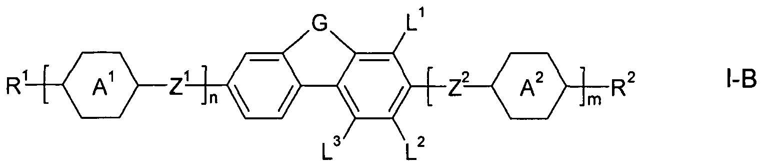 dans lesquelles les paramètres présentent la signification donnée selon la revendication 1.

3. Composé selon au moins l'une des revendications 1 et 2, choisi parmi le groupe des composés des sous-formules I-A1 à I-A3 et I-B1 à I-B3 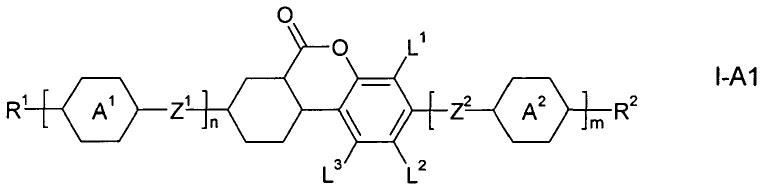 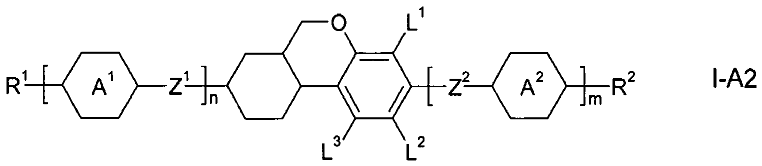 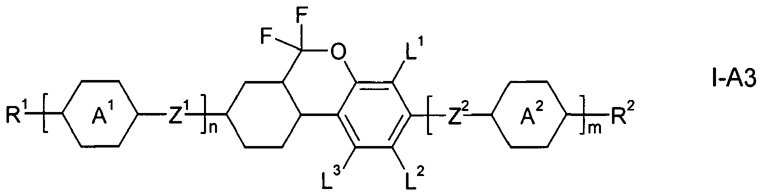 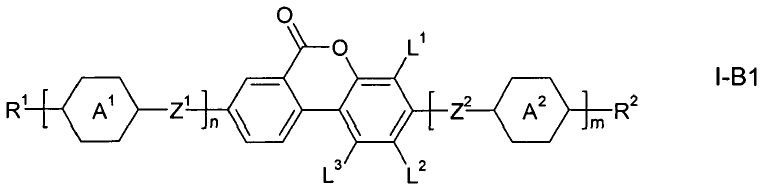 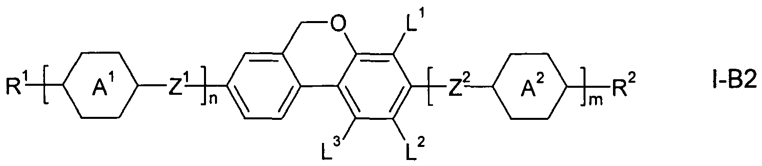 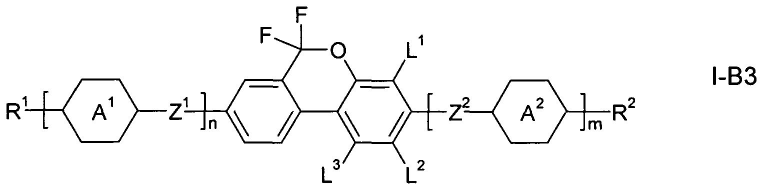 dans lesquelles les paramètres présentent la signification donnée selon la revendication 1.

4. Composé selon au moins l'une des revendications 1 à 3, **caractérisé en ce que** Z¹ et Z² représentent tous deux une liaison simple.

5. Milieu cristallin liquide, **caractérisé en ce qu'**il comprend un ou plusieurs composés de la formule I comme défini selon au moins l'une des revendications 1 à 4.

6. Milieu cristallin liquide selon la revendication 5, **caractérisé en ce qu'**il présente une phase nématique.

7. Milieu cristallin liquide selon la revendication 6, **caractérisé en ce qu'**il présente une anisotropie diélectrique positive.

8. Milieu cristallin liquide selon au moins l'une des revendications 5 à 7, **caractérisé en ce qu'**il comprend
un ou plusieurs composé(s) diélectriquement positif(s) de la formule II 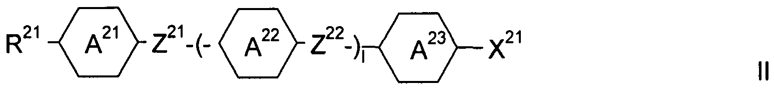 dans laquelle
R²¹ présente la signification donnée pour R¹ selon la revendication 1,
X²¹ représente halogène, -CN, -SCN, -NCS, -OCN, -SF₅, -CF₃, -CHF₂, -CH₂F, -OCF₃, -OCHF₂, un groupe alkyle comportant de 1 à 15 atomes de C, lequel est monosubstitué par CN ou CF₃ ou au moins monosubstitué par halogène, où, en outre, un ou plusieurs groupes CH₂ peut/peuvent être remplacé(s), dans chaque cas indépendamment les uns des autres, par -O-, -S-, -CH=CH-, -CF=CF-, -CF=CH-, -CH=CF-, 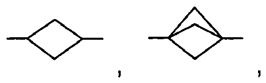 -CO-, -CO-O-, -O-CO- ou -O-CO-O- de telle sorte que ni des atomes de O, ni des atomes de S ne soient liés directement les uns aux autres,
Z²¹ et Z²² présentent, chacun indépendamment des autres, la signification donnée pour Z¹ selon la revendication 1,
au moins l'un des cycles 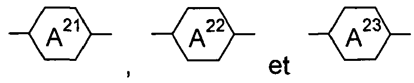 présents
représente 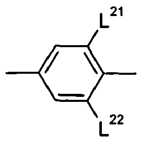 et les autres représentent, dans chaque cas indépendamment les uns des autres, 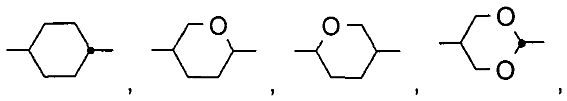 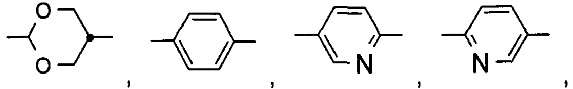 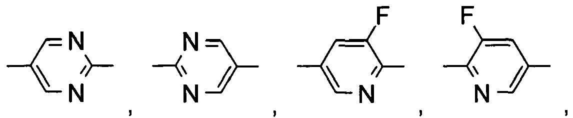 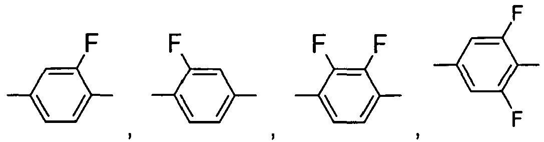 ou 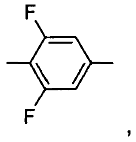
L²¹ et L²² représentent, indépendamment l'un de l'autre, H ou F,
I représente 0, 1 ou 2.

9. Milieu cristallin liquide selon au moins l'une des revendications 5 à 8, **caractérisé en ce qu'**il comprend un ou plusieurs composé(s) diélectriquement neutre(s) de la formule III 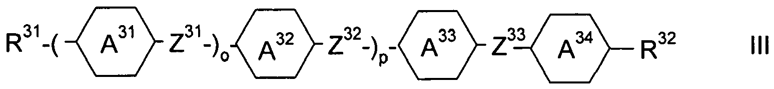 dans laquelle
R³¹ et R³² présentent, chacun indépendamment de l'autre, la signification donnée ci-avant pour R¹ dans le cas de la formule I, et
Z³¹, Z³² et Z³³ représentent, chacun indépendamment l'un de l'autre, -CH₂CH₂-, -CH=CH-, -COO- ou une liaison simple, 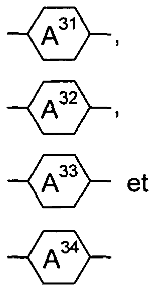 représentent, chacun indépendamment des autres, 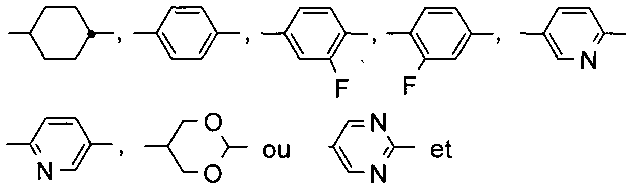
o et p, représentent, indépendamment de l'autre, 0 ou 1,
où, dans le cas du cycle phénylène, un ou plusieurs atomes de H peut/peuvent être remplacé(s), indépendamment les uns des autres, par F ou CN, de préférence par F, et un ou deux groupes CH₂ non adjacents du cycle cyclohexylène ou de l'un des cycles cyclohexylène peut/peuvent être remplacé(s) par des atomes de O.

10. Utilisation d'un milieu cristallin liquide selon au moins l'une des revendications 5 à 9 dans un affichage électro-optique.

11. Affichage électro-optique contenant un milieu cristallin liquide selon au moins l'une des revendications 5 à 9.

12. Affichage selon la revendication 11, **caractérisé en ce qu'**il s'agit d'un affichage TN ou IPS.
